(19) Europäisches Patentamt
European Patent Office
Office européen des brevets

(11) Veröffentlichungsnummer: **0 037 797**
**A2**

(12) **EUROPÄISCHE PATENTANMELDUNG**

(21) Anmeldenummer: 81810078.6

(22) Anmeldetag: 05.03.81

(51) Int. Cl.³: C 07 D 501/20
C 07 D 501/59, A 61 K 31/545
//C07D275/02

(30) Priorität: 11.03.80 CH 1901/80

(43) Veröffentlichungstag der Anmeldung:
14.10.81 Patentblatt 81/41

(84) Benannte Vertragsstaaten:
AT BE CH DE FR GB IT LI LU NL SE

(71) Anmelder: CIBA-GEIGY AG
Patentabteilung Postfach
CH-4002 Basel(CH)

(72) Erfinder: Müller, Beat, Dr.
Vogesenstrasse 31
CH-4106 Therwil(CH)

(54) **Cephalosporinverbindungen, Verfahren zu ihrer Herstellung, sie enthaltende pharmazeutische Zusammensetzungen und ihre Verwendung.**

(57) 7β-Aminoisothiazolylacetamido-3-cephem-4-carbonsäureverbindungen der Formel

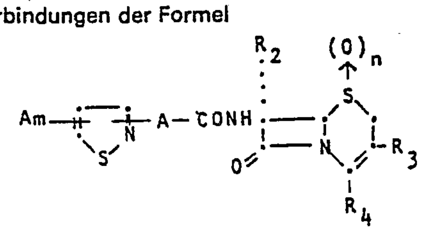

(1),

worin n für 0 oder 1 steht, Am eine gegebenenfalls geschützte Aminogruppe darstellt, A eine Gruppe $-CH_2-$, -C(=O)-, oder $-C(=N-O-R_1)-$ bedeutet, worin $R_1$ für Wasserstoff, gegebenenfalls substituiertes Niederalkyl oder Cycloalkyl, oder gegebenenfalls N-substituiertes Carbamoyl steht, $R_2$ Wasserstoff oder Niederalkoxy bedeutet, $R_3$ für Wasserstoff, veräthertes oder verestertes Hydroxy, veräthertes Mercapto, Formyl oder den Rest der Formel $-CH_2-R_3^{\circ}$ steht, worin $R_3^{\circ}$ für Wasserstoff, Hydroxy, Acyloxy, Heterocyclylthio oder Ammonio steht, und $R_4$ eine gegebenenfalls geschützte Carboxylgruppe darstellt, und Salze von solchen Verbindungen, die eine salzbildende Gruppierung aufweisen, zeigen antibiotische Eigenschaften gegen gram-positive und gram-negative Mikroorganismen. Ebenfalls umfasst sind Zwischenprodukte und Ausgangsstoffe.

EP 0 037 797 A2

CIBA-GEIGY AG  4-12748/+

Basel (Schweiz)

BEZEICHNUNG GEÄNDERT
siehe Titelseite

Isothiazolylverbindungen, Verfahren zu ihrer Herstellung, pharmazeutische Präparate enthaltend solche Verbindungen und Verwendung von letzteren

Die Erfindung betrifft neue 7β-Aminoisothiazolylacetamido-3-cephem-4-carbonsäureverbindungen, Verfahren zu ihrer Herstellung, pharmazeutische Präparate, die solche Verbindungen enthalten und deren Verwendung als Antibiotika.

Die Erfindung betrifft insbesondere 7β-Aminoisothiazolylacetamido-3-cephem-4-carbonsäureverbindungen der Formel

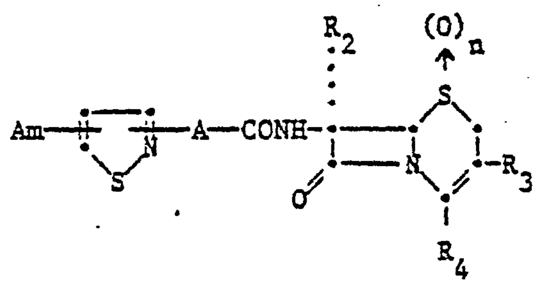

(I),

worin n für 0 oder 1 steht, Am eine gegebenenfalls geschützte Aminogruppe darstellt, A eine Gruppe $-CH_2-$, $-C(=O)-$, oder $-C(=N-O-R_1)-$ bedeutet, worin $R_1$ für Wasserstoff, gegebenenfalls substituiertes Niederalkyl oder Cycloalkyl, oder gegebenenfalls N-substituiertes Carbamoyl steht, $R_2$ Wasserstoff oder Niederalkoxy bedeutet, $R_3$ für Wasserstoff, veräthertes oder verestertes Hydroxy, veräthertes Mercapto, Formyl oder den Rest der Formel $-CH_2-R_3^o$ steht, worin $R_3^o$ für Wasserstoff, Hydroxy, Acyloxy, Heterocyclylthio oder Ammonio steht, und $R_4$ eine gegebenenfalls geschützte Carboxylgruppe darstellt, und Salze von solchen Verbindungen, die eine salzbildende Gruppierung aufweisen, Verfahren zur Herstellung dieser Verbindungen, solche Verbindungen enthaltende pharmazeutische Mittel und deren Verwendung zur Herstellung von pharmakologischen Präparaten oder als pharmakologisch wirksame Verbindungen.

In der obigen Formel I steht n in erster Linie für 0, kann aber auch 1 bedeuten. In letzterem Fall kann die entsprechende Sulfoxidverbindung in der α- oder β-Form vorliegen.

In der vorliegenden Beschreibung der Erfindung bedeutet der im Zusammenhang mit Definitionen von Gruppen und Verbindungen verwendete Ausdruck "nieder", z.B. in Gruppen wie Niederalkyl, Niederalkylen, Niederalkoxy, Niederalkanoyl oder Niederalkylamino, oder in Verbindungen wie niederer Alkohol und dergleichen, dass die entsprechenden Gruppen bzw. Verbindungen, sofern nicht ausdrücklich anders definiert, bis zu 6, bevorzugt bis zu 4 C-Atome enthalten.

Eine gegebenenfalls geschützte Aminogruppe Am steht in 3-, 4- oder bevorzugt in 5-Stellung des Isothiazolringes und ist eine primäre, sekundäre oder tertiäre Aminogruppe, wie Amino, Niederalkylamino oder Diniederalkylamino, wobei die primären oder sekundären Aminogruppen wie weiter unten angegeben geschützt sein können. In einer Niederalkyl- oder Diniederalkylaminogruppe enthält Niederalkyl 1-4 C-Atome und ist beispielsweise Methyl, Aethyl, Propyl oder Butyl. Am ist bevorzugt gegebenenfalls geschütztes Amino oder Methylamino.

Die Gruppe A ist mit ihrem Kohlenstoffatom an die 4-, 5- oder bevorzugt die 3-Stellung des Isothiazolringes gebunden.

Eine Niederalkylgruppe $R_1$ enthält vorzugsweise 1-4 C-Atome und ist beispielsweise Aethyl, Propyl, Butyl oder insbesondere Methyl.

Eine Cycloalkylgruppe $R_1$ enthält vorzugsweise 3-8, in erster Linie 3-6 Ringglieder, und ist z.B. Cyclobutyl, Cyclopentyl oder Cyclohexyl, und insbesondere Cyclopropyl.

Substituenten von substituiertem Niederalkyl oder Cycloalkyl $R_1$ sind u.a. gegebenenfalls veräthertes Hydroxy, z.B. Niederalkoxy, tertiäres Amino, wie Diniederalkylamino, gegebenenfalls funktionell abgewandeltes, inkl. verestertes, amidiertes und geschütztes Carboxyl

oder Sulfo, sowie gegebenenfalls durch Niederalkyl N-substituiertes Ureidocarbonyl. Bevorzugt sind die substituierten Niederalkyl- und Cycloalkylgruppen durch eine Carboxyl- oder Sulfogruppe substituiert, wobei diese bevorzugt am Kohlenstoffatom, das mit dem Sauerstoffatom der Oxyiminogruppe verbunden ist, stehen. Solche substituierte Niederalkyl- und Cycloalkylgruppen $R_1$ sind beispielsweise Carboxymethyl, 1- oder 2-Carboxyäthyl, 1-, 2- oder 3-Carboxyprop-1-yl, 1-, 2- oder 3-Carboxyprop-2-yl, 1-Carboxybut-1-yl, 1-Carboxycycloprop-1-yl und 1-Carboxycyclobut-1-yl, sowie entsprechende durch Sulfo substituierte Niederalkyl- und Cycloalkylgruppen.

Die Carboxy- und Sulfogruppen im Rest $R_1$ können beispielsweise durch Niederalkyl, wie Methyl oder Aethyl, oder eine der physiologisch abspaltbaren Gruppen, z.B. Pivaloyloxymethyl, verestert oder durch ein Amin, wie $NH_3$ oder ein primäres oder sekundäres Amin, wie ein Mono- oder Diniederalkylamin, wie Methyl- oder Aethylamin, oder Dimethyl- oder Diäthylamin amidiert sein, oder wie etwa für $R_4$ angegeben, geschützt sein. In einer Ureidocarbonylgruppe im Rest $R_1$ können die beiden Stickstoffatome unabhängig voneinander durch Niederalkyl, wie Methyl oder Aethyl, substituiert sein. Solche Gruppen sind beispielsweise Ureidocarbonyl oder 1,3-Dimethylureidocarbonyl.

Gegebenenfalls N-substituiertes Carbamoyl als Rest $R_1$ ist z.B. N-niederalkyliertes Carbamoyl, wie N-Methyl- oder N-Aethylcarbamoyl.

Eine Niederalkoxygruppe $R_2$ enthält bevorzugt 1-4 C-Atome und ist beispielsweise Aethoxy, Propoxy, Butoxy oder insbesondere Methoxy.

Veräthertes Hydroxy $R_3$ ist in erster Linie Niederalkoxy, z.B. Methoxy, ferner Aethoxy, n-Propyloxy, Isopropyloxy, n-Butyloxy, Isobutyloxy oder tert.-Butyloxy, sowie n-Pentyloxy, n-Hexyloxy oder n-Heptyloxy, aber auch Arylniederalkoxy, worin Aryl gegebenenfalls z.B. durch Niederalkoxy, Halogen oder Nitro substituiert ist, z.B. Benzyloxy, Diphenylmethoxy, p-Methobenzyloxy oder p-Nitrobenzyloxy.

Verestertes Hydroxy $R_3$ ist durch eine anorganische oder organische Säure verestertes Hydroxy, in erster Linie Halogen, ferner Niederalkanoyloxy, Niederalkylsulfonyloxy oder Arylsulfonyloxy, z.B. insbesondere Chlor, aber auch Fluor, Brom oder Jod, Acetyloxy, Propionyloxy, Methylsulfonyloxy, Aethylsulfonyloxy, oder o-, m- oder p-Toluolsulfonyloxy.

Veräthertes Mercapto $R_3$ ist in erster Linie Heterocyclylthio, worin Heterocyclyl vorzugsweise einen fünf- oder sechsgliedrigen, gegebenenfalls teilweise gesättigten aromatischen Heterocyclylrest darstellt, der mindestens ein Ringstickstoffatom und gegebenenfalls weitere Ringstickstoff-, Ringsauerstoff- und/oder Ringschwefelatome enthält und über ein Ringkohlenstoffatom mit dem Thioschwefel verbunden ist, und der gegebenenfalls substituiert sein kann, z.B. einer der unten genannten Heterocyclylthioreste $R_3^o$. $R_3$ kann aber auch für gegebenenfalls, z.B. durch Hydroxy, Amino oder Carboxy, substituiertes Niederalkylmercapto stehen.

In einer Acyloxygruppe $R_3^o$ ist Acyl beispielsweise der Acylrest einer gegebenenfalls substituierten niederaliphatischen Carbonsäure, einer Niederalkoxycarbonsäure, einer Niederalkansulfonsäure, einer aromatischen Sulfonsäure, einer aromatischen Carbonsäure, einer durch Aryl-substituierten Niederalkancarbonsäure oder einer gegebenenfalls N-substituierten Carbaminsäure.

Solche Acyloxygruppen $R_3^o$ sind beispielsweise Niederalkanoyloxy, insbesondere Acetyloxy, ferner Formyloxy, Propionyloxy, Butyryloxy, Isobutyryloxy, Valeryloxy, Isovaleryloxy, Hexanoyloxy, Heptanoyloxy, Pivaloyloxy, Acetoacetoxy, Oxalyloxy, Succinyloxy und dergleichen, Niederalkoxycarbonyloxy, wie Methoxycarbonyloxy, Aethoxycarbonyloxy, Propoxycarbonyloxy, 1-Cyclopropyläthoxycarbonyloxy, Isopropoxycarbonyloxy, Butoxycarbonyloxy, tert.-Butoxycarbonyloxy, Pentyloxycarbonyloxy, tert.-Pentyloxycarbonyloxy, Hexyloxycarbonyloxy und dergleichen, Niederalkansulfonyloxy, wie Mesyloxy, Aethansulfonyloxy, Propansulfonyloxy , Isopropansulfonyloxy, Butansulfonyloxy und dergleichen, Arensulfonyloxy, wie Benzolsulfonyloxy, Tosyloxy und dergleichen, Aroyl, wie Benzoyl, Toluoyl, Naphthoyl, Phthaloyl, Indancarbonyl und dergleichen, Arylniederalkanoyl, wie Phenylacetyl und dergleichen, wobei der oben angegebene Acylrest einen oder zwei geeignete Substituenten, wie Halogen, z.B. Chlor, Brom, Jod oder Fluor, Hydroxy, Cyano, Nitro, niederes Alkoxy, z.B. Methoxy, Aethoxy, Propoxy, Isopropoxy und dergleichen, niederes Alkyl, z.B. Methyl, Aethyl, Propyl, Isopropyl, Butyl und dergleichen, niederes Alkenyl, z.B. Vinyl, Allyl und dergleichen oder z.B. Phenyl, Tolyl und dergleichen aufweisen kann. Geeignete Beispiele für das Acyloxy mit einem oder mehreren dieser Substituenten sind vorzugsweise Halogenniederalkanoyloxy, z.B. Chloracetyloxy, Dichloracetyloxy, Trichloracetyloxy oder Trifluoracetyloxy.

In dem Acylrest einer gegebenenfalls N-substituierten Carbaminsäure sind N-Substituenten gegebenenfalls durch Halogen, z.B. Chlor, oder durch Niederalkanoyl, z.B. Acetyl oder Propionyl, substituierte Niederalkylreste, z.B. Methyl, Aethyl, 2-Chloräthyl, oder 2-Acetoxyäthyl.Solche Acyloxygruppen $R_3$ sind z.B. Carbamoyloxy, N-Methylcarbamoyloxy, N-Aethylcarbamoyloxy, N-(2-Chloräthyl)-carbamoyloxy oder N-(2-Acetoxyäthyl)-carbamoyloxy.

In einer Heterocyclylthiogruppe $R_3^o$ ist Heterocyclyl insbesondere ein gegebenenfalls substituierter, über ein Ringkohlenstoffatom an die Thiogruppe gebundener, heterocyclischer Rest mit 1 bis 4 Ringstickstoffatomen und gegebenenfalls einem weiteren Ringheteroatom der Gruppe Sauerstoff und Schwefel.

Solche Heterocyclylreste sind in erster Linie gegebenenfalls substituierte, z.B. die unten genannten Substituenten enthaltende, monocyclische, fünf- oder sechsgliedrige diaza-, triaza-, tetraza-, thiaza-, thiadiaza-, thiatriaza-, oxaza- oder oxadiazacyclische Reste aromatischen oder partiell gesättigten Charakters.

Substituenten solcher Heterocyclylreste sind u.a. Niederalkyl, insbesondere Methyl, sowie Aethyl, n-Propyl, Isopropyl oder geradkettiges oder verzweigtes Butyl, oder durch Hydroxy, verestertes Hydroxy, wie Niederalkanoyloxy, Halogen, wie Chlor, Carboxy, verestertes Carboxy, wie Niederalkoxycarbonyl, Sulfo, amidiertes Sulfo, Amino, Mono- oder Diniederalkylamino, Acylamino, wie Niederalkanoylamino, oder durch substituiertes, wie durch Carboxy oder Halogen substituiertes Niederalkanoylamino substituiertes Niederalkyl, beispielsweise 2-Hydroxyäthyl, 2-Acetoxyäthyl, 2-Chloräthyl, Carboxymethyl- 2-Carboxyäthyl, Aethoxycarbonylmethyl, 2-Aethoxycarbonyläthyl, Sulfomethyl, 2-Sulfoäthyl, Sulfamylmethyl, 2-Sulfamyläthyl, 2-Aminoäthyl, 2-Dimethylaminoäthyl oder 2-Acetylaminoäthyl. Weitere Substituenten des heterocyclischen Restes sind Cycloalkyl, z.B. Cyclopentyl oder Cyclohexyl, Aryl, wie gegebenenfalls durch Halogen, z.B. Chlor, oder Nitro substituiertes Phenyl, Arylniederalkyl, z.B. Benzyl, oder Heterocyclyl, wie Furyl, z.B. 2-Furyl, Thienyl, z.B. 2-Thienyl, oder Oxazolyl, z.B. 2- oder 5-Oxazolyl, oder funktionelle Gruppen, wie Halogen, z.B. Fluor, Chlor oder Brom, gegebenenfalls substituiertes Amino, wie gegebenenfalls durch Niederalkyl mono- oder di-substituiertes Amino, z.B. Amino, Methylamino oder Dimethylamino, Acylamino, wie Niederalkanoylamino oder durch Halogen oder Carboxy substituiertes Niederalkanoylamino, wie Acetylamino, 3-Chlorpropionylamino oder 3-Carboxypropionylamino, Nitro, Hydroxy, Niederalkoxy, z.B. Methoxy, Aethoxy, n-Butyloxy oder 2-Aethylhexyloxy, oder gegebenenfalls funktionell abgewandeltes Carboxy, wie Carboxy, verestertes Carboxy, wie Niederalkoxycarbonyl, z.B. Methoxycarbonyl oder Aethoxycarbonyl, gegebenenfalls substituiertes, wie N-mono- oder N,N-diniederalkyliertes Carbamoyl, z.B. N-Methylcarbamoyl oder N,N-Dimethylcarbamoyl, oder Cyan, sowie Oxo oder Oxido, wobei einer oder mehrere solche Substituenten, die in erster Linie mit Ringkohlenstoffatomen aber auch, insbesondere Niederalkyl und Oxido, mit Ringstickstoffatomen verbunden sind, vorhanden sein können.

Bevorzugte Heterocyclylthiogruppen $R_3^o$, worin der heterocyclische Rest einen entsprechenden monocyclischen, fünfgliedrigen Rest darstellt, sind u.a. Imidazolylthio, z.B. 2-Imidazolylthio, gegebenenfalls durch Niederalkyl und/oder Phenyl substituiertes Triazolylthio, z.B. 1H-1,2,3-Triazol-4-ylthio, 1-Methyl-1H-1,2,3-triazol-4-ylthio, 1H-1,2,4-Triazol-3-ylthio, 5-Methyl-1H-1,2,4-triazol-3-ylthio, 3-Methyl-1-phenyl-1H-1,2,4-triazol-5-ylthio, 4,5-Dimethyl-4H-1,2,4-triazol-3-ylthio oder 4-Carboxymethyl- oder 4-Phenyl-4H-1,2,4-triazol-3-ylthio, insbesondere gegebenenfalls wie angegeben substituiertes Tetrazolylthio, z.B. 1H-Tetrazol-5-ylthio, 1-Methyl-1H-tetrazol-5-ylthio, 1-Carboxymethyl-1H-tetrazol-5-ylthio, 1-(2-Carboxyäthyl)-1H-tetrazol-5-ylthio, 1-Sulfomethyl-1H-tetrazol-5-ylthio, 1-(2-Sulfoäthyl)-1H-tetrazol-5-ylthio, 1-(2-Dimethylaminoäthyl)-1H-tetrazol-5-ylthio, 1-Phenyl-1H-tetrazol-5-ylthio oder 1-(4-Chlorphenyl)-1H-tetrazol-5-ylthio, gegebenenfalls durch Niederalkyl oder Thienyl substituiertes Thiazolylthio oder Isothiazolylthio, z.B. 2-Thiazolylthio, 4-(2-Thienyl)-2-thiazolylthio, 4,5-Dimethyl- 2-thiazolylthio, 3-Isothiazolylthio, 4-Isothiazoylthio oder 5-Isothiazolylthio, insbesondere auch gegebenenfalls wie angegeben substituiertes Thiadiazolylthio, z.B. 1,2,3-Thiadiazol-4-ylthio, 1,2,3-Thiadiazol-5-ylthio, 1,3,4-Thiadiazol-2-ylthio, 2-Methyl-1,3,4-thiadiazol-5-ylthio, 2-(3-Carboxypropionylamino)-1,3,4-thiadiazol-5-ylthio, 1,2,4-Thiadiazol-5-ylthio, 3-Methyl-1,2,4-thiadiazol-5-ylthio oder 1,2,5-Thiadiazol-3-ylthio, Thiatriazolylthio, z.B. 1,2,3,4-Thiatriazolyl-5-ylthio, gegebenenfalls wie angegeben substituiertes Oxazolylthio oder Isoxazolylthio, z.B. 5-Oxazolylthio, 4-Methyl-5-oxazolylthio, 2-Oxazolylthio, 4,5-Diphenyl-2-oxazolylthio oder 3-Methyl-5-isoxazolylthio, oder gegebenenfalls wie angegeben substituiertes Oxadiazolylthio, z.B. 1,2,4-Oxadiazol-5-ylthio, 2-Methyl-1,3,4-oxadiazol-5-ylthio, 2-Phenyl-1,3,4-oxadiazol-5-ylthio, 5-(4-Nitrophenyl)-1,3,4-oxadiazol-2-ylthio oder 2-(2-Thienyl)-1,3,4-oxadiazol-5-ylthio.

Bevorzugte Heterocyclylthiogruppen $R_3^o$, worin der heterocyclische Rest einen entsprechenden monocyclischen, sechsgliedrigen Rest oder einen entsprechenden partiell gesättigten Rest darstellt, sind u.a. gegebenenfalls durch Halogen substituiertes 1-Oxidopyridylthio, z.B. 1-Oxido-2-pyridylthio oder 4-Chlor-1-oxido-2-pyridylthio, gegebenenfalls durch Hydroxy substituiertes Pyridazinylthio, z.B. 3-Hydroxy-6-pyridazinylthio, gegebenenfalls durch Niederalkyl, Niederalkoxy oder Halogen substituiertes N-Oxido-pyridazinylthio, z.B. 2-Oxido-6-pyridazinylthio,

3-Chlor-1-oxido-6-pyridazinylthio, 3-Methyl-2-oxido-6-pyridazinylthio, 3-Methoxy-1-oxido-6-pyridazinylthio, 3-Aethoxy-1-oxido-6-pyridazinylthio, 3-n-Butyloxy-1-oxido-6-pyridazinylthio oder 3-(2-Aethylhexyloxy)-1-oxido-6-pyridazinylthio, oder gegebenenfalls durch Niederalkyl, Amino, Diniederalkylamino oder Carboxy substituiertes 2-Oxo-1,2-dihydropyrimidinylthio, z.B. 2-Oxo-1,2-dihydro-4-pyrimidinylthio, 6-Methyl-2-oxo-1,2-dihydro-4-pyrimidinylthio, 5-Methyl-2-oxo-1,2-dihydro-4-pyrimidinylthio, 6-Amino-2-oxo-1,2-dihydro-4-pyrimidinylthio, 6-Dimethylamino-2-oxo-1,2-dihydro-4-pyrimidinylthio, 5-Carboxy-2-oxo-1,2-dihydro-4-pyrimidinylthio oder 6-Carboxy-2-oxo-1,2-dihydro-4-pyrimidinylthio oder gegebenenfalls auf die gleiche Weise substituiertes 4-Oxo-3,4-dihydro-2-pyrimidinylthio, wie insbesondere das unsubstituierte 4-Oxo-3,4-dihydro-2-pyrimidinylthio, oder gegebenenfalls durch Niederalkyl, wie Methyl oder Aethyl, und bis zu zwei Oxo substituiertes Tetrahydrotriazinylthio, insbesondere N-Niederalkyl-5,6-dioxo-1,2,5,6-tetrahydro-1,2,4-triazin-3-ylthio, z.B. 1-, 2- oder 4-Methyl-5,6-dioxo-1,2,5,6-tetrahydro-1,2,4-triazin-3-ylthio, oder die den Oxoverbindungen entsprechenden tautomeren aromatischen Hydroxyverbindungen.

$R_3^o$ als Ammonio ist in erster Linie gegebenenfalls substituiertes Pyridinio, wobei Substituenten z.B. Hydroxy, Niederalkyl, Carboxy oder Carbamoyl sein können. Solche Gruppen sind z.B. Pyridinio, 3-Hydroxypyridinio, 3- oder 4-Methylpyridinio, 3- oder 4-Carboxypyridinio oder 3- oder 4-Carbamoylpyridinio.

Die in Verbindungen der Formel (I) vorhandenen funktionellen Gruppen, insbesondere Carboxyl- und Amino-, ferner Hydroxy- und Sulfogruppen, sind gegebenenfalls durch Schutzgruppen geschützt, die in der Penicillin-, Cephalosporin- und Peptidchemie verwendet werden.

Solche Schutzgruppen sind leicht, das heisst ohne dass unerwünschte Nebenreaktionen stattfinden, beispielsweise solvolytisch, reduktiv, photolytisch oder auch unter physiologischen Bedingungen, abspaltbar.

Schutzgruppen dieser Art, sowie ihre Abspaltung sind beispielsweise beschrieben in "Protective Groups in Organic Chemistry", Plenum Press, London, New York, 1973, ferner in "The Peptides", Vol. I, Schröder and Lübke, Academic Press, London, New York, 1965, sowie in "Methoden der organischen Chemie", Houben-Weyl, 4. Auflage, Bd. 15/1, Georg Thieme Verlag, Stuttgart, 1974.

So sind Carboxylgruppen, wie die Carboxylgruppe $R_4$, z.B. in üblicher Weise in veresterter Form geschützt, wobei solche Estergruppierungen unter schonenden Bedingungen leicht spaltbar sind.

Geeignete geschützte Carboxylgruppen sind beispielsweise Niederalkoxycarbonyl, z.B. Methoxy- oder Aethoxycarbonyl, oder insbesondere tert.-Niederalkoxycarbonyl, z.B. tert.-Butyloxycarbonyl, Polycycloalkoxycarbonyl, z.B. Adamantyloxycarbonyl, Arylmethoxycarbonyl, worin Aryl vorzugsweise einen oder zwei, gegebenenfalls, z.B. durch Niederalkyl, insbesondere tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellt, wie gegebenenfalls, z.B. wie oben erwähnt, substituiertes Benzyloxycarbonyl, z.B. 4-Nitro-benzyloxycarbonyl oder 4-Methoxybenzyloxycarbonyl, oder gegebenenfalls, z.B. wie oben erwähnt, substituiertes Diphenylmethoxycarbonyl, z.B. Diphenylmethoxycarbonyl oder Di-(4-methoxyphenyl)-methoxycarbonyl, oder 2-Halogenniederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, 2-$(S_1)(S_2)(S_3)$-Silyläthoxycarbonyl, worin die Substituenten $S_1$, $S_2$ und $S_3$ unabhängig voneinander je einen gegebenenfalls substituierten, z.B. durch Niederalkyl, Niederalkoxy, Aryl, Halogen oder Nitro substituierten, aliphatischen, araliphatischen, cycloaliphatischen oder aromatischen Kohlenwasserstoffrest mit z.B. bis zu 15 C-Atomen, wie einen entsprechenden gegebenenfalls substituierten Niederalkyl-, Arylniederalkyl-, Cycloalkyl- oder Arylrest, bedeuten, z.B. 2-Triniederalkylsilyläthoxycarbonyl, wie 2-Trimethylsilyläthoxycarbonyl oder 2-(Dibutyl-methylsilyl)-äthoxycarbonyl, oder 2-Triarylsilyläthoxycarbonyl, wie 2-Triphenylsilyläthoxycarbonyl, oder 1-Niederalkoxyniederalkoxycarbonyl, wie Methoxymethoxy-

carbonyl, 1-Methoxyäthoxycarbonyl oder 1-Aethoxymethoxycarbonyl, oder 1-Niederalkylthioniederalkoxycarbonyl, wie 1-Methylthiomethoxycarbonyl oder 1-Aethylthioäthoxycarbonyl oder Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, oder Polyhalogenaryloxycarbonyl, wie Pentachlorphenyloxycarbonyl. Veresterte Carboxylgruppen sind ebenfalls entsprechende Silyloxycarbonyl-, insbesondere organische Silyloxycarbonylgruppen oder entsprechende Stannyloxycarbonylgruppen. In diesen enthält das Silicium- bzw. Zinnatom vorzugsweise Niederalkyl, insbesondere Methyl, ferner Niederalkoxy, z.B. Methoxy, und/oder Halogen, z.B. Chlor, als Substituenten. Geeignete Silyl- bzw. Stannylschutzgruppen sind in erster Linie Triniederalkylsilyl, insbesondere Trimethylsilyl, ferner Dimethyl-tert.-butylsilyl, Niederalkoxy-niederalkyl-halogen-silyl, z.B. Methoxy-methyl-chlor-silyl, oder Diniederalkyl-halogensilyl, z.B. Dimethylchlor-silyl, oder entsprechend substituierte Stannylverbindungen, z.B. Tri-n-butyl-stannyl.

Bevorzugte geschützte Carboxylgruppen sind tert.-Butyloxycarbonyl, gegebenenfalls, z.B. wie erwähnt, substituiertes Benzyloxycarbonyl, z.B. 4-Nitrobenzyloxycarbonyl und Diphenylmethoxycarbonyl.

Eine unter physiologischen Bedingungen spaltbare, veresterte Carboxylgruppe ist in erster Linie eine Acyloxymethoxycarbonylgruppe, worin Acyl z.B. den Rest einer organischen Carbonsäure, in erster Linie einer gegebenenfalls substituierten Niederalkancarbonsäure bedeutet, oder worin Acyloxymethyl den Rest eines Lactons bildet. Solche Gruppen sind Niederalkanoyloxymethoxycarbonyl, z.B. Acetyloxymethyloxycarbonyl oder Pivaloyloxymethoxycarbonyl, Aminoniederalkanoyloxymethoxycarbonyl, insbesondere $\alpha$-Amino-niederalkanoyloxymethoxycarbonyl, z.B. Glycyloxymethoxycarbonyl, L-Valyloxymethoxycarbonyl oder L-Leucyloxymethoxycarbonyl, ferner Phthalidyloxycarbonyl, z.B. 2-Phthalidyloxycarbonyl, oder Indanyloxycarbonyl, z.B. 5-Indanyloxycarbonyl.

Eine geschützte Aminogruppe, wie die Gruppe Am, kann z.B. in Form einer leicht spaltbaren Acylamino-, Mono-, Di- oder Triarylmethylamino-,

verätherten Mercaptoamino-, 1-Acyl-2-niederalkylidenamino-, Silyl- oder Stannylaminogruppe oder als Azidogruppe vorliegen.

In einer entsprechenden Acylaminogruppe ist Acyl beispielsweise der Acylrest einer organischen Carbonsäure mit z.B. bis zu 18 C-Atomen, insbesondere einer gegebebenfalls, z.B. durch Halogen oder Aryl, substituierten aliphatischen Carbonsäure oder gegebenenfalls, z.B. durch Halogen, Niederalkoxy oder Nitro, substituierten aromatischen Carbonsäure, oder eines Kohlensäurehalbesters. Solche Acylgruppen sind beispielsweise Niederalkanoyl, wie Formyl, Acetyl, Propionyl, Halogenniederalkanoyl, wie 2-Halogenacetyl, insbesondere 2-Chlor-, 2-Brom-, 2-Jod-, 2,2-Dichlor- oder 2,2,2-Trichloracetyl, Phenylacetyl, Phenoxyacetyl, Thienylacetyl, Benzoyl, 4-Chlor-, 4-Methoxy- oder 4-Nitrobenzoyl, Niederalkoxycarbonyl, insbesondere tert.-Niederalkoxycarbonyl, z.B. tert.-Butyloxycarbonyl, Polycycloalkoxycarbonyl, z.B. Adamantyloxycarbonyl, Arylmethoxycarbonyl, worin Aryl vorzugsweise einen oder zwei, gegebenenfalls, z.B. durch Niederalkyl, insbesondere tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellt, wie gegebenenfalls substituiertes Benzyloxycarbonyl, z.B. 4-Nitro-benzyloxycarbonyl, oder substituiertes Diphenylmethoxycarbonyl, z.B. Benzhydryloxycarbonyl oder Di-(4-methoxyphenyl)-methoxycarbonyl, oder 2-Halogenniederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, 2-$(S_1)(S_2)(S_3)$-Silyläthoxycarbonyl, worin die Substituenten $S_1$, $S_2$ und $S_3$ unabhängig voneinander je einen gegebenenfalls substituierten, z.B. durch Niederalkyl, Niederalkoxy, Aryl, Halogen oder Nitro, substituierten, aliphatischen, araliphatischen, cycloaliphatischen oder aromatischen Kohlenwasserstoffrest mit z.B. bis zu 15 C-Atomen, wie einen entsprechenden gegebenenfalls substituierten Niederalkyl-, Arylniederalkyl-, Cycloalkyl- oder Arylrest, bedeuten, z.B. 2-Triniederalkylsilyläthoxycarbonyl, wie 2-Trimethylsilyläthoxycarbonyl oder 2-(Dibutyl-methyl-silyl)-äthoxycarbonyl, oder 2-Triarylsilyläthoxycarbonyl, wie 2-Triphenylsilyläthoxycarbonyl, oder Aroylmethoxycarbonyl, worin die Aroylgruppe vorzugsweise gegebenenfalls, z.B. durch Halogen, wie Brom, substituiertes Benzoyl, darstellt, z.B. Phenacyloxycarbonyl.

Weitere Acylgruppen in Acylaminogruppen sind Reste organischer Phosphor-, Phosphon- oder Phosphinsäuren der Formel $(R^1)(R^2)P(=O)-$, worin $R^1$ und $R^2$ unabhängig voneinander eine durch einen Kohlenwasserstoffrest verätherte Hydroxygruppe oder einen Kohlenwasserstoffrest bedeuten, wobei die Kohlenwasserstoffreste bevorzugt bis zu 15 C-Atome enthalten, beispielsweise aliphatischer, araliphatischer, cycloaliphatischer oder aromatischer Natur sind, gegebenenfalls z.B. durch Niederalkyl, Niederalkoxy, Aryl, Halogen oder Nitro substituiert sind, und beispielsweise Alkyl, insbesondere Niederalkyl, wie Methyl, Aethyl, Propyl, Isopropyl oder Butyl, Phenylniederalkyl, wie Benzyl, p-Nitro- oder p-Chlorbenzyl, Cycloalkyl, wie Cyclopropyl, Cyclopentyl, Cyclohexyl oder Cycloheptyl, oder Homoaryl, wie Phenyl, o-, m- oder p-Tolyl, p-Methoxyphenyl, p-Biphenylyl, p-Chlorphenyl oder p-Nitrophenyl, bedeuten. Solche Reste sind beispielsweise Dimethylphosphoryl, Diäthylphosphoryl, Dipropylphosphoryl, Diisopropylphosphoryl, Dibenzylphosphoryl, Di-p-nitrobenzylphosphoryl,. Dicyclohexylphosphoryl, Diphenylphosphoryl, Phenoxy-phenyl-phosphonyl, Diäthylphosphinyl und Diphenylphosphinyl.

In einer Mono-, Di- oder Triarylmethylaminogruppe sind die Arylreste insbesondere gegebenenfalls substituierte Phenylreste. Solche Gruppen sind beispielsweise Benzyl-, Diphenylmethyl- oder Tritylamino.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z.B. 4-Nitrophenylthio.

In einem als Aminoschutzgruppe verwendbaren 1-Acyl-2-niederalkylidenrest ist Acyl vorzugsweise der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie

Chlor, und/oder Nitro substituierten Benzoesäure oder eines Kohlensäurehalbesters, wie eines Kohlensäure-niederalkylhalbesters. Entsprechende Schutzgruppen sind in erster Linie 1-Niederalkanoyl-2-propyliden, z.B. 1-Acetyl-2-propyliden, oder 1-Niederalkoxycarbonyl-2-propyliden, z.B. 1-Aethoxycarbonyl-2-propyliden.

Eine Silyl- oder Stannylaminogruppe ist in erster Linie eine organische Silyl- bzw. Stannylaminogruppe, worin das Silicium- bzw. Zinnatom vorzugsweise Niederalkyl, insbesondere Methyl, ferner Niederalkoxy, z.B. Methoxy, und/oder Halogen, z.B. Chlor, als Substituenten enthält. Entsprechende Silyl- oder Stannylgruppen sind in erster Linie Triniederalkylsilyl, insbesondere Trimethylsilyl, ferner Dimethyl-tert.-butyl-silyl, Niederalkoxy-niederalkyl-halogen-silyl, z.B. Methoxy-methyl-chlor-silyl, oder Diniederalkyl-halogensilyl, z.B. Dimethyl-chlorsilyl, oder entsprechend substituiertes Stannyl, z.B. Tri-n-butylstannyl.

Eine Aminogruppe kann auch in protonierter Form geschützt werden; als Anionen kommen in erster Linie diejenigen von starken anorganischen Säuren, wie von Halogenwasserstoffsäuren, z.B. das Chlor- oder Bromanion, oder von Sulfonsäuren, wie p-Toluolsulfonsäure, in Frage.

Bevorzugte Aminoschutzgruppen sind die Acylreste von Kohlensäurehalbestern, insbesondere tert.-Butyloxycarbonyl, gegebenenfalls, z.B wie angegeben, substituiertes Benzyloxycarbonyl oder Diphenylmethoxycarbonyl, oder 2-Halogen-niederalkoxycarbonyl, wie 2,2,2-Trichloräthoxycarbonyl.

Hydroxyschutzgruppen sind z.B. Acylreste, wie 2,2-Dichloracetyl oder insbesondere die im Zusammenhang mit einer geschützten Aminogruppe genannten Acylreste von Kohlensäurehalbestern, insbesondere 2,2,2-Trichloräthoxycarbonyl, oder organischen Silyl- oder Stannylreste, ferner leicht abspaltbare 2-oxa- oder 2-thia-aliphatische oder -cycloaliphatische Kohlenwasserstoffreste, in erster Linie 1-Niederalkoxy-niederalkyl oder 1-Niederalkylthio-niederalkyl, z.B. Methoxymethyl, 1-Methoxyäthyl, 1-Aethoxyäthyl, 1-Methylthiomethyl, 1-Methylthio-

äthyl oder 1-Aethylthioäthyl, oder 2-Oxa- oder 2-Thiacycloniederalkyl mit 5-7 Ringatomen, z.B. 2-Tetrahydrofuryl oder 2-Tetrahydropyranyl oder entsprechende Thiaanaloge, sowie leicht abspaltbare, gegebenenfalls substituierte α-Phenylniederalkylreste, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Eine geschützte Sulfogruppe ist in erster Linie eine mit einem aliphatischen, cycloaliphatischen, cycloaliphatisch-aliphatischen, aromatischen oder araliphatischen Alkohol, wie einem Niederalkanol, oder mit einem Silyl- oder Stannylrest, wie Triniederalkylsilyl, veresterte Sulfogruppe. In einer Sulfogruppe kann die Hydroxygruppe beispielsweise wie die Hydroxygruppe in einer veresterten Carboxygruppe veräthert sein.

Salze sind insbesondere diejenigen von Verbindungen der Formel I mit sauren Gruppen, z.B. mit freien Carboxyl- und Sulfogruppen. Solche Salze sind in erster Linie Metall- oder Ammoniumsalze, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen, wie Niederalkylamine, z.B. Triäthylamin, Hydroxyniederalkylamine, z.B. 2-Hydroxyäthylamin, Bis-(2-hydroxyäthyl)-amin oder Tris-(2-hydroxyäthyl)-amin, basische aliphatische Ester von Carbonsäuren, z.B. 4-Aminobenzoesäure-2-diäthylaminoäthylester, Niederalkylenamine, z.B. 1-Aethyl-piperidin, Cycloalkylamine, z.B. Dicyclohexylamin, oder Benzylamine, z.B. N,N'-Dibenzyl-äthylendiamin, ferner Basen vom Pyridintyp, z.B. Pyridin, Collidin oder Chinolin. Verbindungen der Formel I mit einer basischen Gruppe können Säureadditionssalze, z.B. mit anorganischen Säuren, wie Salzsäure, Schwefelsäure oder Phosphorsäure, oder mit geeigneten organischen Carbon- oder Sulfonsäuren, z.B. Trifluoressigsäure, sowie mit Aminosäuren, wie Arginin und Lysin bilden. Bei Anwesenheit von mehreren sauren oder basischen

Gruppen können Mono- oder Polysalze gebildet werden. Verbindungen der Formel I mit einer freien Carboxylgruppe und freien Aminogruppe können auch in Form von inneren Salzen, d.h. in zwitterionischer Form, vorliegen, oder es kann ein Teil des Moleküls als inneres Salz, und ein anderer Teil als normales Salz vorliegen.

Zur Isolierung oder Reinigung können auch pharmazeutisch ungeeignete Salze Verwendung finden. Zur therapeutischen Anwendung gelangen nur pharmazeutisch unbedenkliche Salze, die deshalb bevorzugt werden.

Die Gruppe $=N-O-R_1$ kann in syn- (Z-) oder anti-(E-) Form vorliegen, wobei die syn-Form bevorzugt ist.

Die Aminoisothiazolylverbindungen der vorliegenden Erfindung können als 5-Aminoisothiazol-3-yl-, 5-Aminoisothiazol-4-yl-, 4-Aminoisothiazol-3-yl-, 4-Aminoisothiazol-5-yl-, 3-Aminoisothiazol-4-yl- oder 3-Aminoisothiazol-5-yl-verbindungen vorliegen, wobei die 5-Aminoisothiazol-3-ylverbindungen bevorzugt sind.

Die Aminoisothiazolylverbindungen, worin Am eine primäre oder sekundäre Aminogruppe ist, können gegebenenfalls in den dazu tautomeren Formen vorliegen, beispielsweise können die 3- und 5-Aminoisothiazolylverbindungen in ihren tautomeren Formen die 3- oder 5-Iminoisothiazolinylgruppen der Teilformeln

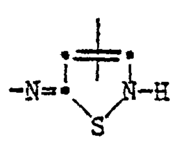

oder

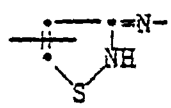

oder eine Mischung beider Formen enthalten. Das Gleichgewicht zwischen den beiden Tautomeren hängt von der Art der Substituenten und äusseren Faktoren, wie Temperatur, Lösungsmittel oder pH-Wert, ab. Im Rahmen der

BAD ORIGINAL

vorliegenden Erfindung wird diese Gruppe in der Beschreibung und in den Ansprüchen nur als "Aminoisothiazolyl" bezeichnet, obgleich die Iminoisothiazolinylform ebenfalls umfasst werden soll.

Die Verbindungen der Formel I, worin n den Wert 0 hat und Schutzgruppen entfernt sind, worin aber Carboxylgruppen gegebenenfalls in phyiologisch spaltbarer Form verestert sind, und ihre pharmazeutisch verwendbaren, nichttoxischen Salze sind wertvolle, antibiotisch wirksame Substanzen, die insbesondere als antibakterielle Antibiotika verwendet werden können. Beispielweise sind sie in vitro gegen gram-positive und gram-negative Mikroorganismen, wie gegen Kokken, z.B. gegen Staphylococcus aureus, Streptococcus spp., Neisseria gonorrhea und Neisseria meningitis in Minimalkonzentrationen von 0,01 bis 32 µg/ml, gegen Anaerobes, z.B. Clostridium, in Minimalkonzentrationen von etwa 0,005 bis etwa 16 µg/ml, gegen Enterobakterien, z.B. Escherichia coli, Salmonella, Klebsiella, Enterobacter, Proteus spp. und Serratia, sowie gegen Haemophilus und Pseudomonas in Minimalkonzentrationen von etwa 0,01 bis etwa 32 µg/ml wirksam. In vivo, bei subcutaner Applikation an der Maus, sind sie bei systemischen Infektionen wirksam, z.B. bei durch gram-positive Kokken, z.B. Staphylococcus aureus, verursachten, im Dosisbereich von etwa 10 bis etwa 80 mg/kg, und bei durch gram-negative Bakterien, z.B. Enterobakterien, verursachten im Dosisbereich von etwa 5 bis etwa 100 mg/kg. Bei der systemischen Infektion mit Escherichia coli an der Maus liegt die $ED_{50}$ bei etwa 4 mg/kg bis 30 mg/kg s.c.

Die neuen Verbindungen können deshalb entsprechend, z.B. in Form von antibiotisch wirksamen Präparaten zur Behandlung von durch gram-positive oder gram-negative Bakterien und Kokken, insbesondere durch von Enterobakterien, wie Escherichia coli, verursachten Infektionen Verwendung finden.

Verbindungen der Formel I, worin die funktionellen Gruppen geschützt sind, inklusiv die 1-Oxide, worin funktionelle Gruppen frei sind, werden als Ausgangsmaterialien zur Herstellung von Verbindungen der Formel I verwendet, worin n den Wert 0 hat.

Die vorliegende Erfindung betrifft in erster Linie diejenigen Verbindungen der Formel I, worin n für 0 oder 1 steht, Am Amino oder Methylamino bedeutet, A eine Gruppe $-CH_2-$, $-C(=O)-$ oder $-C(=N-OR_1)-$ bedeutet, worin $R_1$ für Wasserstoff, Niederalkyl, insbesondere Methyl, durch Carboxy substituiertes Niederalkyl oder Cycloalkyl, insbesondere 2-Carboxy-2-propyl oder 1-Carboxy-1-cyclopropyl, oder für gegebenenfalls N-niederalkyliertes Carbamoyl steht, $R_2$ Wasserstoff oder Methoxy darstellt, $R_3$ für Wasserstoff, Niederalkoxy, Halogen, Niederalkylthio, Heterocyclylthio, worin Heterocyclyl für einen fünf- oder sechsgliedrigen monocyclischen, gegebenenfalls partiell gesättigten, aromatischen Heterocyclylrest steht, der mindestens 1 Ringstickstoffatom und gegebenenfalls 1-3 zusätzliche Ringstickstoffatome und/oder ein Ringsauerstoff- oder Ringschwefelatom enthält, und der durch gegebenenfalls Substituenten, wie Hydroxy, Amino, Diniederalkylamino, Carboxy, Carbamoyl oder Sulfo enthaltendes Niederalkyl, durch gegebenenfalls Substituenten, z.B. Niederalkyl, Niederalkoxy und/oder Halogen, enthaltendes Phenyl, oder durch Thienyl substituiert sein kann, oder für den Rest der Formel $-CH_2-R_3^o$ steht, worin $R_3^o$ Niederalkanoyloxy, wie Acetoxy, Carbamoyloxy, N-Niederalkylcarbamoyloxy, gegebenenfalls substituiertes, mit einem Ringkohlenstoffatom an die Thiogruppe gebundenes Heterocyclylthio, wie Triazolylthio, Tetrazolylthio, Thiazolylthio, Thiatriazolylthio, Thiadiazolylthio, Oxazolylthio, Oxadiazolylthio, Pyridazinylthio, Pyrimidinylthio oder Triazinylthio, worin die heterocyclischen Ringe partiell hydriert und gegebenenfalls beispielsweise durch Niederalkyl, wie Methyl, N,N-Diniederalkylaminoniederalkyl, wie 2-N,N-Dimethylaminoäthyl, Carboxyniederalkyl, wie Carboxymethyl oder 2-Carboxyäthyl, Sulfoniederalkyl, wie Sulfomethyl oder 2-Sulfoäthyl, Amino, Carboxyniederalkanoylamino, Carbamoyl, Oxo oder Hydroxy substituiert sein können, oder gegebenenfalls durch Carbamoyl substituiertes Pyridinio bedeutet, und $R_4$ Carboxyl oder in physiologisch spaltbarer Form verestertes Carboxyl, z.B. Acyloxymethoxycarbonyl, darstellt, insbesondere die entsprechenden 5-Am-isothiazol-3-ylverbindungen, pharmazeutisch verwendbare Salze von solchen Verbindungen, sowie die zu ihrer Herstellung verwendbaren Ausgangsstoffe und Zwischenprodukte.

Besonders hervorzuheben sind Verbindungen der Formel I, worin n für O steht, Am Amino oder Methylamino bedeutet, A eine Gruppe der Formel $-C(=N-O-R_1)$ bedeutet, worin $R_1$ für Wasserstoff, Methyl, 2-Carboxy-2-propyl oder 1-Carboxy-1-cyclopropyl steht, $R_2$ Wasserstoff oder Methoxy darstellt, $R_3$ für Wasserstoff, Niederalkoxy, z.B. Methoxy, Halogen, z.B. Chlor, Heterocyclylthio, worin Heterocyclyl gegebenenfalls durch Niederalkyl, z.B. Methyl, substituiertes Oxadiazolyl oder Thiadiazolyl, wie 1,3,4-Oxadiazol-2-yl oder 1,3,4-Thiadiazol-2-yl, gegebenenfalls durch Niederalkyl, z.B. Methyl, substituiertes Triazolyl, wie 1,2,3-Triazol-4-yl, oder gegebenenfalls durch Niederalkyl, z.B. Methyl oder Aethyl, das Diniederalkylamino, z.B. Dimethylamino, Carboxy, Carbamoyl oder Sulfo als Substituenten enthalten kann, substituiertes 1H-Tetrazol-5-yl bedeutet, oder einen Rest der Formel $-CH_2-R_3^o$ steht, worin $R_3^o$ Acetoxy, Carbamoyloxy, gegebenenfalls durch Niederalkyl oder Carboxyniederalkyl substituiertes Triazolylthio, wie 4-Carboxymethyl-1,2,4-triazolylthio, gegebenenfalls durch Niederalkyl, Carboxyniederalkyl, Sulfoniederalkyl oder Diniederalkylaminoniederalkyl substituiertes Tetrazolylthio, z.B. 1-Methyl-1H-tetrazol-5-ylthio, 1-Sulfomethyl-1H-tetrazol-5-ylthio, 1(2-Sulfoäthyl)-1H-tetrazol-5-ylthio, 1-Carboxymethyl-1H-tetrazol-5-ylthio, 1-(2-Dimethylaminoäthyl)-1H-tetrazol-5-ylthio, gegebenenfalls durch Niederalkyl substituiertes Thiadiazolylthio, z.B. 2-Methyl-1,3,4-thiadiazol-5-ylthio oder 3-Methyl-1,2,4-thiadiazol-5-ylthio, gegebenenfalls durch Hydroxy substituiertes Pyridazinylthio, z.B. 3-Hydroxy-pyridazin-6-ylthio, gegebenenfalls durch Hydroxy substituiertes Pyrimidinylthio, z.B. 4-Hydroxy-2-pyrimidinyl- bzw. das dazu tautomere 4-Oxo-3,4-dihydro-2-pyrimdinylthio, durch Niederalkyl und zwei Hydroxy substituiertes Triazinylthio, wie 2-Niederalkyl-5,6-dihydroxy-1,2,4-triazin-3-ylthio, bzw. dazu tautomeres 1,2,5,6-Tetrahydro-2-niederalkyl-5,6-dioxo-1,2,4-triazin-3-yl-thio, z.B. 1,2,5,6-Tetrahydro-2-methyl-5,6-dioxo-1,2,4-triazinyl-3-ylthio, oder durch Carbamoyl substituiertes Pyridinio, z.B. 4-Carbamoylpyridinio, darstellt und $R_4$ Carboxyl oder in physiologisch spaltbarer Form verestertes Carboxyl, z.B. Pivaloyloxymethoxycarbonyl, oder Phthalidyloxycarbonyl, bedeutet, insbesondere die entsprechenden 5-Am-isothiazol-3-ylverbindungen, pharmazeutisch verwendbare Salze von solchen Verbindungen, sowie die zu ihrer Herstellung verwendbaren Ausgangsstoffe und Zwischenprodukte.

Die Erfindung betrifft insbesondere Verbindungen der Formel I, worin n für 0 steht, Am in 5-Stellung des Isothiazolringes steht und Amino bedeutet, A mit der 3-Stellung des Isothiazolringes verknüpft ist und eine Gruppe $-C(=N-O-R_1)-$in syn(Z)-Konfiguration bedeutet, worin $R_1$ für Methyl steht, $R_2$ Wasserstoff darstellt, $R_3$ für Wasserstoff oder eine Gruppe $-CH_2-R_3^o$ steht, worin $R_3^o$ Niederalkanoyloxy, wie Acetoxy, gegebenenfalls durch Methyl oder Carboxyniederalkyl, wie Carboxymethyl substituiertes Tetrazolylthio, z.B. 1-Methyl-1H-tetrazol-5-ylthio oder 1-Carboxymethyl-1H-tetrazol-5-ylthio, oder Carbamoylpyridinio, z.B. 4-Carbamoylpyridinio, darstellt und $R_4$ Carboxyl bedeutet und pharmazeutisch verwendbare Salze von solchen Verbindungen.

Die Erfindung betrifft insbesondere die in den Beispielen beschriebenen Verbindungen der Formel I, deren pharmazeutisch unbedenkliche Salze, sowie die dort beschriebenen neuen Ausgangstoffe und Zwischenprodukte.

Die Verbindungen der vorliegenden Erfindung werden nach an sich bekannten Verfahren erhalten.

Verbindungen der Formel I und Salze von solchen Verbindungen, die eine salzbildende Gruppe aufweisen, werden beispielsweise hergestellt, indem man

a) in einer Verbindung der Formel

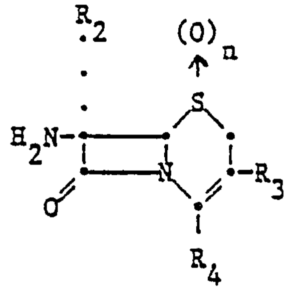

(II),

worin die 7β-Aminogruppe gegebenenfalls durch eine die Acylierung erlaubende Gruppe substituiert ist, und worin weitere gegebenenfalls vorhandene reaktionsfähige funktionelle Gruppen geschützt sein können, die 7β-Aminogruppe durch Behandeln mit einem den Acylrest einer Carbonsäure der Formel

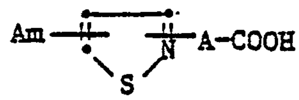 (III)

einführenden Acylierungsmittel, worin Am und A die oben angegebenen Bedeutungen haben, worin weitere gegebenenfalls vorhandene reaktionsfähige funktionelle Gruppen geschützt sein können, acyliert oder

b) eine 2-Cephemverbindung der Formel

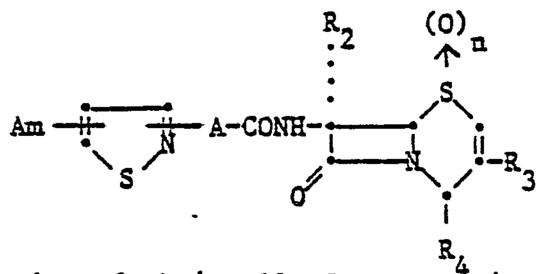 (IV),

worin gegebenenfalls vorhandene funktionelle Gruppen in geschützter Form vorliegen können, zur entsprechenden 3-Cephemverbindung isomerisiert, oder

c) zur Herstellung einer Verbindung der Formel (I), worin A eine Gruppe der Formel -C(=O)- ist, eine Verbindung der Formel

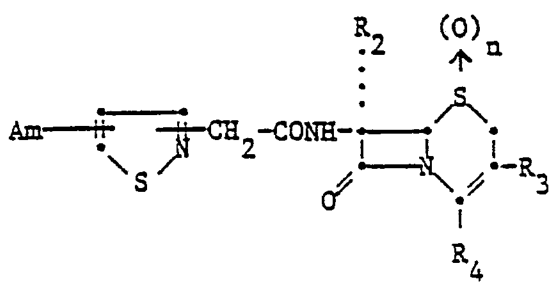 (Ic),

worin gegebenenfalls vorhandene funktionelle Gruppen in geschützter Form vorliegen können, mit einem geeigneten Oxidationsmittel behandelt, oder

d) zur Herstellung einer Verbindung der Formel (I), worin A eine Gruppe der Formel $-C(=N-O-R_1)-$ ist, eine Verbindung der Formel

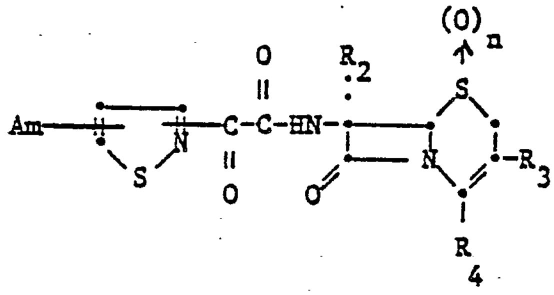

(Id),

worin gegebenenfalls vorhandene funktionelle Gruppen in geschützter Form vorliegen können, mit einem gegebenenfalls O-substituierten Hydroxylamin der Formel $H_2N-O-R_1$ (V) umsetzt, oder

e) zur Herstellung einer Verbindung der Formel (I), worin A eine Gruppe der Formel $-C(=N-O-R_1)-$ ist, worin $R_1$ Wasserstoff bedeutet, eine Verbindung der Formel

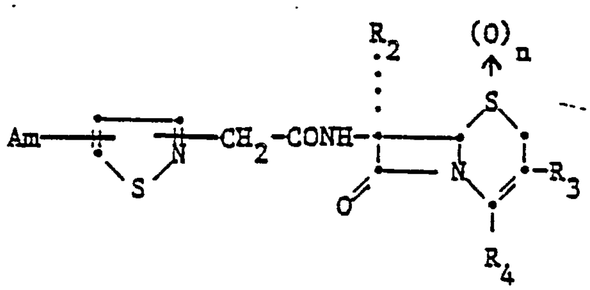

(Ie),

worin gegebenenfalls vorhandene funktionelle Gruppen in geschützter Form vorliegen können mit einem Nitrosierungsmittel behandelt, oder

f) zur Herstellung einer Verbindung der Formel (I), worin A eine Gruppe der Formel $-C(=N-O-R_1)-$ ist, worin $R_1$ gegebenenfalls substituiertes Niederalkyl bedeutet, in einer Verbindung der Formel

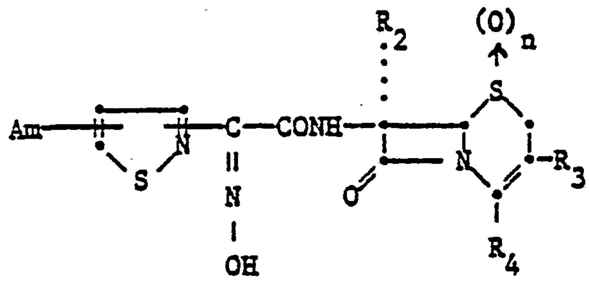

(If),

worin weitere gegebenenfalls vorhandene reaktionsfähige, funktionelle Gruppen geschützt sein können, die Hydroxyiminogruppe mit einem den gegebenenfalls substituierten Niederalkylrest $R_1$ einführenden Alkylierungsmittel behandelt, oder

g) zur Herstellung einer Verbindung der Formel (I), worin $R_2$ Niederalkoxy bedeutet, in eine Verbindung der Formel

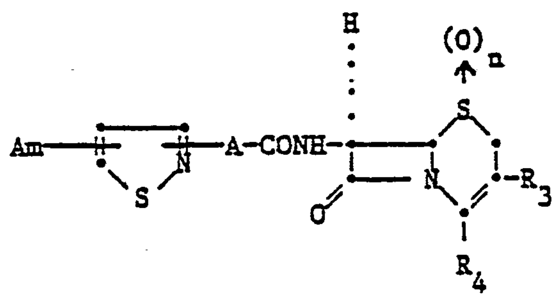

(Ig),

worin funktionelle Gruppen in geschützter Form vorliegen können, in 7α-Stellung eine Niederalkoxygruppe einführt, oder

h) zur Herstellung einer Verbindung der Formel (I), worin $R_3$ eine Gruppe der Formel $-CH_2-R_3^o$ bedeutet, worin $R_3^o$ für Heterocyclthio steht, eine Verbindung der Formel

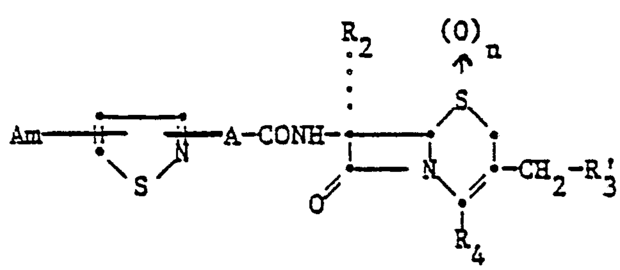

(Ih),

worin weitere gegebenenfalls vorhandene reaktionsfähige funktionelle Gruppen geschützt sein können, und $R_3'$ einen durch einen Heterocyclylthiorest $R_3^o$ ersetzbaren Rest bedeutet, mit einem Mercaptan $H-R_3^o$, behandelt, oder

i) zur Herstellung einer Verbindung der Formel (I), worin $R_3^o$ für Acyloxy steht, eine Verbindung der Formel

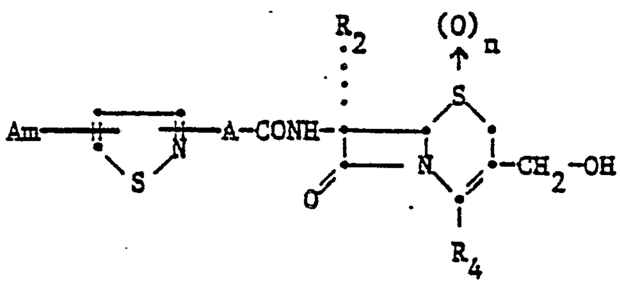

(Ii),

worin weitere gegebenenfalls vorhandene reaktionsfähige funktionelle Gruppen geschützt sein können, mit einem Acylierungsmittel behandelt, oder

j) zur Herstellung einer Verbindung der Formel (I), worin $R_3$ den Rest der Formel $-CH_2-R_3^o$ darstellt, worin $R_3^o$ eine Ammoniogruppe bedeutet, eine Verbindung der Formel

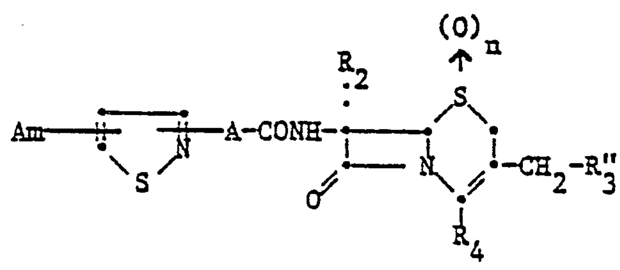

(Ij),

worin $R_3''$ einen durch nucleophile Substitution ersetzbaren Rest darstellt, und worin gegebenenfalls vorhandene funktionelle Gruppen in geschützter Form vorliegen können, mit einer organischen tertiären Aminbase umsetzt, oder

k) zur Herstellung einer Verbindung der Formel (I), worin $R_3$ für Wasserstoff steht,

ka) in einer Verbindung der Formel

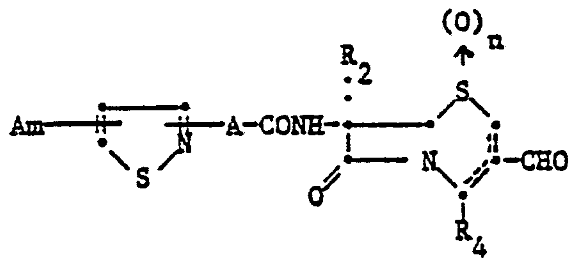

(Ika)

worin die Doppelbindung in 2,3- oder in 3,4-Stellung sein kann, und worin gegebenenfalls vorhandene funktionelle Gruppen in geschützter Form vorliegen können, die Formylgruppe mittels Decarbonylierung durch Wasserstoff ersetzt, oder

kb) in einer Verbindung der Formel

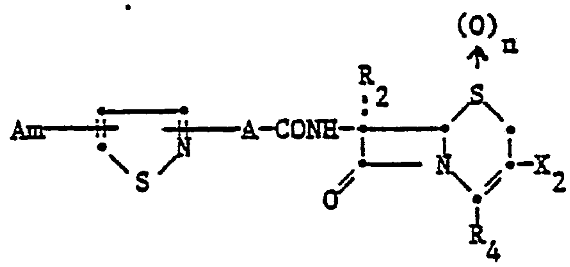

(Ikb)

worin $X_2$ Hydroxy oder gegebenenfalls substituiertes Amino darstellt, und worin weitere gegebenenfalls vorhandene funktionelle Gruppen in geschützter Form vorliegen können, oder eine entsprechende tautomere Verbindung davon, $X_2$ durch Wasserstoff ersetzt, oder

l) zur Herstellung einer Verbindung der Formel I, worin $R_3$ für veräthertes oder verestertes Hydroxy steht, eine Verbindung der Formel

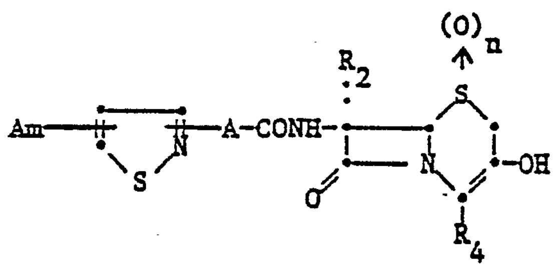

(Il),

oder die entsprechende tautomere 3-Oxo-cepham-Verbindung, worin $R_4$ funktionell abgewandeltes Carboxy darstellt, und weitere gegebenenfalls vorhandene funktionelle Gruppen in geschützter Form vorliegen können, mit einem veräthernden oder veresternden Mittel behandelt, oder

m) zur Herstellung von Verbindungen der Formel (I), worin Am eine primäre, sekundäre oder tertiäre Aminogruppe ist, eine Verbindung der Formel

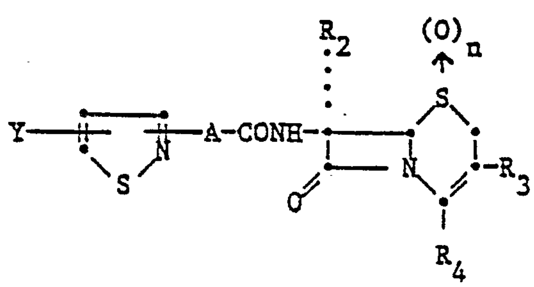

(Im),

worin y Halogen ist, worin weitere gegebenenfalls vorhandene reaktionsfähige funktionelle Gruppen geschützt sein können, mit Ammoniak oder einem primären oder sekundären Amin Am-H, oder einem Metallamid davon umsetzt, und wenn erwünscht, eine erfindungsgemäss erhältliche Verbindung der Formel I in eine andere Verbindung der Formel I umwandelt, und/oder, wenn erwünscht oder notwendig, in einer erfindungsgemäss erhältlichen Verbindung der Formel I eine in geschützter Form vorliegende funktionelle Gruppe in die freie

funktionelle Gruppe überführt, und/oder, wenn erwünscht, ein erfindungsgemäss erhältliches Salz in die freie Verbindung oder in ein anderes Salz überführt, und/oder, wenn erwünscht, eine erfindungsgemäss erhältliche freie Verbindung mit einer salzbildenden Gruppe in ein Salz überführt, und/oder, wenn erwünscht, ein erfindungsgemäss erhältliches Gemisch von isomeren Verbindungen der Formel I in die einzelnen Isomeren auftrennt.

Verfahren a) (Acylierung): Gegebenenfalls vorhandene, die Aminogruppe substituierende und deren Acylierung erlaubende Reste in einem Ausgangsmaterial der Formel II sind beispielsweise organische Silyl- oder Stannylgruppen, ferner auch Ylidengruppen, die zusammen mit der Aminogruppe eine Schiff'sche Base bilden. Die genannten organischen Silyl- oder Stannylgruppen sind z.B. die gleichen, die auch mit der 4-Carboxylgruppe am Cephemring eine geschützte Carboxylgruppe zu bilden vermögen. Bei der Silylierung oder Stannylierung einer Carboxylgruppe in einem Ausgangsmaterial der Formel II, kann, bei Verwendung eines Ueberschusses des Silylierungs- oder Stannylierungsmittels, die Aminogruppe ebenfalls silyliert oder stannyliert werden.

Die genannten Ylidengruppen sind in erster Linie Arylmethylengruppen, worin Aryl insbesondere für einen carbocyclischen, in erster Linie monocyclischen Arylrest, z.B. für gegebenenfalls, wie durch Nitro oder Hydroxy, substituiertes Phenyl steht.

Die übrigen in den Ausgangsstoffen der Formel II vorhandenen funktionellen Gruppen können durch die bereits unter den Verbindungen der Formel I genannten Schutzgruppen geschützt sein. Bevorzugt sind alle nicht an der Acylierungsreaktion teilnehmenden reaktionsfähigen funktionellen Gruppen, insbesondere aber gegebenfalls vorhandene, acylierbare Amino-, Hydroxy- und Mercaptogruppen, geschützt.

Den Acylrest einer Carbonsäure der Formel III einführende Acylierungsmittel sind beispielsweise die Carbonsäure selbst oder reaktionsfähige funktionelle Derivate davon.

Falls eine freie Säure der Formel III, worin alle funktionellen Gruppen ausser der reagierenden Carboxylgruppe geschützt sein können, zur Acylierung eingesetzt wird, verwendet man üblicherweise geeignete Kondensationsmittel, wie Carbodiimide, beispielsweise N,N'-Diäthyl-, N,N'-Dipropyl-, N,N'-Dicyclohexyl- oder N-Aethyl-N'-3-dimethylaminopropyl-carbodiimid, geeignete Carbonylverbindungen, beispielsweise Carbonyldiimidazol, oder Isoxazolinium-3'-sulfonat und N-tert.-Butyl-5-methyl-isoxazoliniumperchlorat, oder eine Acylaminoverbindung, z.B. 2-Aethoxy-1-äthoxycarbonyl-1,2-dihydro-chinolin.

Zur Acylierung kann auch z.B. analog DT-OS 2.927.536, ein Salz einer Säure der Formel III, z.B. ein Alkalimetall-, wie Natriumsalz, oder ein tertiäres Aminsalz, z.B. ein Triäthylaminsalz, in Gegenwart eines am Phosphoratom trichlorierten Bis-(3-oxo-oxazolidin-1-yl)-phosphorans, worin der Oxazolidin-3-onring gegebenenfalls durch Niederalkylgruppen substituiert sein kann, eingesetzt werden.

Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran, wenn erwünscht oder notwendig, unter Kühlen oder Erwärmen und/oder in einer Inertgasatmosphäre, durchgeführt.

Ein reaktionsfähiges, d.h. Amid-bildendes funktionelles Derivat einer Säure der Formel III, worin alle funktionellen Gruppen ausser der reagierenden Säuregruppe geschützt sein können, ist in erster Linie ein Anhydrid einer solchen Säure, inklusive und vorzugsweise ein gemischtes Anhydrid. Gemischte Anhydride sind z.B. diejenigen mit anorganischen Säuren, wie Halogenwasserstoffsäuren, d.h. die entsprechenden Säurehalogenide, z.B. -chloride oder -bromide, ferner mit Stickstoffwasserstoffsäure, d.h. die entsprechenden Säureazide, mit einer phosphorhaltigen Säure, z.B. Phosphorsäure, Diäthylphosphorsäure oder phosphoriger Säure, oder mit einer schwefelhaltigen Säure, z.B. Schwefelsäure oder mit Cyanwasserstoffsäure. Weitere gemischte Anhydride sind z.B. diejenigen mit organischen Carbonsäuren, wie mit gegebenenfalls

z.B. durch Halogen, wie Fluor oder Chlor, substituierten Niederalkancarbonsäuren, z.B. Pivalinsäure oder Trichloressigsäure, oder mit Halbestern, insbesondere Niederalkylhalbestern der Kohlensäure, wie dem Aethyl- oder Isobutylhalbester der Kohlensäure, oder mit organischen, insbesondere aliphatischen oder aromatischen, Sulfonsäuren, z.B. p-Toluolsulfonsäure.

Weitere zur Reaktion mit der Aminogruppe geeignete Säurederivate in einer Säure der Formel III, worin alle funktionellen Gruppen ausser der reagierenden Carboxylgruppe geschützt sein können, sind aktivierte Ester, wie Ester mit vinylogen Alkoholen (d.h. Enolen, wie vinylogen Niederalkenolen, oder Iminomethylesterhalogeniden, wie Dimethyliminomethylesterchlorid (hergestellt aus der Carbonsäure und Dimethylchlormethyliden-iminium-chlorid der Formel $[(CH_3)_2N{=}CHCl]^{\oplus}Cl^{\ominus}$, oder Arylester, wie 4-Nitrophenyl- oder 2,3-Dinitrophenylester, heteroaromatische Ester, wie Benztriazol-, z.B. 1-Benztriazolester, oder Diacyliminoester, wie Succinylimino- oder Phthaliminoester.

Die Acylierung mit einem Säurederivat, wie einem Anhydrid, insbesondere mit einem Säurehalogenid, wird bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyl-diisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Aethylenoxid oder Propylenoxid, durchgeführt.

Die obigen Acylierungen werden bevorzugt in einem inerten, vorzugsweise wasserfreien Lösungsmittel oder Lösungsmittelgemisch vorgenommen, beispielsweise in einem Carbonsäureamid, wie einem Formamid,

z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder einem Nitril, z.B. Acetonitril, oder Mischungen davon, bei Raumtemperatur, wenn notwendig, bei erniedrigter oder erhöhter Temperatur, etwa bei -40° bis etwa 100°, bevorzugt bei -10° bis +40°, und/oder in einer Inertgas-, z.B. Stickstoffatmosphäre.

Die Acylierung kann auch in Gegenwart einer die gewünschte Amidbindung herstellenden Acylase erfolgen. Solche Acylasen sind bekannt und werden durch eine Reihe von Mikroorganismen gebildet, z.B. durch Acetobacter, wie Acetobacter aurantium, Achromobacter, wie Achromobacter aeris, Aeromonas, wie Aeromonas hydrophila, oder Bacillus megaterium, z.B. 400. In die enzymatische Acylierung werden insbesondere Amide, Ester oder Thioester, wie entsprechende Niederalkyl-, z.B. Methyl- oder Aethylester der Carbonsäure der Formel III eingesetzt. Die enzymatische Acylierung wird in einem den Mikroorganismus enthaltenden Nährmedium, in einem Filtrat der Kulturbrühe oder gegebenenfalls nach Isolierung der Acylase, inklusive nach Adsorption an einen Träger, in einem wässrigen, gegebenenfalls einen Puffer enthaltenden Medium bei etwa 30-40°, bevorzugt bei etwa 37°, durchgeführt.

In einer acylierenden Säure der Formel III oder in einem Säurederivat davon kann eine geschützte Aminogruppe auch in ionischer Form vorliegen, d.h. das Ausgangsmaterial der Formel III kann in Form eines Säureadditionssalzes, vorzugsweise mit einer starken anorganischen Säure, wie einer Halogenwasserstoffsäure, z.B. Salzsäure, oder Schwefelsäure verwendet werden.

Ferner kann ein Säurederivat, wenn erwünscht, in situ gebildet werden. So erhält man z.B. ein gemischtes Anhydrid durch Behandeln einer Säure der Formel III, mit entsprechend geschützten funktionellen Gruppen, oder eines geeigneten Salzes davon, wie eines Ammoniumsalzes, z.B. mit einem organischen Amin, wie Pyridin oder 4-Methylmorpholin, oder eines

Metall-, z.B. Alkalimetallsalzes, mit einem geeigneten Säurederivat, wie einem entsprechenden Säurehalogenid einer gegebenenfalls substituierten Niederalkancarbonsäure, z.B. Trichloracetylchlorid, mit einem Halbester eines Kohlensäurehalbhalogenids, z.B. Chlorameisensäureäthylester oder -isobutylester, oder mit einem Halogenid einer Diniederalkylphosphorsäure, z.B. Diäthylphosphorbromidat, und verwendet das so erhältliche gemischte Anhydrid ohne Isolierung.

Verfahren b) (Isomerisierung): In einer 2-Cephem-Verbindung der Formel IV weist die gegebenenfalls geschützte Carboxylgruppe in 4-Stellung vorzugsweise die α-Konfiguration auf.

2-Cephem-Verbindungen der Formel IV werden isomerisiert, indem man sie mit einem schwach-basischen Mittel behandelt und die entsprechende 3-Cephem-Verbindung isoliert. Geeignete Isomerisierungsmittel sind z.B. organische, stickstoffhaltige Basen, insbesondere tertiäre heterocyclische Basen aromatischen Charakters, in erster Linie Basen des Pyridin-Typs, wie Pyridin selber, sowie Collidine oder Lutidine, ferner Chinolin, tertiäre aromatische Basen, z.B. solche des Anilin-Typs, wie N,N-Diniederalkylaniline, z.B. N,N-Dimethylanilin oder N,N-Diäthylanilin, oder tertiäre aliphatische, azacycloaliphatische oder araliphatische Basen, wie N,N,N-Triniederalkylamine, z.B. N,N,N-Trimethylamin, N,N-Diisopropyl-N-äthylamin, N-Niederalkyl-azacycloalkane, z.B. N-Methyl-piperidin, oder N-Phenyl-niederalkyl-N,N-diniederalkyl-amine, z.B. N-Benzyl-N,N-dimethylamin, sowie Gemische davon, wie das Gemisch einer Base vom Pyridintyp und eines N,N,N-Tri-niederalkylamins, z.B. Pyridin und Triäthylamin. Ferner können auch anorganische oder organische Salze von Basen, insbesondere von mittelstarken bis starken Basen mit schwachen Säuren, wie Alkalimetall-, oder Ammoniumsalze von Niederalkancarbonsäuren, z.B. Natriumacetat, Triäthylammoniumacetat oder N-Methyl-piperidinacetat, sowie andere analoge Basen oder Gemische von solchen basischen Mitteln verwendet werden.

Bei Isomerisierung mit basischen Mitteln arbeitet man vorzugsweise in wasserfreiem Medium, in An- oder Abwesenheit eines Lösungsmittels,

wie eines gegebenenfalls halogenierten, z.B. chlorierten, aliphatischen, cycloaliphatischen oder aromatischen Kohlenwasserstoffs, oder eines Lösungsmittelgemisches, wobei als Reaktionsmittel verwendete, unter den Reaktionsbedingungen flüssige Basen gleichzeitig auch als Lösungsmittel dienen können, unter Kühlen, bei Zimmertemperatur oder unter Erhitzen, vorzugsweise in einem Temperaturbereich von etwa -30° bis etwa +100°, in einer Inertgas-, z.B. Stickstoffatmosphäre, und/oder in einem geschlossenen Gefäss.

Die so erhältlichen 3-Cephem-Verbindungen lassen sich in an sich bekannter Weise, z.B. durch Adsorption und/oder Kristallisation, von gegebenenfalls noch vorhandenen 2-Cephem-verbindungen abtrennen.

Die Isomerisierung von 2-Cephem-verbindungen der Formel IV, worin n 0 ist, wird bevorzugt durchgeführt, indem man diese in 1-Stellung oxidiert, wenn erwünscht, ein erhältliches Isomerengemisch der 1-Oxide trennt, gegebenenfalls die erhältlichen 1-Oxide der entsprechenden 2-Cephem-verbindungen in die 1-Oxide der entsprechenden 3-Cephemverbindungen isomerisiert und gewünschtenfalls die so erhältlichen 1-Oxide zu den Verbindungen der Formel I, worin n 0 ist, reduziert.

Als geeignete Oxidationsmittel für die Oxidation in 1-Stellung von 2-Cephem-verbindungen kommen anorganische Persäuren, die ein Reduktionspotential von wenigstens +1,5 Volt aufweisen und aus nicht-metallischen Elementen bestehen, organische Persäuren oder Gemische aus Wasserstoffperoxid und Säuren, insbesondere organische Carbonsäuren, mit einer Dissoziationskonstante von wenigstens $10^{-5}$ in Frage. Geeignete anorganische Persäuren sind Perjodid- und Perschwefelsäure. Organische Persäuren sind entsprechende Percarbon- und Persulfonsäuren, die als solche zugesetzt oder durch Verwendung von wenigstens einem Aequivalent Wasserstoffperoxid und einer Carbonsäure in situ gebildet werden können. Dabei ist es zweckmässig, einen grossen Ueberschuss der Carbonsäure zu verwenden, wenn z.B. Essigsäure als Lösungsmittel verwendet wird. Geeignete Persäuren sind z.B. Perameisensäure, Peressigsäure, Trifluorperessigsäure

Permaleinsäure, Perbenzoesäure, 3-Chlorperbenzoesäure, Monoperphthalsäure oder p-Toluolpersulfonsäure.

Die Oxidation kann ebenfalls unter Verwendung von Wasserstoffperoxid mit katalytischen Mengen einer Säure mit einer Dissoziationskonstante von wenigstens $10^{-5}$ durchgeführt werden, wobei man niedrige Konzentrationen, z.B. 1-2%, und weniger, aber auch grössere Mengen der Säure einsetzen kann. Dabei hängt die Wirksamkeit des Gemisches in erster Linie von der Stärke der Säure ab. Geeignete Gemische sind z.B. solche von Wasserstoffperoxid mit Essigsäure, Perchlorsäure oder Trifluoressigsäure.

Die obige Oxidation kann in Gegenwart von geeigneten Katalysatoren durchgeführt werden. So kann z.B. die Oxidation mit Percarbonsäuren durch die Anwesenheit einer Säure mit einer Dissoziationskonstante von wenigstens $10^{-5}$ katalysiert werden, wobei ihre Wirksamkeit von ihrer Stärke abhängt. Als Katalysatoren geeignete Säuren sind z.B. Essigsäure, Perchlorsäure und Trifluoressigsäure. Ueblicherweise verwendet man mindestens äquimolare Mengen des Oxidationsmittels, vorzugsweise einen geringen Ueberschuss von etwa 10% bis etwa 20%, wobei man auch grössere Ueberschüsse, d.h. bis zur 10fachen Menge des Oxidationsmittels oder darüber, verwenden kann. Die Oxidation wird unter milden Bedingungen, z.B. bei Temperaturen von etwa -50° bis etwa +100°, vorzugsweise von etwa -10° bis etwa +40° durchgeführt.

Falls die Oxidation in einem nicht polaren Lösungsmittel durchgeführt wird, kann gegebenenfalls ein 1-Oxid einer 2-Cephemverbindung erhalten werden, das durch Behandlung in einem polaren Lösungsmittel, wie Essigsäure, leicht isomerisiert wird. Ueblicherweise findet die Isomerisierung bereits in dem Oxidationsmedium statt.

Die Reduktion der 1-Oxide von 3-Cephem-Verbindungen kann in an sich bekannter Weise durch Behandeln mit einem Reduktionsmittel, wenn notwendig, in Anwesenheit eines aktivierenden Mittels, durchgeführt werden. Als Reduktionsmittel kommen beispielsweise in Betracht:

Katalytisch aktivierter Wasserstoff, wobei Edelmetallkatalysatoren verwendet werden, welche Palladium, Platin oder Rhodium enthalten, und die man gegebenenfalls zusammen mit einem geeigneten Trägermaterial, wie Kohle oder Bariumsulfat, einsetzt; reduzierende Zinn-, Eisen-, Kupfer- oder Mangankationen, welche in Form von entsprechenden Verbindungen oder Komplexen anorganischer oder organischer Art, z.B. als Zinn-II-chlorid, -fluorid, -acetat oder -formiat, Eisen-II-chlorid, -sulfat, -oxalat oder -succinat, Kupfer-I-chlorid, -benzoat oder -oxyd, oder Mangan-II-chlorid, -sulfat, -acetat oder -oxyd, oder als Komplexe, z.B. mit Aethylendiamintetraessigsäure oder Nitrolotriessigsäure, verwendet werden; reduzierende Dithionit-, Jod- oder Eisen-II-cyanid-anionen, welche in Form von entsprechenden anorganischen oder organischen Salzen, wie Alkalimetall-, z.B. Natrium- oder Kaliumdithionit, Natrium- oder Kaliumjodid oder -eisen-II-cyanid, oder in Form der entsprechenden-Säuren,wie Jodwasserstoffsäure, verwendet werden; reduzierende trivalente anorganische oder organische Phosphorverbindungen, wie Phosphine, ferner Ester, Amide und Halogenide der phosphinigen, phosphorigen oder phosphonigen Säure, sowie diesen Phosphorsauerstoffverbindungen entsprechenden Phosphor-Schwefelverbindungen, worin organische Reste in erster Linie aliphatische, aromatische oder araliphatische Reste, z.B. gegebenenfalls substituierte Niederalkyl-, Phenyl- oder Phenylniederalkylgruppen darstellen, wie z.B. Triphenylphosphin, Tri-n-butylphosphin, Diphenylphosphinigsäuremethylester, Diphenylchlorphosphin, Phenyldichlorphosphin, Benzolphosphonigsäuredimethylester, Butanphosphonigsäuremethylester, Phosphorigsäuretriphenylester, Phosphorigsäuretrimethylester, Phoshphortrichlorid, Phosphortribromid, etc.; reduzierende Halogensilanverbindungen, die mindestens ein an das Siliciumatom gebundenes Wasserstoffatom aufweisen, und die ausser Halogen, wie Chlor, Brom oder Jod, auch organische Reste, wie aliphatische oder aromatische Gruppen, z.B. gegebenenfalls substituierte Niederalkyl- oder Phenylgruppen aufweisen können, wie Chlorsilan, Bromsilan, Di- oder Trichlorsilan, Di- oder Tribromsilan, Diphenylchlorsilan, Dimethylchlorsilan, Trimethyljodsilan oder auch Halogensilanverbindungen, worin alle Wasserstoffe durch organische Reste ersetzt sind, wie Triniederalkylhalogensilan, z.B. Trimethyljodsilan, oder cyclische, schwefelhaltige Silane, wie 1,3-Dithia-2,4-disila-cyclobutane oder 1,3,5-Trithia-2,4,6-trisila-cyclohexane, deren Siliciumatome durch Kohlenwasserstoffreste, wie insbesondere Niederalkylreste substituiert sind, z.B. 2,2,4,4-Tetramethyl-1,3-dithia-2,4-disilacyclobutan oder 2,2,4,4,6,6-Hexamethyl-1,3,5-trithia-2,4,6-trisilacyclohexan, etc.; reduzierende quaternäre Chlormethylen-iminiumsalze, insbesondere -chloride oder -bromide, worin die Iminiumgruppe durch einen bivalenten oder zwei monovalente organische Reste, wie gegebenenfalls substituierte Niederalkylen- oder Niederalkylgruppen substituiert ist, wie N-Chlormethylen-N,N-diäthyliminiumchlorid oder N-Chlormethylen-pyrrolidiniumchlorid; und komplexe Metallhydride, wie Natriumborhydrid, in Gegenwart von geeigneten Aktivierungsmitteln, wie Cobalt-II-chlorid, sowie Borandichlorid.

Als aktivierende Mittel, die zusammen mit denjenigen der obgenannten Reduktionsmittel verwendet werden, welche selber nicht Lewissäure-Eigenschaften aufweisen, d.h. die in erster Linie zusammen mit den Dithionit-, Jod- oder Eisen-II-cyanid- und den nicht halogenhaltigen trivalenten Phosphor-Reduktionsmitteln oder bei der katalytischen Reduktion eingesetzt werden, sind insbesondere organische Carbon- und Sulfonsäurehalogenide, ferner Schwefel-, Phosphor- oder Siliciumhalogenide mit gleicher oder grösserer Hydrolysenkonstante zweiter Ordnung als Benzoylchlorid, z.B. Phosgen, Oxalylchlorid, Essigsäurechlorid oder -bromid, Chloressigsäurechlorid; Pivalinsäurechlorid, 4-Methoxybenzoesäurechlorid, 4-Cyanbenzoesäurechlorid, p-Toluolsulfonsäurechlorid, Methansulfonsäurechlorid, Thionylchlorid, Phosphoroxychlorid, Phosphortrichlorid, Phosphortribromid, Phenyldichlorphosphin, Benzolphosphonigsäuredichlorid, Dimethylchlorsilan oder Trichlorsilan, ferner geeignete Säureanhydride, wie Trifluoressigsäureanhydrid, oder cyclische Sultone, wie Aethansulton, 1,3-Propansulton, 1,4-Butansulton oder 1,3-Hexansulton zu erwähnen.

Die Reduktion wird vorzugsweise in Gegenwart von Lösungsmitteln oder Gemischen davon durchgeführt, deren Auswahl in erster Linie durch

die Löslichkeit der Ausgangsstoffe und die Wahl des Reduktionsmittels bestimmt wird, so z.B. Niederalkancarbonsäuren oder Ester davon, wie Essigsäure und Essigsäureäthylester, bei der katalytischen Reduktion, und z.B. gegebenenfalls substituierte, wie halogenierte oder nitrierte aliphatische, cycloaliphatische, aromatische oder araliphatische Kohlenwasserstoffe, z.B. Benzol, Methylenchlorid, Chloroform oder Nitromethan, geeignete Säurederivate, wie Niederalkancarbonsäureester oder -nitrile, z.B. Essigsäureäthylester oder Acetonitril, oder Amide von anorganischen oder organischen Säuren, z.B. Dimethylformamid oder Hexamethylphosphoramid, Aether, z.B. Diäthyläther, Tetrahydrofuran oder Dioxan, Ketone, z.B. Aceton, oder Sulfone, insbesondere aliphatische Sulfone, z.B. Dimethylsulfon oder Tetramethylensulfon, etc., zusammen mit den chemischen Reduktionsmitteln, wobei diese Lösungsmittel vorzugsweise kein Wasser enthalten. Dabei arbeitet man gewöhnlicherweise bei Temperaturen von etwa -20°C bis etwa 100°C, wobei bei Verwendung von sehr reaktionsfähigen Aktivierungsmitteln die Reaktion bei tieferen Temperaturen durchgeführt werden kann.

Verfahren c) (Oxidation zu α-Oxoverbindungen): Die α-Ketoverbindung der Formel I, worin A eine Gruppe-C(=O)- ist, wird z.B. erhalten, indem man eine Verbindung der Formel Ic mit einem geeigneten Oxidationsmittel, z.B. Selendioxid, behandelt. Die Reaktion wird in einem inerten Lösungsmittel, wie einem Aether, z.B. Dioxan oder Tetrahydrofuran, einem Amid, z.B. Dimethylformamid, oder einem tertiären Amin, z.B. Pyridin, bei erniedrigter oder erhöhter Temperatur, z.B. bei etwa 0°C bis etwa 100°C, bevorzugt bei Zimmertemperatur bis zu etwa 80°C, durchgeführt.

Verfahren d) (Umsatz der Oxomethylengruppe mit Hydroxylaminen): In einer Verbindung der Formel Id, worin funktionelle Gruppen gegebenenfalls geschützt sind, wird die Oxogruppe in der 7β-Acylamidogruppe durch Behandeln mit Hydroxylamin oder einem substituierten Hydroxylamin der Formel $H_2N-O-R_1$ (V) in eine Hydroxyiminomethylen- bzw. $R_1$-substituierte Hydroxyiminomethylengruppe übergeführt.

Die Reaktion der Oxogruppe mit der Hydroxylaminverbindung wird auf übliche Weise ausgeführt, z.B. indem man die beiden Reaktionspartner in einem Lösungsmittel, wie Wasser oder einem organischen Lösungsmittel, wie einem Alkohol, , z.B. Methanol, bei leicht erhöhter oder erniedrigter Temperatur, wie zwischen etwa -20° bis etwa +100°, bevorzugt bei etwa 0° bis etwa 30°C gegebenenfalls in einer Inertgas-, wie Stickstoffatmosphäre, reagieren lässt. Die Hydroxylaminverbindung kann, auch in situ, aus einem ihrer Salze, beispielsweise einem Hydrohalogenid, wie Hydrochlorid, durch Behandeln mit einer anorganischen Base, wie einem Alkalimetallhydroxid, z.B. Natriumhydroxid, oder einer organischen Base, wie einem tertiären Amin, z.B. einem Triniederalkylamin, wie Triniederalkylamin, wie Triäthylamin oder Aethyl-diisopropylamin, oder einer heterocyclischen tertiären Base, wie Pyridin, in Freiheit gesetzt werden.

Verfahren e) (Nitrosierung der Methylengruppe): In einer Verbindung der Formel Ie sind die funktionellen Gruppen bevorzugt geschützt.

Geeignete Nitrosierungsmittel sind salpetrige Säure und davon abgeleitete Derivate, wie Nitrosylhalogenide, z.B. Nitrosylchlorid oder Nitrosylbromid, Salze der salpetrigen Säure, wie Alkalimetallsalze, z.B. Natrium- oder Kaliumnitrit, oder insbesondere Ester der salpetrigen Säure, wie Niederalkylester, z.B. Butyl-, Pentyl- oder insbesondere iso-Amylnitrit.

Wenn als Nitrosierungsmittel ein Salz der salpetrigen Säure eingesetzt wird, wird die Reaktion vorzugsweise in Gegenwart einer starken anorganischen oder auch organischen Säure, wie Chlorwasserstoffsäure, Schwefelsäure, Ameisensäure oder Essigsäure, durchgeführt. Bei Verwendung eines Esters der salpetrigen Säure wird die Reaktion vorzugsweise in Gegenwart einer starken Base, wie eines Alkalimetallalkylates durchgeführt.

Die Nitrosierung wird in einem geeigneten Lösungsmittel, wie Wasser, einer Carbonsäure, z.B. Essigsäure, einem niederen Alkohol,

z.B. Methanol oder Aethanol, einem Aether, z.B. Dioxan oder Tetrahydrofuran, oder einem Kohlenwasserstoff, z.B. Hexan oder Benzol, oder in Mischungen davon, unter Kühlung oder Erwärmung, insbesondere bei etwa -15° bis Raumtemperatur, durchgeführt.

Verfahren f) (Alkylierung der Hydroxyiminogruppe): In dem Ausgangsmaterial der Formel If sind alkylierbare Gruppen, ausser der zu alkylierenden Hydroxyiminogruppe, bevorzugt geschützt.

Die Verätherung der freien Hydroxygruppe wird mit einem den gewünschten, gegebenenfalls substituierten Niederalkylrest $R_1$ einführenden konventionellen Verätherungsmittel durchgeführt.

Geeignete Verätherungsmittel sind beispielsweise Diazoverbindungen, wie Diazoniederalkane, z.B. Diazomethan, Diazoäthan oder Diazo-n-butan, die gegebenenfalls im Alkanteil substituiert sein können. Diese Reagentien werden in Gegenwart eines geeigneten inerten Lösungsmittels, wie eines aliphatischen, cycloaliphatischen oder aromatischen Kohlenwasserstoffs, wie Hexan, Cyclohexan, Benzol oder Toluol, eines halogenierten aliphatischen Kohlenwasserstoffs, z.B. Methylenchlorid, oder eines Aethers, wie eines Diniederalkyläthers, z.B. Diäthyläther, oder eines cyclischen Aethers, z.B. Tetrahydrofuran oder Dioxan, oder eines Lösungsmittelgemisches, und je nach Diazoreagens unter Kühlen, bei Raumtemperatur oder unter leichtem Erwärmen, ferner, wenn notwendig, in einem geschlossenen Gefäss und/oder unter einer Inertgas-, z.B. Stickstoffatmosphäre, zur Anwendung gebracht.

Weitere geeignete Verätherungsmittel sind Ester entsprechender Alkohole, in erster Linie solche mit starken anorganischen oder organischen Säuren, wie Mineralsäuren, z.B. Halogenwasserstoffsäuren, wie Chlorwasserstoff-, Bromwasserstoff- oder Jodwasserstoffsäure, ferner Schwefelsäure oder Halogen-schwefelsäure, z.B. Fluorschwefelsäure, oder starken organischen Sulfonsäuren, wie gegebenenfalls, z.B. durch Halogen, wie Fluor, substituierten Niederalkansulfonsäuren, oder aromatischen

BAD ORIGINAL

Sulfonsäuren, wie z.B. gegebenenfalls, z.B. durch Niederalkyl, wie Methyl, Halogen, wie Brom oder Nitro substituierten Benzolsulfonsäuren, z.B. Methansulfon-, Trifluormethansulfon- oder p-Toluolsulfonsäure. Solche Ester sind u.a. Niederalkylhalogenide, Diniederalkylsulfate, wie Dimethylsulfat, ferner Fluorsulfonsäureniederalkylester, z.B. Fluorsulfonsäuremethylester oder gegebenenfalls Halogen-substituierte Methansulfonsäure-niederalkylester, z.B. Trifluormethansulfonsäuremethylester. Sie werden üblicherweise in Gegenwart eines inerten Lösungsmittels, wie eines gegebenenfalls halogenierten, wie chlorierten aliphatischen, cycloaliphatischen oder aromatischen Kohlenwasserstoffs, z.B. Methylenchlorid, eines Aethers, wie Dioxan oder Tetrahydrofuran, oder eines Gemisches verwendet. Dabei wendet man vorzugsweise geeignete Kondensationsmittel, wie Alkalimetallcarbonate oder -hydrogencarbonate, z.B. Natrium- oder Kaliumcarbonat oder -hydrogencarbonat (üblicherweise zusammen mit einem Sulfat), oder organischen Basen, wie, üblicherweise sterisch gehinderte, Triniederalkylamine, z.B. N,N-Diisopropyl-N-äthyl-amin (vorzugsweise zusammen mit Niederalkylhalogensulfaten oder gegebenenfalls Halogen-substituierten Methansulfonsäureniederalkylestern) an, wobei unter Kühlen bei Raumtemperatur oder unter Erwärmen, z.B. bei Temperaturen von etwa -20° C bis etwa 50° C und, wenn notwendig, in einem geschlossenen Gefäss und/oder in einer Inertgas-, z.B. Stickstoffatmosphäre, gearbeitet wird.

Weitere Verätherungsmittel sind entsprechende Acetale, z.B. gem-Niederalkoxyniederalkane, wie 2,2-Dimethoxy-propan, die in Gegenwart einer starken organischen Sulfonsäure, wie p-Toluolsulfonsäure, und eines geeigneten Lösungsmittels, wie eines Diniederalkyl- oder Niederalkylensulfoxids, z.B. Dimethylsulfoxyd, zur Anwendung gelangen, oder Orthoester, z.B. Orthoameisensäure-triniederalkylester, z.B. Orthoameisensäure-triäthylester, z.B. Orthoameisensäure-triäthylester, die in Gegenwart einer starken Mineralsäure, z.B. Schwefelsäure, oder einer starken organischen Sulfonsäure, wie p-Toluolsulfonsäure, und eines geeigneten Lösungsmittels, wie eines Aethers, z.B. Dioxan, verwendet werden.

Weitere Verätherungsmittel sind entsprechende Tri-$R_1$-oxoniumsalze

(sogenannte Meerweinsalze), Di-$R_1$-O-Carbeniumsalze oder Di-$R_1$-Haloniumsalze, worin $R_1$ den einzuführenden Rest darstellt, beispielsweise Triniederalkyloxoniumsalze, sowie Diniederalkoxycarbenium- oder Diniederalkylhaloniumsalze, insbesondere die entsprechenden Salze mit komplexen, fluorhaltigen Säuren, wie die entsprechenden Tetrafluorborate, Hexafluorphosphate, Hexafluorantimonate oder Hexachlorantimonate. Solche Reagentien sind z.B. Trimethyloxonium- oder Triäthyloxoniumhexafluorantimonat, -hexachlorantimonat, -hexafluorphosphat, oder -tetrafluorborat, Dimethoxycarbeniumhexafluorphosphat oder Dimethylbromonium-hexafluorantimonat. Man verwendet diese Verätherungsmittel vorzugsweise in einem inerten Lösungsmittel, wie einem Aether oder einem halogenierten Kohlenwasserstoff, z.B. Diäthyläther, Tetrahydrofuran oder Methylenchlorid, oder in einem Gemisch davon, wenn notwendig, in Gegenwart einer Base, wie einer organischen Base, z.B. eines, vorzugsweise sterisch gehinderten, Triniederalkylamins, z.B. N,N-Diisopropyl-N-äthyl-amin, und unter Kühlen, bei Raumtemperatur oder unter leichtem Erwärmen, z.B. bei etwa -20°C bis etwa 50°C, wenn notwendig, in einem geschlossenen Gefäss und/oder in einer Inertgas-, z.B. Stickstoffatmosphäre.

Weitere Verätherungsmittel sind schliesslich entsprechende 1-$R_1$-3-Aryltriazenverbindungen, worin $R_1$ den einzuführenden Rest und Aryl vorzugsweise einen gegebenenfalls substituierten Phenylrest, z.B. Niederalkylphenyl, wie 4-Methylphenyl bedeutet. Solche Triazenverbindungen sind 3-Aryl-1-niederalkyl-triazene, z.B. 3-(4-Methylphenyl)-1-methyl-triazen. Diese Reagentien werden üblicherweise in Gegenwart von inerten Lösungsmitteln, wie gegebenenfalls halogenierten Kohlenwasserstoffen oder Aethern, z.B. Benzol, oder Lösungsmittelgemischen, und unter Kühlen, bei Raumtemperatur und vorzugsweise bei erhöhter Temperatur, z.B. bei etwa 20°C bis etwa 100°C, wenn notwendig, in einem geschlossenen Gefäss und/oder in einer Inertgas-, z.B. Stickstoffatmosphäre, verwendet.

Verfahren g) (Alkoxylierung der 7α-Stellung): In einer Verbindung der Formel Ig, worin alle funktionellen Gruppen in geschützter Form vorliegen, kann die 7α-Niederalkoxygruppe $R_2$ auf an sich bekannte Weise eingeführt werden, beispielsweise indem man das genannte Zwischenprodukt nacheinander mit einem Anionen-bildenden Mittel, einem N-Halogenierungsmittel und einem Niederalkanol behandelt.

Ein geeignetes Anion-bildendes Mittel ist in erster Linie eine metallorganische Base, insbesondere eine Alkalimetall-, in erster Linie eine Lithium-organische Base. Solche Verbindungen sind insbesondere entsprechende Alkoholate, wie geeignete Lithium-niederalkanolate, in erster Linie Lithiummethylat, oder entsprechende Metall-Kohlenwasserstoffbasen, wie Lithium-niederalkane und Lithiumphenyl. Die Umsetzung mit der Anion-bildenden metallorganischen Base wird üblicherweise unter Kühlen, z.B. von etwa 0°C bis etwa -80°C, und in Gegenwart eines geeigneten Lösungs- oder Verdünnungsmittels, z.B. eines Aethers, wie Tetrahydrofuran, bei Verwendung von Lithiummethylat auch in Gegenwart von Methanol, und, wenn erwünscht, in einem geschlossenen Gefäss und/oder in einer Inertgas-, z.B. Stickstoffatmosphäre, vorgenommen.

Als N-halogenierendes Mittel verwendet man üblicherweise ein sterisch gehindertes, organisches Hypohalogenit, insbesondere -chlorit, und in erster Linie ein entsprechendes aliphatisches Hypohalogenit, z.B. -chlorit, wie ein tert.-Niederalkyl-hypohalogenit, z.B. -chlorit. In erster Linie wendet man das tert.-Butylhypochlorit an, das man mit dem nichtisolierten Produkt der Anionisierungsreaktion umsetzt.

Die N-halogenierte Zwischenverbindung wird bei Anwesenheit eines Ueberschusses der Anion-bildenden Base, insbesondere von Lithiummethylat, unter den Reaktionsbedingungen und ohne isoliert zu werden, in eine 7-Acylaminocephemverbindung umgewandelt, und diese durch Zugabe des Niederalkanols in eine 7α-Niederalkoxy-cephem-Verbindung übergeführt. Falls notwendig, müssen aus dem N-halogenierten Zwischenprodukt die Elemente der Halogenwasserstoff-, insbesondere der Chlorwasserstoffsäure, abgespalten werden; dies geschieht unter Zugabe einer Halogenwasserstoffabspaltenden Base, wie eines geeigneten Alkalimetall-niederalkanolats, z.B. Lithium-tert.-butylat, wobei diese Reaktion üblicherweise unter den

Bedingungen der Anion- und N-Halogenverbindung-bildenden Reaktion stattfindet, wobei man in Gegenwart von Methanol arbeiten und anstelle der Acyliminoverbindung direkt die 7α-Niederalkoxy-cephem-Verbindung erhalten kann. Man geht üblicherweise aus von einer Verbindung der Formel If, worin funktionelle Gruppen in geschützter Form vorliegen, setzt diese mit einem Ueberschuss des Anion-bildenden Mittels, z.B. Lithiummethylat oder Phenyllithium, in Gegenwart von Methanol um, behandelt dann mit dem N-Halogenierungsmittel, z.B. tert.-Butylhypochlorit, und erhält so direkt die gewünschte Verbindung der Formel I, worin funktionelle Gruppen geschützt sind. Man kann auch das Niederalkanol nachträglich zugeben, wobei man die Dehydrohalogenierung und die Zugabe des Niederalkanols bei etwas höheren Temperaturen als die Anion- und N-Halogenverbindung-bildenden Reaktionen, z.B. bei etwa 0°C bis etwa -20°C, wenn notwendig, in einem geschlossenen Gefäss und/oder in einer Inertgas-, z.B. Stickstoffatmosphäre, durchführen kann.

Bei vorstehenden Reaktionen, die unter basischen Bedingungen durchgeführt werden, können 3-Cephemverbindungen, gegebenenfalls teilweise, zu 2-Cephemverbindungen isomerisieren. Eine erhaltene 2-Cephemverbindung oder ein Gemisch aus einer 2- und einer 3-Cephemverbindung kann in an sich bekannter Weise zur gewünschten 3-Cephemverbindung isomerisiert werden.

Verfahren h) (Thiolisierung): In einem Ausgangsmaterial der Formel Ih ist ein geeigneter, durch eine Heterocyclylthiogruppe ersetzbarer Rest $R_3'$ beispielsweise eine der genannten Acyloxygruppen $R_3^o$, wie eine durch eine gegebenenfalls substituierte niederaliphatische Carbonsäure veresterte Hydroxygruppe, oder eine der genannten Sulfonyloxygruppen $R_3^o$. Solche veresterten Hydroxygruppen $R_3'$ sind insbesondere Acetyloxy, ferner Formyloxy und Acetoacetyl; sowie Mesyloxy oder Tosyloxy.

Die Reaktion einer solchen Verbindung mit einer heterocyclischen Mercaptanverbindung $H-R_3^o$ kann unter neutralen oder schwach basischen Bedingungen in Gegenwart von Wasser und gegebenenfalls einem mit Wasser mischbaren organischen Lösungsmittel durchgeführt werden. Die basischen

Bedingugen können beispielsweise durch Zugabe einer anorganischen Base, wie eines Alkalimetall- oder Erdalkalimetallhydroxids, -carbonats oder -hydrogencarbonats, z.B. von Natrium-, Kalium- oder Calciumhydroxid, -carbonat oder -hydrogencarbonat, eingestellt werden. Als organische Lösungsmittel können z.B. mit Wasser mischbare Alkohole, z.B. Niederalkanole, wie Methanol oder Aethanol, Ketone, z.B. Niederalkanone, wie Aceton, Amide, z.B. Niederalkancarbonsäureamide, wie Dimethylformamid, oder Nitrile, z.B. Niederalkansäurenitrile, wie Acetonitril, und ähnliche verwendet werden. Die Reaktion wird bei Raumtemperatur oder, je nach Reaktionsfähigkeit, unter Kühlen oder leichtem Erwärmen, durchgeführt. Gegebenenfalls kann die Thiolysierung in Gegenwart von in Wasser löslichen Salzen der Jodwasserstoffsäure und/oder der Thiocyansäure, wie entsprechenden Alkali-, Erdalkali-, Ammonium- oder quaternären Ammoniumsalzen einschliesslich phasentransferierenden Salzen, erfolgen. Geeignete Salze sind beispielsweise Lithium-, Kalium-, Magnesium-, Calcium- und insbesondere Natrium- und Tetra-n.butylammoniumjodid. Die Reaktion wird dann bevorzugt in konzentrierten wässrigen Lösungen dieser Salze durchgeführt.

Verfahren i) (Acylierung der 3-Hydroxymethylgruppe): In einem Ausgangsmaterial der Formel Ii liegen bevorzugt alle acylierbaren Gruppen, ausser der zu acylierenden 3-Hydroxymethylgruppe, in geschützter Form vor.

Die Acylierung erfolgt nach an sich bekannten Methoden durch Behandeln mit einem den gewünschten Acylrest einführenden Acylierungsmittel. Geeignete Acylierungsmittel sind die freie Säure selbst oder ein reaktionsfähiges funktionelles Derivat davon, wie ein Anhydrid, inklusive ein gemischtes Anhydrid oder ein inneres Anhydrid, oder ein aktivierter Ester.

Falls eine freie Säure $H-R_3^o$ zur Acylierung eingesetzt wird, verwendet man geeignete Kondensationsmittel, wie Carbodiimide, beispielsweise N,N'-Diäthyl-, N,N'-Dipropyl-, N,N'-Dicyclohexyl- oder N-Aethyl-N'-3-dimethylaminopropyl-carbodiimid, geeignete Carbonylverbindungen, beispielsweise Carbonyldiimidazol, oder Isoxazolinium-3'-sulfonat und N-tert.-Butyl-5-methyl-isoxazoliniumperchlorat, oder eine Acylaminoverbindung, z.B. 2-Aethoxy-1-äthoxycarbonyl-1,2-dihydrochinolin.

Zur Acylierung kann auch, z.B. analog DT-OS 2.927.536, ein Salz einer Säure der Formel III, z.B. ein Alkalimetall-, wie Natriumsalz, oder ein tertiäres Aminsalz, z.B. ein Triäthylaminsalz, in Gegenwart eines am Phosphoratom trichlorierten Bis-(3-oxo-oxazolidin-1-yl)-phosphorans, worin der Oxazolidin-3-onring gegebenenfalls durch Niederalkylgruppen substituiert sein kann, eingesetzt werden.

Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran, wenn erwünscht oder notwendig, unter Kühlen oder Erwärmen und/oder in einer Inertgasatmosphäre, durchgeführt.

Ein reaktionsfähiges, d.h. Ester-bildendes funktionelles Derivat einer Säure ist in erster Linie ein Anhydrid einer solchen Säure, inklusive und vorzugsweise ein gemisches Anhydrid, aber auch ein inneres Anhydrid, d.h. ein entsprechendes Keten oder ein entsprechendes Isocyanat. Gemischte Anhydride sind z.B. diejenigen mit anorganischen Säuren, wie Halogenwasserstoffsäuren, d.h. die entsprechenden Säurehalogenide, z.B. -chloride oder -bromide, ferner mit Stickstoffwasserstoffsäure, d.h. die entsprechenden Säureazide, mit einer phosphorhaltigen Säure, z.B. Phosphorsäure oder phosphoriger Säure, oder mit einer schwefelhaltigen Säure, z.B. Schwefelsäure, oder mit Cyanwasserstoffsäure. Weitere gemischte Anhydride sind z.B. diejenigen mit organischen Carbonsäuren, wie mit gegebenenfalls, z.B. durch Halogen, wie Fluor oder Chlor, substituierten Niederalkancarbonsäure, z.B. Pivalinsäure oder Trichloressigsäure, oder mit Halbestern, insbesondere Niederalkylhalbestern der Kohlensäure, wie dem Aethyl- oder Isobutylhalbester der Kohlensäure, oder mit organischen, insbesondere aliphatischen oder aromatischen, Sulfonsäuren, z.B. p-Toluolsulfonsäure.

Zur Reaktion mit der Hydroxygruppe geeignete aktivierte Ester sind z.B. Ester mit vinylogen Alkoholen (d.h. Enolen), wie vinylogen Niederalkenolen, oder Iminomethylesterhalogeniden, wie Dimethyliminomethylesterchlorid (hergestellt aus der Carbonsäure und Dimethyl-chlormethyliden-iminium-chlorid der Formel $[(CH_3)_2N{=}CHCl]^{\oplus}Cl^{\ominus}$), oder Arylester, wie 4-Nitrophenyl- oder 2,3-Dinitrophenylester, heteroaromatische Ester, wie Benztriazol-, z.B. 1-Benztriazolester, oder Diacyliminoester, wie Succinylimino- oder Phthaliminoester.

Die Acylierung mit einem Säurederivat, wie einem Anhydrid, insbesondere mit einem Säurehalogenid, wird bevorzugt in Anwesenheit eines säurebindenden Mittels, beispielsweise einer organischen Base, wie eines organischen Amines, z.B. eines tertiären Amins, wie Triniederalkylamin, z.B. Trimethylamin, Triäthylamin oder Aethyl-diisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Aethylenoxid oder Propylenoxid, durchgeführt.

Die obigen Acylierungen werden bevorzugt in einem inerten, vorzugsweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder einem Nitril, z.B. Acetonitril, oder Mischungen davon, bei Raumtemperatur, wenn notwendig bei erniedrigter oder erhöhter Temperatur, etwa bei -40° bis etwa +100°, bevorzugt bei -10° bis +40°, und/oder in einer Inertgas-, z.B. Stickstoffatmosphäre.

Ferner kann ein Säurederivat, wenn erwünscht, in situ gebildet werden. So erhält man z.B. ein gemischtes Anhydrid durch Behandeln einer Säure $H-R_3^o$ mit entsprechend geschützten funktionellen Gruppen, oder eines geeigneten Salzes davon, wie eines Ammoniumsalzes, z.B. mit einem organischen Amin, wie Pyridin oder 4-Methylmorpholin, oder eines Metall-, z.B. Alkalimetallsalzes, mit einem geeigneten Säurederivat, wie einem entsprechenden Säurehalogenid einer gegebenenfalls substituierten Niederalkancarbonsäure, z.B. Trichloracetylchlorid, mit einem Halbester eines Kohlensäurehalbhalogenids, z.B. Chlorameisensäureäthylester oder -isobutylester, oder mit einem Halogenid einer Diniederalkylphosphorsäure, z.B. Diäthylphosphorbromidat, und verwendet das so erhältliche gemischte Anhydrid ohne Isolierung.

Zur Einführung einer Carbamoylgruppe wird die Verbindung der Formel If mit einem geeigneten Kohlensäurederivat, insbesondere mit einer Isocyanat- oder Carbaminsäureverbindung, wie einem Silylisocyanat, z.B. Silyltetraisocyanat, einem Sulfonylisocyanat, z.B. Chlorsulfonylisocyanat, mit einem Carbaminsäurehalogenid, z.B. -chlorid (die zu N-substituierten 3-Aminocarbonyloxymethyl-Verbindungen führen), oder dann mit einer N-substituierten Isocyanat- oder mit einer N-mono- oder N,N-disubstituierten Carbaminsäure-Verbindung, wie einem entsprechenden Carbaminsäurehalogenid, z.B. -chlorid, umgesetzt, wobei man üblicherweise in Gegenwart eines Lösungs- oder Verdünnungsmittels und, wenn notwendig, unter Kühlen oder Erwärmen, in einem geschlossenen Gefäss und/oder in einer Inertgas-, z.B. Stickstoffatmosphäre, arbeitet.

Verfahren j) (Einführung einer Ammoniogruppe): In einem Ausgangsmaterial der Formel Ij stellt die Gruppe $R_3''$ vorzugsweise eine veresterte Hydroxygruppe dar, insbesondere eine durch eine organische Säure, wie Niederalkancarbonsäure, z.B. Ameisen- oder Essigsäure, ferner eine anorganische Säure, wie Halogenwasserstoffsäure, z.B. Brom- oder Jodwasserstoffsäure, veresterte Hydroxygruppe.

Die Reaktion mit einer tertiären organischen Base, insbesondere einem gegebenenfalls substituierten Pyridin, wird unter neutralen oder schwach sauren Bedingungen, vorzugsweise bei einem pH-Wert von etwa 6,5, in Gegenwart von Wasser und gegebenenfalls in einem mit Wasser mischbaren organischen Lösungsmittel vorgenommen. Die schwachsauren Bedingungen können durch Zugabe einer geeigneten organischen oder anorganischen Säure, beispielsweise Essigsäure, Chlorwasserstoffsäure, Phsophorsäure oder auch Schwefelsäure eingestellt werden. Als organische Lösungsmittel können beispielsweise mit Wasser mischbare Lösungsmittel, wie Niederalkanole, z.B. Methanol oder Aethanol, Niederalkanone, z.B. Aceton, Niederalkancarbonsäureamide, z.B. Dimethylformamid, oder Niederalkancarbonsäurenitrile, z.B. Acetonitril, verwendet werden. Ferner können, z.B. zur Erhöhung der Ausbeuten, dem Reaktionsgemisch geeignete Salze, wie Alkalimetall-, z.B. Natrium- und insbesondere Kaliumsalze, von anorganischen Säuren, wie Halogen-wasserstoffsäuren, z.B. Chlorwasserstoff- und insbesondere Jodwasserstoffsäuren, sowie der Thiocyansäure, oder von organischen Säuren, wie Niederalkancarbonsäuren, z.B. Essigsäure, insbesondere Kaliumjodid und Kaliumthiocyanat, zugesetzt werden. Auch Salze von geeigneten Anionenaustauschern mit Säuren, z.B. Essigsäure, z.B. flüssige Ionenaustauscher in Salzform, z.B. Amberlite LA-1 (flüssige sekundäre Amine mit einem Molekulargewicht von 351-393; Oel-löslich und Wasser-unlöslich; mAeq./g = 2,5-2,7, z.B. in Acetatform), können für diesen Zweck verwendet werden.

Ammoniogruppen $R_3^o$ können vorteilhafterweise unter Verwendung eines Zwischenprodukts der Formel Ij, in welchem $R_3''$ für eine substituierte, insbesondere für eine aromatische substituierte Carbonylthiogruppe und in erster Linie für die Benzoylthiogruppe steht, eingeführt werden. Ein solches Zwischenprodukt kann man z.B. durch Umsetzen eines Ausgangsmaterials der Formel Ij, worin $R_3''$ eine veresterte Hydroxygruppe, insbesondere Niederalkanoyloxy, z.B. Acetyloxy, bedeutet, mit einem geeigneten Salz, wie einem Alkalimetall-, z.B. Natriumsalz, einer Thiocarbonsäure, wie einer aromatischen Thiocarbonsäure, z.B. Thiobenzoesäure, erhalten. Das Zwischenprodukt wird

dann mit der tertiären Aminbase, insbesondere einer entsprechenden heterocyclischen Base, wie einem gegebenenfalls substituierten Pyridin, umgesetzt, wobei man die gewünschte Ammonioverbindung erhält. Die Reaktion wird üblicherweise in Gegenwart eines geeigneten Entschwefelungsmittels, insbesondere eines Quecksilbersalzes, z.B. Quecksilber-II-perchlorat, und eines geeigneten Lösungs- oder Verdünnungsmittels oder eines Gemisches, wenn notwendig, unter Kühlen oder Erwärmen, in einen geschlossenen Gefäss und/oder in einer Inertgas-, z.B. Stickstoffatmosphäre, durchgeführt.

Verfahren ka) (Decarbonylierung): In einem Ausgangsmaterial der Formel Ika sinddie gegebenenfalls vorhandenen funktionellen Gruppen üblicherweise geschützt.

Der Ersatz der Formylgruppe durch Wasserstoff wird in erster Linie durch Behandeln des Ausgangsmaterials mit einem Kohlenmonoxydaufnehmenden Schwermetallkomplex durchgeführt. Solche Schwermetallkomplexe sind insbesondere Platinmetall-Komplexe, wobei man unter einem Platinmetall ausser Platin auch Iridium, Rhodium, Palladium und Osmium versteht.

Vorzugsweise verwendet man Bis-trisubstituierte Phosphin-platinhalogenide, Bis-trisubstituierte Phosphincarbonyliridiumhalogenide oder Tris-trisubstituierte Phosphin-iridiumhalogenide, in erster Linie Tris-trisubstituierte Phosphin-rhodiumhalogenide, worin die Substituenten des Phosphins vorzugsweise Niederalkyl, z.B. n-Butyl, und in erster Linie Phenyl und die Halogenide vorzugsweise Chloride sind. Solche Phosphinplatinmetall-Komplexe, welche Kohlenmonoxyd in kovalenter Bindung aufzunehmen vermögen, sind z.B. Bis-triphenylphosphin-platin-II-chlorid-$[(C_6H_5)_3P]_2PtCl_2$ oder Bis-triphenyl-phosphincarbonyliridium-II-chlorid $[(C_6H_5)_3P]_2\ Ir(CO)Cl$, sowie Tris-triphenylphosphin-iridium-I-chlorid $[(C_6H_5)_3P]_3IrCl$, in erster Linie aber Tristriphenyl-phosphin-rhodium-I-chlorid $[(C_6H_5)_3P]_3RhCl$.

Wenn erwünscht oder notwendig, kann die Decarbonylierung mit den obgenannten Schwermetallkomplexen in Gegenwart von geeigneten Katalysatoren oder Aktivierungsmitteln, z.B. Lewissäure, wie Bortrifluorid (das man z.B. zusammen mit dem Bis-triphenylphosphin-platinchlorid verwenden kann), ferner eines Perchlorats, wie Alkalimetallperchlorat, z.B. Natriumperchlorat (das man z.B. zusammen mit Bis-triphenylphosphin-carbonyliridiumchlorid verwenden kann), durchgeführt werden.

Vorzugsweise arbeitet man in Gegenwart von inerten Lösungsmitteln, insbesondere von Kohlenwasserstoffen, wie aliphatischen oder cycloaliphatischen, insbesondere aromatischen Kohlenwasserstoffen, wie entsprechenden aliphatischen oder aromatischen Chlorkohlenwasserstoffen, z.B. Methylenchlorid oder Chlorbenzol, Aethern, wie aliphatischen, cycloaliphatischen oder aromatischen, ferner gemischten Aethern, z.B. Di-n-butyläther, Dioxan, Diphenyläther oder Anisol, Nitrilen, wie aliphatischen oder aromatischen Nitrilen, z.B. Acetonitril oder Benzonitril, oder Ketonen, insbesondere aliphatischen Ketonen, wie Niederalkanonen, z.B. Aceton, Aethylmethylketon oder Isobutylmethylketon, oder Gemischen von solchen Lösungsmitteln. Dabei wird die Reaktion unter Kühlen, bei Zimmertemperatur oder unter Erwärmen, z.B. in einem Temperaturbereich von etwa 10°C bis etwa 150°C, wie von etwa 40°C bis etwa 120°C, ferner, wenn notwendig, in einem geschlossenen Gefäss und/oder in einer inerten Gasatmosphäre, z.B. unter Stickstoff oder Argon, durchgeführt.

Ausgehend von 2-Cephem-Ausgangsstoffen der Formel IX kann man verfahrensgemäss zu den 3-Cephem-Verbindungen der Formel I gelangen, indem unter den Reaktionsbedingungen der Decarbonylierung die Isomerisierung von einer 2-Cephem- zur erwünschten 3-Cephem-Verbindung erfolgen kann.

<u>Verfahren kb) (Austausch einer Hydroxygruppe oder einer gegebenenfalls substituierten Aminogruppe durch Wasserstoff):</u>

Gegebenenfalls substituiertes Amino $X_2$ in einem Ausgangsmaterial

der Formel Ikb kann einen oder zwei einwertige oder einen zweiwertigen Substituenten, wie Niederalkyl, Niederalkylen, Oxaniederalkylen oder N-substituiertes, z.B. N-niederalkyliertes Azaniederalkylen enthalten, und ist z.B. Amino, Methylamino, Dimethylamino, Diäthylamino, Pyrrolidino, Piperidino oder Morpholino.

Der Ersatz der Gruppe $X_2$ in einer Verbindung der Formel Ikb durch Wasserstoff wird vorzugsweise in zwei Stufen durch Reduktion der 3,4-Doppelbindung im 3-Cephemring, gegebenenfalls Ueberführen einer Hydroxygruppe $X_2$ in eine veresterte Hydroxygruppe, und anschliessende Abspaltung der Elemente von Wasser, einer Säure oder eines Amins durchgeführt.

Beim Austausch von $X_2$ gegen Wasserstoff erfolgt die Reduktion der 3,4-Doppelbindung in Ausgangsstoffen der Formel Ikb z.B. mittels geeigneter Hydrid-Reduktionsmittel. Solche sind in erster Linie komplexe Metallhydride, vorzugsweise entsprechende Borhydride, wie Diboran oder Alkalimetallborhydride, z.B. Natriumborhydrid oder Lithiumborhydrid, ferner Zinkborhydrid, sowie organische Alkalimetallaluminiumhydride, wie Tri-niederalkoxy-alkalimetall-aluminiumhydride, z.B. Tri-(tert.-butyloxy)-lithiumaluminiumhydrid, die man üblicherweise in Gegenwart von Lösungsmitteln, insbesondere von relativ polaren Lösungsmitteln, wie Alkoholen, z.B. Niederalkanolen, wie Methanol oder Aethanol, oder Aethern, wie aliphatischen Aethern, z.B. Glycol- und Polyglycoläthern, wie Aethylenglycoldimethyläther oder Diäthylenglycoldimethyläther, oder cyclischen Aethern, wie Tetrahydrofuran oder Dioxan, oder Lösungsmittelgemischen, auch von wasserhaltigen Lösungsmitteln, anwendet, wobei man bei Temperaturen von etwa -20°C bis etwa 80°C, wenn notwendig, in einem geschlossenen Gefäss und/oder in einer Inertgas-, z.B. Stickstoffatmosphäre arbeitet.

Falls die obige Reduktion in Gegenwart eines geeigneten Wasser- oder Amin-abspaltenden Mittels durchgeführt wird, kann die erwünschte 3-unsubstituierte 3-Cephem-Verbindung der Formel I unmittelbar erhalten werden. Es kann aber auch zunächst eine entsprechende 3-$X_2$-Cepham-Verbindung entstehen, die entweder isoliert oder in situ weiterverarbeitet werden kann.

Wenn erwünscht, kann auf der Stufe der so erhältlichen Cepham-Verbindung eine Hydroxygruppe $X_2$ durch Verestern nach an sich bekannten Methoden (siehe z.B. Verfahren 1 ), z.B. durch Behandeln mit einem geeigneten Säureanhydrid, wie einem symmetrischen oder gemischten Säureanhydrid, inkl. einem entsprechenden Säurehalogenid, z.B. -chlorid, oder mit einem geeigneten Halogenierungsmittel, wie einer organischen Halogen-Phosphorverbindung, in eine veresterte Hydroxygruppe $X_2$ überführen. Veresterte Hydroxygruppen sind vorzugsweise durch starke organische oder anorganische Säuren, wie starke Mineralsäuren, z.B. Halogenwasserstoffsäuren, wie Chlorwasserstoffsäure, oder starke organische Sulfonsäuren, wie entsprechende niederaliphatische oder aromatische Sulfonsäuren, z.B. Methansulfonsäure oder p-Toluolsulfonsäure, veresterte Hydroxygruppen und stellen z.B. Halogen, wie Chlor, Niederalkylsulfonyloxy, z.B. Methylsulfonyloxy, oder Aethylsulfonyloxy, oder Arylsulfonyloxy, z.B. p-Toluolsulfonyloxy dar.

In der zweiten Verfahrensstufe erfolgt dann die Abspaltung der Elemente von Wasser, einer Säure oder eines Amins, aus so erhältlichen Cepham-Verbindungen, wobei man diese vorzugsweise mit geeigneten Wasser-, Säure- oder Amin-abspaltenden Mitteln behandelt. Wasser und Amine werden üblicherweise mittels einer starken organischen Carbon-, wie einer Halogen-niederalkancarbonsäure, oder einer starken organischen Sulfonsäure, z.B. p-Toluolsulfonsäure oder Methansulfonsäure, abgespalten. Wasser kann auch mittels eines geeigneten wasserbindenden Säurederivats, wie eines Anhydrids oder insbesondere Säurehalogenids, wie Säurechlorids, einer von Phosphor oder Schwefel abgeleiteten Säure, wie einer entsprechenden anorganischen Säure, z.B. mittels Phosphoroxychlorid oder Thionylchlorid, oder einer organischen Sulfonsäure, wie einer niederaliphatischen oder aromatischen Sulfonsäure, z.B. mittels Tosylchlorid oder Mesylchlorid, abgespalten werden. Man arbeitet dabei vorzugsweise in Gegenwart einer Base, wie eines tertiären Amins, z.B. Triniederalkylamin oder einer heterocyclischen Base, wie Triäthylamin oder Pyridin, oder eines geeigneten Ionenaustauschers, z.B. eines mit Kationen beladenen, sulfonierten Polystyrol-Ionenaustauschers.

Ferner kann man zur Wasserabspaltung auch dehydratisierende Carbodiimidverbindungen, z.B. Dicyclohexylcarbodiimid, oder dehydratisierende, über Stickstoffatome disubstituierte Carbonylverbindungen, z.B. Carbonyldiimidazol, verwenden. Dabei wendet man diese Mittel üblicherweise in Gegenwart eines Lösungsmittels, wie eines gegebenenfalls halogenierten, niederaliphatischen oder aromatischen Kohlenwasserstoffs, z.B. Benzol oder Toluol, oder eines Lösungsmittelgemisches an, wobei geeignete saure Mittel, wie Trifluoressigsäure, gleichzeitig auch als Lösungsmittel verwendet werden können. Wenn notwendig, kann man ein wasser-adsorbierendes Mittel oder einen Wasserabscheider einsetzen. Man arbeitet unter Kühlen oder Erwärmen und/oder in einer Inertgas-, z.B. Stickstoffatmosphäre.

<u>Verfahren l )</u> <u>(Verätherung und Veresterung von 3-Hydroxy):</u>
In einem Ausgangsmaterial der Formel II, worin die Gruppe $R_4$ als funktionell abgewandelte Carboxylgruppe vorliegt, und andere, gegebenenfalls vorhandene funktionelle Gruppen vorzugsweise geschützt sind, kann die Hydroxygruppe in an sich bekannter Weise veräthert werden.

Geeignete Verätherungsmittel sind beispielsweise entsprechende Diazoverbindungen, wie gegebenenfalls substituierte Diazoniederalkane, z.B. Diazomethan, Diazoäthan, Diazo-n-butan oder Diphenyldiazomethan. Diese Reagentien werden in Gegenwart eines geeigneten inerten Lösungsmittels, wie eines aliphatischen, cycloaliphatischen oder aromatischen Kohlenwasserstoffs, wie Hexan, Cyclohexan, Benzol oder Toluol, eines halogenierten aliphatischen Kohlenwasserstoffs, z.B. Methylenchlorid, oder eines Aethers, wie eines Diniederalkyläthers, z.B. Diäthyläther, oder eines cyclischen Aethers, z.B. Tetrahydrofuran oder Dioxan, oder eines Lösungsmittelgemisches, und, je nach Diazoreagens, unter Kühlen,bei Raumtemperatur oder unter leichtem Erwärmen, ferner,wenn notwendig, in einem geschlossenen Gefäss und/ oder unter einer Inertgas-, z.B. Stickstoffatmosphäre zur Anwendung gebracht.

Weitere geeignete Verätherungsmittel sind Ester entsprechender Alkohole, in erster Linie solche mit starken anorganischen oder organischen Säuren, wie Mineralsäuren, z.B. Halogenwasserstoffsäuren, wie Chlorwasserstoff-, Bromwasserstoff- oder Jodwasserstoffsäure,ferner Schwefelsäure,oder Halogen-schwefelsäuren, z.B. Fluorschwefelsäure, oder starken organischen Sulfonsäuren, wie gegebenenfalls, z.B. durch Halogen, wie Fluor, substituierten Niederalkansulfonsäuren, oder aromatischen Sulfonsäuren, wie gegebenenfalls, z.B. durch Niederalkyl, wie Methyl, Halogen, wie Brom, und/oder Nitro substituierten Benzolsulfonsäuren, z.B. Methansulfon-, Trifluormethansulfon- oder p-Toluolsulfonsäure. Solche Ester sind u.a. Niederalkylhalogenide, Diniederalkylsulfate, wie Dimethylsulfat, ferner Fluorsulfonsäureester, wie -niederalkylester,z.B. Fluorsulfonsäuremethylester, oder gegebenenfalls Halogen-substituierte Methansulfonsäure-niederalkylester, z.B. Trifluormethansulfonsäuremethylester. Sie werden üblicherweise in Gegenwart eines inerten Lösungsmittels, wie eines gegebenenfalls halogenierten, wie chlorierten, aliphatischen, cycloaliphatischen oder aromatischen Kohlenwasserstoffs, z.B. Methylenchlorid, eines Aethers, wie Dioxan oder Tetrahydrofuran, oder eines Gemisches verwendet. Dabei wendet man vorzugsweise geeignete Kondensationsmittel, wie Alkalimetallcarbonate oder -hydrogencarbonate, z.B. Natrium- oder Kaliumcarbonat oder -hydrogencarbonat (üblicherweise zusammen mit einem Sulfat), oder organische Basen, wie, üblicherweise sterische gehinderte, Triniederalkylamine, z.B. N,N-Diisopropyl-N-äthyl-amin (vorzugsweise zusammen mit Halogensulfonsäure-niederalkylestern oder gegebenenfalls Halogensubstituierten Methansulfonsäure-niederalkylestern) an, wobei unter Kühlen, bei Raumtemperatur

oder unter Erwärmen, z.B. bei Temperaturen von etwa -20°C bis etwa 50°C und, wenn notwendig, in einem geschlossenen Gefäss und/oder in einer Inertgas-, z.B. Stickstoffatmosphäre gearbeitet wird.

Durch Phasentransfer-Katalyse (siehe Dehmlow, Angewandte Chemie, Bd. 5, S. 187 (1974) kann die oben beschriebene Verätherungsreaktion wesentlich beschleunigt werden. Als Phasentransfer-Katalysatoren können quartäre Phosphoniumsalze und insbesondere quartäre Ammoniumsalze, wie gegebenenfalls substituierte Tetraalkylammoniumhalogenide, z.B. Tetrabutylammoniumchlorid, -bromid oder -jodid, oder auch Benzyl-triäthylammoniumchlorid, in katalytischen oder bis zu äquimolaren Mengen verwendet werden. Als organische Phase kann irgendeines der mit Wasser nicht mischbaren Lösungsmittel dienen, beispielsweise einer der gegebenenfalls halogenierten, wie chlorierten niederaliphatischen, cycloaliphatischen oder aromatischen Kohlenwasserstoffe, wie Tri- oder Tetrachloräthylen, Tetrachloräthan, Tetrachlorkohlenstoff, Chlorbenzol, Toluol oder Xylol. Die als Kondensationsmittel geeigneten Alkalimetallcarbonate oder -hydrogencarbonate, z.B. Kalium- oder Natriumcarbonat oder -hydrogencarbonat, Alkalimetallphosphate, z.B. Kaliumphosphat, und Alkalimetallhydroxide, z.B. Natriumhydroxid, können bei basenempfindlichen Verbindungen der Reaktonsmischung titrimetrisch, z.B. mittels eines Titrierautomaten, zugesetzt werden, damit der pH-Wert während der Verätherung zwischen etwa 7 und etwa 8.5 bleibt.

Weitere Verätherungsmittel sind geeignete Acetal-Verbindungen, z.B. gem-Di-niederalkoxy-niederalkane, wie 2,2-Dimethoxy-propan, die in Gegenwart von starken organischen Sulfonsäuren, wie p-Toluolsulfonsäure, und eines geeigneten Lösungsmittel, wie eines Diniederalkyl- oder Niederalkylensulfoxyds, z.B. Dimethylsulfoxyd, zur Anwendung gelangen, oder geeignete Orthoester, z.B. Orthoameisensäuretriniederalkylester, z.B. Orthoameisensäure-triäthylester, die in Gegenwart einer starken Mineralsäure, z.B. Schwefelsäure, oder einer starken organischen Sulfonsäure, wie p-Toluosulfonsäure, und eines

geeigneten Lösungsmittels, wie eines Aethers, z.B. Dioxan, verwendet werden.

Weitere Verätherungsmittel sind entsprechende trisubstituierte Oxoniumsalze (sogenannte Meerweinsalze), oder disubstituierte Carbenium- oder Haloniumsalze, worin die Substituenten die veräthernden Reste sind, beispielsweise Triniederalkyloxoniumsalze, sowie Diniederalkoxycarbenium- oder Diniederalkylhaloniumsalze, insbesondere die entsprechenden Salze mit komplexen, fluorhaltigen Säuren, wie die entsprechenden Tetrafluorborate, Hexafluorphosphate, Hexafluorantimonate oder Hexachlorantimonate. Solche Reagentien sind z.B. Trimethyloxonium- oder Triäthyloxonium-hexafluorantimonat, -hexachlorantimonat, -hexafluorphosphat oder -tetrafluorborat, Dimethoxycarbeniumhexafluorphosphat oder Dimethylbromoniumhexafluorantimonat. Man verwendet diese Verätherungsmittel vorzugsweise in einem inerten Lösungsmittel, wie einem Aether oder einem halogenierten Kohlenwasserstoff, z.B. Diäthyläther, Tetrahydrofuran oder Methylenchlorid, oder in einem Gemisch davon, wenn notwendig, in Gegenwart einer Base, wie einer organischen Base, z.B. eines, vorzugsweise sterisch gehinderten, Triniederalkylamins, z.B. N,N-Diisopropyl-N-äthyl-amin, und unter Kühlen, bei Raumtemperatur oder unter leichtem Erwärmen, z.B. bei etwa -20°C bis etwa 50°C, wenn notwendig, in einem geschlossenen Gefäss und/oder in einer Inertgas-, z.B. Stickstoffatmosphäre.

Weitere Verätherungsmittel sind schliesslich entsprechende 1-substituierte 3-Aryltriazenverbindungen, worin der Substituent den veräthernden Rest, und Aryl vorzugsweise gegebenenfalls substituiertes Phenyl, z.B. Niederalkylphenyl, wie 4-Methylphenyl, bedeutet. Solche Triazenverbindungen sind 3-Aryl-1-niederalkyltriazene, z.B. 3-(4-Methylphenyl)-1-methyl-triazen, 3-(4-Methylphenyl)-1-äthyl-triazen oder 3-(4-methylphenyl)-1-isopropyl-triazen. Diese Reagentien werden üblicherweise in Gegenwart von inerten Lösungsmitteln, wie gegebenenfalls halogenierten Kohlenwasserstoffen

oder Aethern, z.B. Benzol, oder Lösungsmittelgemischen, und unter Kühlen, bei Raumtemperatur und vorzugsweise bei erhöhter Temperatur, z.B. bei etwa 20°C bis etwa 100°C, wenn notwendig, in einem geschlossenen Gefäss und/oder in einer Inertgas-, z.B. Stickstoffatmosphäre verwendet.

In einem Ausgangsmaterial der Formel II worin die Gruppe $R_4$ als funktionell abgewandeltes Carboxyl vorliegt, und andere, gegebenenfalls vorhandene funktionelle Gruppen vorzugsweise geschützt sind, kann die Hydroxygruppe in an sich bekannter Weise verestert werden. So kann man die Hydroxygruppe durch Behandeln des Ausgangsmaterials der Formel II mit einem, den gewünschten Acylrest einer organischen Carbonsäure einführenden Acylierungsmittel in eine Acyloxygruppe umwandeln. Dabei verwendet man die entsprechende Carbonsäure oder ein reaktionsfähiges Derivat davon, insbesondere ein Anhydrid, inkl. ein gemischtes oder inneres Anhydrid einer solchen Säure. Gemischte Anhydride sind z.B. diejenigen mit Halogenwasserstoffsäuren, d.h. die entsprechenden Säurehalogenide, insbesondere -chloride, ferner mit Cyanwasserstoffsäure, oder dann diejenigen mit geeigneten Kohlensäurehalbderivaten, wie entsprechenden -halbestern (wie die z.B. mit einem Halogen-ameisensäure-niederalkyl-, wie Chlorameisensäure-äthylester oder -isobutylester, gebildeten gemischten Anhydride) oder mit gegebenenfalls substituierten, z.B. Halogen, wie Chlor, enthaltenden Niederalkancarbonsäuren (wie die mit Pivalinsäurechlorid oder Trichloressigsäurechlorid gebildeten gemischten Anhydride). Innere Anhydride sind z.B. diejenigen von organischen Carbonsäuren, d.h. Ketene, wie Keten oder Diketen, oder diejenigen von Carbamin- oder Thiocarbaminsäuren, d.h. Isocyanate oder Isothiocyanate. Weitere reaktonsfähige, als Acylierungsmittel verwendbare Derivate von organischen Carbonsäuren sind aktivierte Ester, wie geeignet substituierte Niederalkyl-, z.B. Cyanmethylester, oder geeignet substituierte Phenyl-, z.B. Pentachlorphenyl- oder 4-Nitrophenylester. Die Veresterung kann, wenn notwendig, in Gegenwart von geeigneten Kondensationsmitteln, bei Verwendung von freien Carbonsäuren z.B. in Gegenwart von Carbodiimidverbindungen, wie Dicyclohexylcarbodiimid, oder Carbonylverbindungen, wie Diimidazolylcarbonyl, und bei Verwendung von reaktonsfähigen Säurederivaten z.B. in Gegenwart von basischen Mitteln, wie Triniederalkylaminen, z.B. Triäthylamin, oder heterocyclischen Basen, z.B. Pyridin, durchgeführt werden. Die Acylierungsreaktion kann in Abwesenheit oder in Gegenwart eines Lösungsmittels oder Lösungsmittelgemisches, unter Kühlen, bei Raumtemperatur oder unter Erwärmen, und, wenn notwendig, in einem geschlossenen Gefäss und/oder in einer Inertgas-, z.B. Stickstoffatmosphäre durchgeführt werden. Geeignete Lösungsmittel sind z.B. gegebenenfalls substituierte, insbesondere gegebenenfalls chlorierte, aliphatische, cycloaliphatische oder aromatische Kohlenwasserstoffe, wie Benzol oder Toluol, wobei man geeignete Veresterungsreagentien, wie Essigsäureanhydrid, auch als Verdünnungsmittel verwenden kann.

Eine durch eine organische Sulfonsäure, z.B. Niederalkansulfonsäure, wie Methansulfonsäure, oder eine aromatische Sulfonsäure, wie p-Toluolsulfonsäure, veresterte Hydroxygruppe kann man vorzugsweise durch Behandeln des Ausgangsmaterials der Formel I1 mit einem reaktionsfähigen Sulfonsäurederivat, wie einem entsprechenden Halogenid, z.B. Chlorid, wenn notwendig, in Gegenwart eines Säure-neutralisierenden basischen Mittels, z.B. einer anorganischen oder organischen Base, z.B. in analoger Weise wie die entsprechenden Ester mit organischen Carbonsäuren, bilden.

Die Ueberführung der freien Hydroxygruppe in einem Ausgangsmaterial der Formel I1 in eine, durch eine Halogenwasserstoffsäure veresterte Hydroxygruppe, d.h. in ein Halogenatom, kann in verschiedenartiger Weise, üblicherweise durch Behandeln mit einem halogenierenden, insbesondere chlorierenden Mittel durchgeführt werden.

Geeignete Halogenierungsmittel sind beispielsweise Halogen-Phosphor-Reagentien, die enolische Hydroxylgruppen durch Halogen ersetzen können, wie Di-halogen-triorganyl-phosphorane, Trihalogen-diorganyl-phosphorane oder ein Gemisch bestehend aus einem Triorganylphosphin und einem Tetrahalogenmethan, worin der organische Rest z.B. gegebenenfalls substituiertes, wie Diniederalkylamino enthaltendes Niederalkyl oder vorzugsweise Phenyl sein kann.

Repräsentative Vertreter der genannten Phosphorane sind z.B. Difluor-triphenyl-, Trifluor-diphenyl-, Dichlor-triphenyl-, Trichlor-diphenyl, Dibrom-triphenyl- und Tribrom-diphenylphosphoran, worin eine der Phenylgruppen durch ein geeignetes Polymeres, wie einem mit Divinylbenzol vernetzten Polystyrol, oder dann durch Niederalkyl oder Diniederalkylaminoniederalkyl, wie Dimethylaminomethyl, ersetzt sein kann.

Repräsentative Vertreter der genannten Phosphine sind Triäthyl-, Methyl-propyl-phenyl-, Bis-(3-dimethylaminopropyl)-phenyl- und insbesondere Triphenylphosphin, worin eine der Phenylgruppen durch ein Polymeres, wie einem mit Divinylbenzol vernetzten Polystyrol, ersetzt sein kann.

Tetrahalogenmethane sind z.B. Tetrabrom- und insbesondere Tetrachlorkohlenstoff.

Die Reaktion mit den halogenierenden Phosphorreagentien findet in an sich bekannter Weise, z.B. in einem inerten, aprotischen, bevorzugt polaren Lösungsmittel, wie einem chlorierten Kohlenwasserstoff, z.B. Methylenchlorid, Chloroform, Tetrachlorkohlenstoff oder 1,2-Dichloräthan, einem Nitril, wie Acetonitril oder Benzonitril, oder einem N,N-disubstituierten Carbonsäureamid, wie Dimethylformamid oder N,N-Dimethylacetamid, oder Mischungen davon, je nach Reaktivität des eingesetzten Reagens unter Kühlen oder Erwärmen, d.h. bei Temperaturen zwischen etwa -60°C bis zur Rückflusstemperatur des

verwendeten Lösungsmittels, gegebenenfalls in einer Inertgas-, wie Stickstoffatmosphäre, statt. Bei Verwendung von Triniederalkyl- oder Tris-(diniederalkylamino)-phosphinen und Tetrachlorkohlenstoff oder Tetrabromkohlenstoff wird gewöhnlich in einem Temperaturbereich von -60°C bis -20°C gearbeitet. Bei der Umsetzung mit Phosphoranen kann dem Reaktionsmedium zum Abfangen entstehenden Halogenwasserstoffs eine schwache Base, wie Pyridin oder ein N,N-Diniederalkylanilin, z.B. N,N-Dimethylanilin, zugesetzt werden.

Weitere geeignete Halogenierungsmittel sind entsprechende N,N-disubstituierte Halogen-iminiumhalogenid-Verbindungen, insbesondere der Formel $(Alkyl)_2N^{\oplus}=C(Hal)(H \text{ oder } Alkyl)\ Hal^{\ominus}$, worin Hal Chlor bzw. Brom ist. Diese Verbindungen werden üblicherweise in situ hergestellt, indem man ein geeignetes N,N-disubstituiertes Amid, wie Dimethylformamid oder N,N-Dimethylacetamid, mit einem Chlorierungs- bzw. Bromierungsmittel, wie z.B. Phosgen, Carbonyldibromid, Oxalylchlorid, Oxalylbromid, Thionylchlorid, Thionylbromid, Phosphortrichlorid, Phosphoroxychlorid, Phosphortribromid, Phosphoroxybromid oder Phosphorpentachlorid, insbesondere Phosphortrichlorid oder Phosphortribromid, behandelt.

Die obige Reaktion wird üblicherweise in Gegenwart eines Lösungs- oder Verdünnungsmittels durchgeführt, wobei man neben dem normalerweise im Ueberschuss vorliegenden und auch als Lösungsmittel verwendbaren Amid z.B. auch ätherartige Lösungsmittel, z.B. Tetrahydrofuran oder Dioxan, Halogenkohlenwasserstoffe, z.B. Methylenchlorid, oder Sulfoxide, z.B. Dimethylsulfoxid, verwenden kann.

Die Chlorierungs- und Bromierungsmittel werden üblicherweise in Mengen verwendet, die zwei Aequivalenten des Ausgangsmaterials der Formel I1 entsprechen. Die Reaktion kann man z.B. so durchführen, dass man das Chlorierungs- bzw. Bromierungsmittel unter Kühlen zu einer Lösung des Ausgangsmaterials in Dimethylformamid zugibt, worauf man das Reaktionsgemisch während einigen Stunden bei Raumtemperatur stehen lässt.

Die Chlorierung oder Bromierung kann auch so durchgeführt werden, dass man zunächst das Chlorierungs- bzw. Bromierungsmittel mit dem Amid, insbesondere dem Dimethylformamid mischt, so das Halogeniminiumhalogenid-Reagens bildet, und darauf die Lösung des Ausgangsmaterials im Amid, z.B. Dimethylformamid, dem noch ein zusätzliches Lösungsmittel zugegeben werden kann, oder in einem anderen Lösungsmittel, z.B. Tetrahydrofuran, zufügt. Falls notwendig, werden diese Reaktionen in einer Inertgasatmosphäre durchgeführt.

Die Umwandlung der freien Hydroxygruppe in einem Ausgangsmaterial der Formel II in Fluor kann z.B. durch dessen Behandlung mit einem Reagens der Formel $F_3S$-Am erfolgen, worin Am eine disubstituierte Aminogruppe darstellt; solche Reagentien sind u.a. von Markovskij et al., Synthesis, Bd. 1973, S. 787, beschrieben worden. Die Gruppe Am steht in erster Linie für Diniederalkylamino, z.B. Dimethylamino, Diäthylamino, Aethyl-methyl-amino, Methyl-propylamino, Di-n-propyl-amino oder Diisopropylamino, Niederalkyl-phenyl-amino, z.B. Methyl-phenyl-amino oder Aethyl-phenyl-amino, Niederalkylenamino, z.B. Pyrrolidino oder Piperidino, Oxaniederalkylenamino, z.B. Morpholino, oder gegebenenfalls Aza-Niederalkyl-substituiertes Azaniederalkylenamino, z.B. 4-Methyl-piperazino.

Die Reaktion wird vorzugsweise in einem geeigneten inerten Lösungsmittel, wie einem gegebenenfalls substituierten Kohlenwasserstoff, z.B. Cyclopentan, Cyclohexan, Benzol, Toluol, Xylol, Chlorbenzol, Dichlorbenzol, Pentan, Hexan, Chloroform, und vor allem Methylenchlorid, oder einem Aether, wie Diäthyläther, Tetrahydrofuran oder vor allem Dioxan, falls notwendig, unter Kühlen oder Erwärmen, z.B. in einem Temperaturbereich von etwa -20°C bis etwa 80°C, vorzugsweise von etwa 0°C bis etwa 30°C, und/oder unter einer Inertgasatmosphäre durchgeführt.

Verfahren m) (Austausch von Y gegen Amine AmH): In einer Verbindung der Formel Im ist Y Brom, Jod oder bevorzugt Chlor.

Die Amine Am-H werden in freier Form, d.h. als Ammoniak, Niederalkylamin, wie Methyl-, Aethyl-, Propyl- oder Butylamin, oder Diniederalkylamin, wie Dimethyl-, Diäthyl-, Dipropyl-, Dibutyl-, Methyl-äthyl-, Aethyl-propyl-, Methylbutyl-, Propyl-butylamin, oder dergleichen, oder in Form von Metallamiden davon, beispielsweise als Alkalimetallamide, wie Lithium-, Natrium- oder Kaliumamide, eingesetzt.

Die Austauschreaktion findet bevorzugt in einem inerten organischen Lösungsmittel, wie Benzol, Toluol, Dimethylformamid, Tetrahydrofuran, oder dergleichen, oder, beim Austausch von Y gegen $NH_2$, auch in flüssigem Ammoniak statt, wobei gegebenenfalls die Säure H-Y abfangende Basen, wie tertiäre Amine, z.B. Aethyl-diisopropylamin, Pyridin, oder dergleichen zugefügt werden können.

Die Reaktion erfolgt, je nach Reaktionsfähigkeit, bei Zimmertemperatur oder unter Abkühlen oder Erwärmen, zwischen etwa -70° und etwa +100°C, bevorzugt zwischen etwa -70° und etwa +50°.

Erfindungsgemäss erhältliche Verbindungen der Formel I können ausser gemäss den Verfahren c) bis l) auch auf andere an sich bekannte Weise in andere Verbindungen der Formel I übergeführt werden.

Austausch von Niederalkyl $R_1$ gegen Wasserstoff: In einer Verbindung der Formel I, worin funktionelle Gruppen gegebenenfalls geschützt sind, kann eine Niederalkylgruppe $R_1$ abgespalten und durch Wasserstoff ersetzt werden. Diese Abspaltung kann unter sauren Bedingungen, z.B. wie sie bei der Abspaltung von Schutzgruppen gegeben sind, durchgeführt werden, z.B. bei der Behandlung mit Trifluoressigsäure.

Substituierung einer Aminogruppe: Eine gegebenenfalls monosubstituierte Aminogruppe Am in einer erfindungsgemäss erhältlichen Verbindung der Formel I, worin andere gegebenenfalls vorhandene funktionelle Gruppen, insbesondere diejenigen, die analog wie eine Aminogruppe substituiert werden können, vorzugsweise geschützt sind, kann in an sich bekannter Weise, z.B. durch Behandeln mit einem reaktionsfähigen Ester eines Niederalkanols, wie einem Niederalkylhalogenid, z.B. -chlorid, -bromid oder -jodid, vorzugsweise in Gegenwart eines Lösungsmittels, oder, unter Einführung von Methyl, mit Formaldehyd in Gegenwart von Ameisensäure alkyliert werden. Ferner kann man eine gegebenenfalls monosubstituierte Aminogruppe Am mit einem geeigneten Derivat einer Niederalkancarbonsäure, wie einem entsprechenden Halogenid, z.B. Chlorid, acylieren, und in einer so erhältlichen Niederalkanoylamino-Verbindung die Carbonylgruppe, z.B. durch Behandeln mit einem geeigneten Hydridreduktionsreagens, wie Diboran, zu einer Niederalkylgruppe reduzieren. Die Substitutionsreaktion wird in an sich bekannter Weise, bevorzugt in Gegenwart eines Lösungsmittels, und wenn notwendig, unter Kühlen oder Erwärmen und/oder in einer Inertgasatmosphäre durchgeführt.

Ersatz einer veresterten Hydroxygruppe $R_3$ durch Wasserstoff: Eine gegen Wasserstoff austauschbare veresterte Hydroxygruppe $R_3$ in einer Verbindung der Formel I ist vorzugsweise durch eine starke organische oder anorganische Säure, wie eine starke Mineralsäure, z.B. Halogenwasserstoffsäure, wie Chlorwasserstoffsäure, oder eine starke organische Sulfonsäure, wie eine entsprechende niederaliphatische oder aromatische Sulfonsäure, z.B. Methansulfonsäure oder p-Toluolsulfonsäure, veresterte Hydroxygruppe. So stellt $R_3$ z.B. Halogen, wie Chlor, Niederalkylsulfonyloxy, z.B. Methylsulfonyloxy oder Aethylsulfonyloxy, oder Arylsulfonyloxy, z.B. p-Toluolsulfonyloxy, dar.

Der Ersatz von verestertem Hydroxy $R_3$ in einer Verbindung der Formel I durch Wasserstoff kann entweder in zwei Stufen durch Reduktion der 3,4-Doppelbindung im 3-Cephemring und anschliessende Abspaltung einer Säure der Formel $R_3$-H, oder einstufig durch unmittelbare reduktive Abspaltung von $R_3$ erfolgen.

Beim zweistufigen Austausch von $R_3$ gegen Wasserstoff erfolgt die Reduktion der 3,4-Doppelbindung in Verbindungen der Formel I z.B. durch Behandeln mit geeigneten Hydrid-Reduktionsmitteln. Solche sind in erster Linie komplexe Metallhydride, vorzugsweise entsprechende Borhydride, wie Diboran oder Alkalimetallborhydride, z.B. Natriumborhydrid oder Lithiumborhydrid, ferner Zinkborhydrid, sowie organische Alkalimetallaluminiumhydride, wie Tri-niederalkoxy-alkalimetall-aluminiumhydride, z.B. Tri-(tert.-butyloxy)-lithiumaluminiumhydrid, die man in an sich bekannter Weise, z.B. wie oben unter Verfahren kb) beschrieben, verwendet.

Falls die obige Reduktion in Gegenwart geeigneter Säure-abspaltender Mittel durchgeführt wird, kann die erwünschte 3-unsubstituierte 3-Cephem-Verbindung der Formel I unmittelbar erhalten werden. Es kann aber auch zunächst eine 3-$R_3$-Cepham-Verbindung entstehen, die entweder isoliert oder <u>in situ</u> weiterverarbeitet werden kann.

In der zweiten Verfahrensstufe erfolgt dann die Abspaltung der Elemente einer Säure der Formel $R_3$-H aus der erhaltenen 3-$R_3$-Cepham-Verbindung, wobei man diese vorzugsweise mit geeigneten Säure-abspaltenden Mitteln behandelt.

Die einstufige reduktive Abspaltung einer veresterten Hydroxygruppe $R_3$ aus einer Verbindung der Formel I gelingt am besten, wenn $R_3$ Halogen, wie Chlor, oder eine durch eine organische Sulfonsäure, wie eine niederaliphatische oder aromatische Sulfonsäure, z.B. Methansulfonsäure oder p-Toluolsulfonsäure, veresterte Hydroxygruppe ist. In diesem Fall reduziert man vorzugsweise durch Behandeln mit metallischen Reduktionsmitteln ("nascierendem Wasserstoff"), z.B. mit Aluminium oder insbesondere Zink, oder mit reduzierenden Metallverbindungen, z.B. entsprechenden Metalllegierungen, Amalgamen wie Aluminiumamalgam oder Salzen. Diese wendet man üblicherweise in Gegenwart von schwachen Protonendonatoren, wie Wasser, Alkohol (z.B. Methanol oder Aethanol) oder einer niederaliphatischen Carbonsäure, z.B. Essigsäure oder Ameisensäure an. Mit Zink reduziert man beispielsweise in Gegenwart von Essigsäure oder Ameisensäure, mit einem Amalgam z.B. in Gegenwart eines wasserhaltigen, inerten, organischen Lösungsmittel, wie eines Aethers.

Gewisse veresterte Hydroxygruppen $R_3$, insbesondere organisches Sulfonyloxy, z.B. Methylsulfonyloxygruppen, lassen sich in Form einer Säure der Formel $R_3$-H auch durch Adsorption, z.B. an Silikagel, Aluminiumoxyd, etc., und Elution (Chromatographie), abspalten.

Bevorzugt wird in einer Verbindung der Formel I als veresterte Hydroxygruppe $R_3$ Halogen, z.B. Chlor oder Brom, oder organisches Sulfonyloxy, z.B. p-Toluolsulfonyloxy oder Methansulfonyloxy, die z.B. durch Behandeln mit Zink in Gegenwart einer Säure, wie Eisessig oder Ameisensäure, abgespalten werden.

Die Abspaltung von einer Säure der Formel $R_3$-H wird üblicherweise in Gegenwart eines Lösungsmittels durchgeführt. Bei Anwendung von anorganischen Basen kann man dabei in Wasser, einem wasserlöslichen organischen Lösungsmittel, wie Aceton oder Dioxan, oder wässrigen Gemischen davon und bei einem pH von höchstens 9, bei Verwendung von Aminen vorzugsweise auch in einem gegebenenfalls halogenierten, wie chlorierten, niederaliphatische, cycloaliphatischen oder aromatischen Kohlenwasserstoff, wie Methylenchlorid, einem Niederalkanon, z.B. Aceton, oder Aether, z.B. Tetrahydrofuran oder Dioxan, oder einem entsprechenden Lösungsmittelgemisch, inkl. einem wässrigen Gemisch, wenn notwendig unter Kühlen oder Erwärmen und/oder in einer Inertgas-, z.B. Stickstoffatmosphäre, arbeiten.

Austausch von Sulfonyloxy durch Halogen: Eine Sulfonyloxygruppe $R_3$ in einer Verbindung der Formel I, in erster Linie Niederalkylsulfonyloxy, insbesondere Methylsulfonyloxy, ferner Arylsulfonyloxy, worin Aryl vorzugsweise gegebenenfalls, z.B. durch Niederalkyl, wie Methyl, Halogen, z.B. Brom, oder Nitro substituiertes Phenyl, wie 4-Methylphenylsulfonyloxy, kann in ein Halogen-, insbesondere Fluoratom $R_3$ übergeführt werden. So kann man den Austausch einer organischen Sulfonyloxygruppe durch Fluor mittels Behandeln mit einem anorganischen Fluorid in Gegenwart eines Kronenäthers vornehmen.

Ein anorganisches Fluorid ist in erster Linie ein Metallfluorid, insbesondere ein Alkalimetallfluorid, z.B. Natriumfluorid, oder ein Schwermetallfluorid, z.B. Silberfluorid.

Die zusammen mit dem anorganischen Fluorid verwendeten Kronenäther sind gegebenenfalls substituierte 18-Kronen-6-äther, wie der Dicyclohexyl-18-kronen-6-äther.

Die Reaktion wird in Gegenwart eines inerten Lösungsmittels, insbesondere eines Nitrils, z.B. Acetonitril oder Propionitril, oder eines Nitroniederalkans, z.B. Nitromethan oder Nitroäthan, unter im wesentlichen wasserfreien Bedingungen und, falls notwendig, unter Kühlen, z.B. in einem Temperaturbereich von etwa -20°C bis etwa 25°C, vorzugsweise bei etwa Raumtemperatur, und/oder in einer Inertgasatmosphäre durchgeführt.

Ueberführen von verestertem oder veräthertem Hydroxy in veräthertes Mercapto:
In einer erhaltenen Verbindung der Formel I, worin gegebenenfalls vorhandene funktionelle Gruppen vorzugsweise geschützt sind, kann eine durch veräthertes Mercapto ersetzbare veresterte oder verätherte Hydroxygruppe $R_3$ durch Behandeln mit einem Thiol in eine solche übergeführt werden.

Eine durch ein Thiol substituierbare veresterte Hydroxygruppe $R_3$ ist durch eine geeignete anorganische oder organische Säure, wie durch eine Halogen-, z.B. Chlorwasserstoffsäure, eine Schwefel- oder Phosphor- enthaltende Säure, wie eine Schwefel- oder Sulfonsäure, eine Phosphor-, Phosphon- oder Phosphinsäure, oder eine Carbonsäure verestertes Hydroxy. Eine reaktionsfähige verätherte Hydroxygruppe $R_3$ ist durch einen gegebenenfalls substituierten Kohlenwasserstoffrest veräthert, beispielsweise durch einen aliphatischen, cycloaliphatischen oder araliphatischen Kohlenwasserstoffrest mit z.B. bis zu 15 C-Atomen, wie Niederalkyl, z.B. Methyl oder Aethyl, oder Phenylniederalkyl, wie Benzyl. Vorzugsweise ist der gegen veräthertes Mercapto austauschbare Rest $R_3$ Chlor, Brom, Niederalkansulfonyloxy, wie Methan- oder Aethansulfonyloxy, oder Arylsulfonyloxy, wie Benzol-, p-Toluol-, p-Chlorbenzol- oder p-Nitrobenzolsulfonyloxy.

Die Substitution einer solchen Gruppe $R_3$ durch die verätherte Mercaptogruppe findet in an sich bekannter Weise, d.h. unter Kühlen, bei Raumtemperatur oder unter Erwärmen, z.B. in einem Temperaturbereich zwischen etwa 0°C und etwa 40°C, in einem geeigneten inerten Lösungsmittel und vorzugsweise in Gegenwart einer Base statt. Geeignete Basen sind insbesondere sterisch gehinderte Amine, wie entsprechende Triniederalkylamine, z.B. N,N-Diisopropyl-N-äthylamin, bicylische Amidine, wie Diazabicycloalkane, z.B. 1,5-Diazabicyclo-[4.3.0]non-5-en oder 1,5-Diazabicyclo[5.4.0]undec-5-en, sowie Alkalimetallhydride, -amide oder -niederalkanolate, wie Natriumhydrid, Natrium- oder Lithiumamid, Natriumäthylat oder Kalium-tert.-butylat. Die Reaktion kann, wenn notwendig, in einer Inertgas-, wie Stickstoffatmosphäre, und/oder in einem geschlossenen Gefäss unter Druck durchgeführt werden.

Falls $R_3$ in einer Verbindung der Formel I eine verätherte Hydroxygruppe ist, findet unter den basischen Bedingungen zunächst eine Addition des Thiols an die Doppelbindung statt, worauf der Alkohol $H-R_3$ danach abgespalten werden muss. Die Abspaltung des Al-

kohols $H-R_3$ findet in Gegenwart einer Säure, in An- oder Abwesenheit eines geeigneten, inerten Lösungsmittels und unter Kühlen, bei Raumtemperatur oder unter Erwärmen, d.h. bei Temperaturen zwischen etwa -70°C und etwa +100°C, bevorzugt zwischen etwa 5°C und etwa 40° C, statt. Zur Abspaltung des Alkohols $H-R_3$ geeignete Säuren sind starke organische Protonensäure, insbesondere Mineralsäuren, wie Salzsäure, ferner organische Sulfonsäure, wie Niederalkansulfonsäuren, z.B. Methansulfonsäure, oder aromatische Sulfonsäuren, z.B. Benzol- oder Toluolsulfonsäure, ferner halogenierte Niederalkancarbonsäuren, wie Trifluor- oder Trichloressigsäure, oder auch Ameisensäure.

Veresterung einer freien Carboxylgruppe: Die Umwandlung von freiem Carboxyl, in erster Linie einer entsprechenden Gruppe $R_4$, in einer Verbindung der Formel I in verestertes Carboxyl, insbesondere in eine veresterte Carboxylgruppe, die unter physiologischen Bedingungen spaltbar ist, erfolgt nach an sich bekannten Veresterungsmethoden, beispielsweise indem man eine Verbindung der Formel I, worin andere, gegebenenfalls vorhandene funktionelle Gruppen gegebenenfalls in geschützter Form vorliegen, oder ein reaktionsfähiges funktionelles Carboxyderivat, inkl. ein Salz davon, mit einem entsprechenden Alkohol oder einem reaktionsfähigen funktionellen Derivat davon, umsetzt.

Die Veresterung von freiem Carboxyl mit dem gewünschten Alkohol wird in Gegenwart eines geeigneten Kondensationsmittels durchgeführt. Uebliche Kondensationsmittel sind z.B. Carbodiimide, beispielsweise N,N'-Diäthyl-, N,N'-Dipropyl-, N,N'-Dicyclohexyl- oder N-Aethyl-N'-(3-dimethylaminopropyl)-carbodiimid, geeignete Carbonylverbindungen, beispielsweise Carbonyldiimidazol, oder 1,2-Oxazoliumverbindungen, z.B 2-Aethyl-5-phenyl-1,2-oxazolium-3'-sulfonat und 2-tert.-Butyl-5-methyl-isoxazolium-perchlorat, oder eine geeignete Acylaminoverbindung, z.B. 2-Aethoxy-1-äthoxycarbonyl-1,2-dihydrochinolin. Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran und, wenn notwendig, unter Kühlen oder Erwärmen und/oder in einer Inertgasatmosphäre durchgeführt.

Geeignete reaktionsfähige funktionelle Derivate der zu veresternden Carboxylverbindungen der Formel I sind z.B. Anhydride, insbesondere gemischte Anhydride, und aktivierte Ester.

Gemischte Anhydride sind z.B. diejenigen mit anorganischen Säuren, wie Halogenwasserstoffsäuren, d.h. die entsprechenden Säurehalogenide, z.B. -chloride oder -bromide, ferner Stickstoffwasserstoffsäure, d.h. die entsprechenden Säureazide, sowie phosphorhaltigen Säuren, z.B. Phosphorsäure, Diäthylphosphorsäure oder phosphoriger Säure, oder schwefelhaltigen Säuren, z.B. Schwefelsäure, oder Cyanwasserstoffsäure. Gemischte Anhydride sind weiter z.B. diejenigen mit organischen Carbonsäuren, wie mit gegebenenfalls, z.B. durch Halogen, wie Fluro oder Chlor, substituierten Niederalkancarbonsäuren, z.B. Pivalinsäure oder Trichloressigsäure, oder mit Halbestern, insbesondere Niederalkylhalbestern der Kohlensäure, wie Aethyl- oder Isobutylhalbestern der Kohlensäure, oder mit organischen, insbesondere aliphatischen oder aromatischen, Sulfonsäure, z.B. p-Toluolsulfonsäure.

Zur Reaktion mit dem Alkohol geeignete aktivierte Ester sind z.B. Ester mit vinylogen Alkoholen (d.h. Enolen), wie vinylogen Niederalkenolen, oder Iminomethylesterhalogeniden, wie Dimethyliminomethylesterchlorid (hergestellt aus der Carbonsäure und Dimethylchlormethyliden-iminium-chlorid der Formel $[(CH_3)_2N{=}CHCl]^{\oplus}Cl^{\ominus}$), oder Arylester, wie Pentachlorphenyl-, 4-Nitrophenyl- oder 2,3-Dinitrophenylester, heteroaromatische Ester, wie Benztriazol-, z.B. 1-Benztriazolester, oder Diacyliminoester, wie Succinylimino- oder Phthalyliminoester.

Die Acylierung mit einem solchen Säurederivat, wie einem Anhydrid, insbesondere mit einem Säurehalogenid wird bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyl-diisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Aethylenoxid oder Propylenoxid, durchgeführt.

Ein reaktionsfähiges funktionelles Derivat des veresternden Alkohols ist in erster Linie ein entsprechender Ester, vorzugsweise mit einer starken anorganischen oder organischen Säure und stellt insbesondere ein entsprechendes Halogenid, z.B. Chlorid, Bromid oder Jodid, oder eine entsprechende Niederalkyl- oder Aryl-, wie Methyl- oder 4-Methylphenylsulfonyloxyverbindung dar.

Ein solcher reaktionsfähiger Ester eines Alkohols kann mit der freien Carboxylverbindung der Formel I oder einem Salz, wie einen Alkalimetall- oder Ammoniumsalz davon umgesetzt werden, wobei man bei Verwendung der freien Säure vorzugsweise in Gegenwart eines säurebindenden Mittels arbeitet.

Die obigen Veresterungsreaktionen werden in einem inerten, üblicherweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder

einem Nitril, z.B. Acetonitril, oder Mischungen davon, wenn notwendig, unter Kühlen oder Erwärmen, z.B. in einem Temperaturbereich von etwa bei -40°C bis etwa +100°C, vorzugsweise bei etwa -10°C bis etwa +40°C, und/oder in einer Inertgas-, z.B. Stickstoffatmosphäre.

Ferner kann das Säurederivat, wenn erwünscht, in situ gebildet werden. So erhält man z.B. ein gemischtes Anhydrid durch Behandeln der Carbonsäureverbindung mit entsprechend geschützten funktionellen Gruppen oder eines geeigneten Salzes davon, wie eines Ammoniumsalzes, z.B. mit einem organischem Amin, wie Piperidin oder 4-Methylmorpholin, oder eines Metall-, z.B. Alkalimetallsalzes mit einem geeigneten Säurederivat, wie dem entsprechenden Säurehalogenid einer gegebenenfalls substituierten Niederalkancarbonsäure, z.B. Trichloracetylchlorid, mit einem Halbester eines Kohlensäure-halbhalogenids, z.B. Chlorameisensäurehalbester oder -isobutylester, oder mit einem Halogenid einer Diniederalkylphosphorsäure, z.B. Diäthylphosphorbromidat, und verwendet das so erhältliche gemischte Anhydrid ohne Isolierung.

Bildung des 1-Oxids: Eine Verbindung der Formel I, worin n für 0 steht, kann durch Oxidation, z.B. nach einer der unter Verfahren b) beschriebenen Oxidationsmethoden, in eine Verbindung der Formel I, worin n für 1 steht, übergeführt werden.

Reduktion des 1-Oxids: Eine Verbindung der Formel I, worin n für 1 steht, kann durch Reduktion, z.B. nach einer der unter Verfahren b) beschriebenen Reduktionsmethoden, in eine Verbindung der Formel I, worin n für 0 steht, übergeführt werden.

Abspaltung von Schutzgruppen: In einer erhaltenen Verbindung der Formel I, worin eine oder mehrere funktionelle Gruppe geschützt sind, können diese, z.B. geschützte Carboxyl-, Amino-, Hydroxy- und/oder Sulfogruppen, in an sich bekannter Weise, mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt werden.

So kann man tert.-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B.durch Behandeln mit einer geeigneten Säure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlor-mandelsäure oder Weinsäure, oder eines Alkohols oder Thiols, wobei man vorzugsweise Wasser zugibt, in freies Carboxyl überführen. Durch Behandeln mit einem reduzierenden Metall oder Metallsalz, wie oben beschrieben, kann man auch 2-Halogen-niederalkoxycarbonyl (gegebenenfalls nach Umwandlung einer 2-Brom-niederalkoxycarbonylgruppe in eine entsprechende 2-Jod-niederalkoxycarbonylgruppe) oder Aroylmethoxycarbonyl in freies Carboxyl umwandeln, wobei Aroylmethoxycarbonyl ebenfalls durch Behandeln mit einem nucleophilen, vorzugsweise salzbildenden Reagens, wie Natriumthiophenolat oder Natriumjodid, gespalten werden kann. Substituiertes 2-Silyläthoxycarbonyl kann auch durch Behandeln mit einem, das Fluoridanion liefernden Salz der Fluorwasserstoffsäure, wie einem Alkalimetallfluorid, z.B. Natrium- oder Kaliumfluorid, in Anwesenheit eines macrocyclischen Polyäthers ("Kronenäther"), oder mit einem Fluorid einer organischen quaternären Base, wie Tetra-nie-

deralkylammoniumfluorid oder Triniederalkyl-aryl-ammoniumfluorid, z.B. Tetraäthylammoniumfluorid oder Tetrabutylammoniumfluorid, in Gegenwart eines aprotischen polaren Lösungsmittels, wie Dimethylsulfoxid oder N,N-Dimethylacetamid, in freies Carboxyl übergeführt werden. Mit einer organischen Silyl- oder Stannylgruppe, wie Triniederalkylsilyl oder -stannyl, z.B. Trimethylsilyl, verestertes Carboxyl kann in üblicherweise solvolytisch, z.B. durch Behandeln mit Wasser, einem Alkohol oder Säure freigesetzt werden.

Eine geschützte Aminogruppe, z.B. Am, setzt man in an sich bekannter und je nach Art der Schutzgruppen in verschiedenartiger Weise, vorzugsweise mittels Solvolyse oder Reduktion, frei. 2-Halogen-niederalkoxycarbonylamino (gegebenenfalls nach Umwandlung einer 2-Brom-niederalkoxycarbonylaminogruppe in eine 2-Jod-niederalkoxycarbonylaminogruppe), Aroylmethoxycarbonylamino oder 4-Nitrobenzyloxycarbonylamino kann z.B. durch Behandeln mit einem geeigneten chemischen Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitro-benzyloxycarbonylamino auch durch Behandeln mit einem Alkalimetall-, z.B. Natriumdithionit, gespalten werden. Gegebenenfalls substituiertes Diphenylmethoxycarbonylamino, tert.-Niederalkoxycarbonylamino oder 2-trisubstituiertes Silyläthoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino, Formylamino oder 2-Acyl-niederalk-1-en-1-yl-amino, z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine mit einer organischen Silyl- oder Stannylgruppe geschützte Aminogruppe z.B. mittels Hydrolyse oder Alkoholyse freigesetzt werden. Eine

durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat, des Thioharnstoffs und anschliessende Solvolyse, wie Alkoholyse oder Hydrolyse, des entstandenen Kondensationsprodukts freigesetzt werden. Eine durch 2-substituiertes Silyläthoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxylgruppe angegeben, in die freie Aminogruppe übergeführt werden. Eine Phosphor-, Phosphon- oder Phosphin-amidogruppe kann z.B. durch Behandeln mit einer Phosphorhaltigen Säure, wie einer Phosphor-,Phosphon- oder Phosphinsäure, z.B. Orthophosphorsäure oder Polyphosphorsäure, einem sauren Ester, z.B. Monomethyl-, Monoäthyl-, Dimethyl- oder Diäthylphosphat oder Monomethylphosphonsäure, oder einem Anhydrid davon, wie Phosphorpentoxid, in die freie Aminogruppe übergeführt werden.

Ein in Form einer Azidogruppe geschütztes Amino wird z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20°C bis 25°C, oder auch unter Kühlen oder Erwärmen, durchgeführt.

Eine durch eine geeignete Acylgruppe, eine organische Silyl- oder Stannylgruppe oder durch gegebenenfalls substituiertes 1-Phenylniederalkyl geschützte Hydroxygruppe wird wie eine entsprechend geschützte Aminogruppe freigesetzt. Eine durch 2,2-Dichloracetyl geschützte Hydroxygruppe wird z.B. durch basische Hydrolyse, während eine durch tert.-Niederalkyl oder durch einen 2-oxa- oder 2-thia-aliphatischen oder -cycloaliphatischen Kohlenwasserstoffrest verätherte

Hydroxygruppe durch Acidolyse, z.B. durch Behandeln mit einer Mineralsäure oder einer starken Carbonsäure, z.B. Trifluoressigsäure, freigesetzt.

Eine geschützte, insbesondere veresterte Sulfogruppe wird analog einer geschützten Carboxylgruppe freigesetzt.

Die beschriebenen Spaltungsreaktionen werden unter an sich bekannten Bedingungen durchgeführt, wenn notwendig unter Kühlen oder Erwärmen, in einem geschlossenen Gefäss und/oder in einer Inertgas-, z.B. Stickstoffatmosphäre.

Bevorzugt werden beim Vorhandensein von mehreren geschützten funktionellen Gruppen die Schutzgruppen so gewählt, dass gleichzeitig mehr als eine solche Gruppe abgespalten werden kann, beispielsweise acidolytisch, wie durch Behandeln mit Trifluoressigsäure oder Ameisensäure, oder reduktiv, wie durch Behandeln mit Zink und Essigsäure, oder mit Wasserstoff und einem Hydrierkatalysator, wie einem Palladium/Kohle/Katalysator.

Salzbildung: Salze von Verbindungen der Formel I mit salzbildenden Gruppen können in an sich bekannter Weise hergestellt werden. So kann man Salze von Verbindungen der Formel I mit sauren Gruppen z.B. durch Behandeln mit Metallverbindungen, wie Alkalimetallsalzen von geeigneten organischen Carbonsäuren, z.B. dem Natriumsalz der α-Aethyl-capronsäure, oder mit anorganischen Alkali- oder Erdalkalimetallsalzen, z.B. Natriumhydrogencarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauschreagens. Innere Salze von Verbindungen der Formel I, welche z.B. eine freie Carboxylgruppe enthalten, können z.B. durch Neutralisieren von Salzen, wie Säureadditionssalzen, auf

den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Gemische von Isomeren können in an sich bekannter Weise, z.B. durch fraktionierte Kristallisation, Chromatographie, etc. in die einzelnen Isomeren aufgetrennt werden.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den vorstehend als besonders bevorzugt aufgeführten Verbindungen gelangt.

Ausgangsstoffe: Die im Verfahren zur Herstellung der Verbindungen der vorliegenden Erfindung verwendeten Ausgangsstoffe sind bekannt oder, falls neu, können in an sich bekannter Weise hergestellt werden.

Die Ausgangsverbindungen vom Typ der Formel II, sowie entsprechende Verbindungen mit geschützten funktionellen Gruppen sind bekannt.

Die zur Einführung eines Acylrests einer Säure der Formel III verwendeten Ausgangsstoffe sind bisher nicht bekannt geworden. Sie können in an sich bekannter Weise hergestellt werden. Die Herstellungs-

weise wird anhand des folgenden Reaktionsschemas zur Herstellung von 2-(5-Amino-isothiazol-3-yl)-essigsäureverbindungen illustriert:

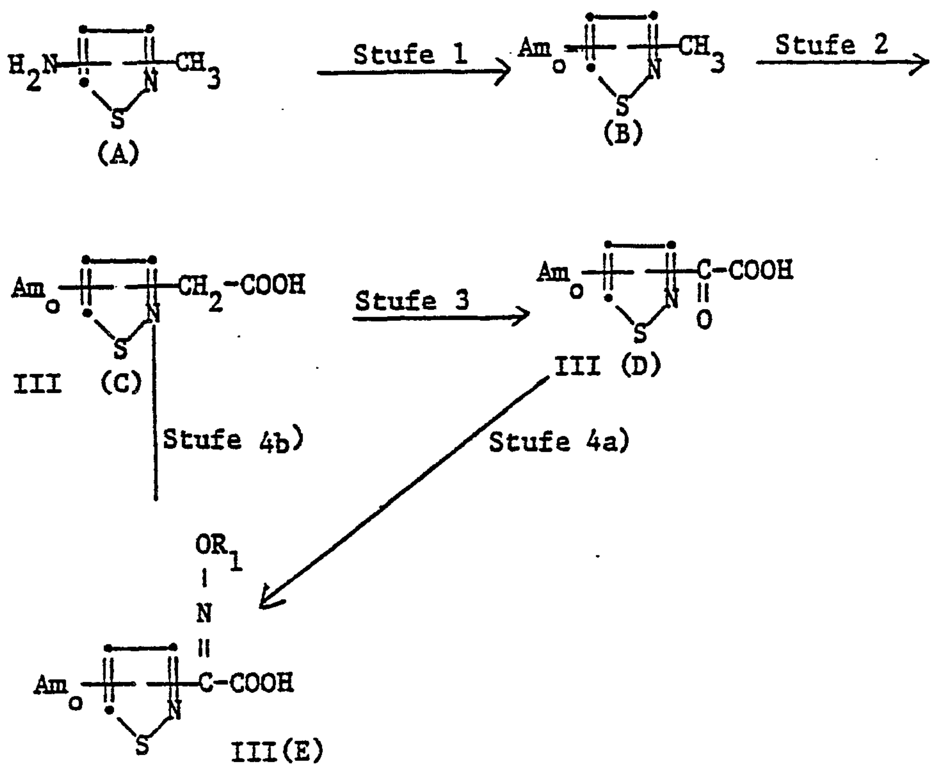


($Am_o$ = geschützte Aminogruppe)

Stufe 1: In einer Verbindung der Formel (A) wird eine primäre oder sekundäre Aminogruppe nach einem der üblichen Verfahren, z.B. Acylierung oder Silylierung und dergleichen, oder Alkylierung, in eine geschützte primäre, sekundäre, geschützte sekundäre, bzw. tertiäre Aminogruppe $Am_o$ übergeführt. Eine geschützte Aminogruppe $Am_o$ ist eine der unter Am aufgeführten geschützten Aminogruppen, insbesondere eine der genannten Acylaminogruppen, wie insbesondere tert.Butoxycarbonylamino oder N-Methyl-tert.butoxycarbonylamino. Eine tertiäre Aminogruppe $Am_o$ ist z.B. eine der genannten Diniederalkylaminogruppen.

Stufe 2: Eine Verbindung der Formel III (C) wird erhalten, indem man eine Verbindung der Formel (B) carboxyliert. Die Carboxylierung erfolgt in an sich bekannter Weise, indem man eine Verbindung der Formel (B)

metalliert und mit Kohlendioxid behandelt. Als Metallierungsmittel kommen insbesondere Organolithiumverbindungen, wie Niederalkyllithium, z.B. Methyl-, Aethyl- oder Butyllithium, Aryllithium, z.B. Phenyllithium, Lithiumamide, wie Lithiumdiniederalkylamide, z.B. Lithiumdiisopropylamid, in Frage. Die Metallierung wird in einem inerten Lösungsmittel, wie einem Aether, z.B. Diäthyläther, Dioxan oder Tetrahydrofuran, oder einem Kohlenwasserstoff, z.B. Hexan oder Benzol, oder einem tertiären Amin oder Diamin, z.B. Tetramethyläthylendiamin, bei erniedrigten Temperaturen, z.B. bei etwa 0°C bis etwa -80°C, durchgeführt. Anschliessend wird bei der gleichen Temperatur Kohlendioxid eingeleitet oder das Metallierungsgemisch wird auf Trockeneis gegossen. Wenn man statt Kohlendioxid einen Halogenameisensäureester benutzt, erhält man einen Ester einer Säure der Formel III (C).

Stufe 3: Eine α-Ketoverbindung der Formel III (D) oder ein Ester davon wird erhalten, indem man eine Verbindung der Formel III (C) oder einen Ester davon mit einem geeigneten Oxidationsmittel behandelt. Zahlreiche solcher Oxidationsmittel sind bekannt. Bevorzugt wird Selendioxid benutzt. Die Reaktion wird in einem inerten Lösungsmittel, wie einem Aether, z.B. Dioxan oder Tetrahydrofuran, einem Amid, z.B. Dimethylformamid, oder einem tertiären Amin, z.B. Pyridin, oder dergleichen, bei erniedrigter oder erhöhter Temperatur, etwa bei 0° bis etwa 100°, bevorzugt bei Zimmertemperatur bis zu etwa 80°, durchgeführt.

Stufe 4a: Eine Verbindung der Formel III (E) kann erhalten werden, indem man eine Verbindung der Formel III (D) mit einem Hydroxylamin der Formel $H_2N-O-R_1$ (V) behandelt. Die Reaktion kann z.B. wie unter Verfahren d) beschrieben durchgeführt werden.

Stufe 4b: Eine Verbindung der Formel III(E) kann auch erhalten werden, indem man eine Verbindung der Formel III (C) nitrosiert und gewünschtenfalls eine erhaltene Verbindung der Formel III (E), worin $R_1$ Wasserstoff bedeutet, in eine Verbindung der Formel III (E) überführt, worin $R_1$ für einen von Wasserstoff verschiedenen Rest $R_1$ steht. Die

Nitrosierung kann z.B. wie unter Verfahren e) und die Substituierung der Hydroxygruppe im Hydroxyiminorest wie unter "Substituierung einer Hydroxyiminogruppe" beschrieben durchgeführt werden.

In einer Verbindung der Formel III (E) kann eine geschützte Aminogruppe $Am_o$ in eine freie Aminogruppe oder in eine andere geschützte Aminogruppe übergeführt werden, oder, falls $R_1$ Wasserstoff ist, kann dieses entsprechend durch einen Rest $R_1$ substituiert werden.

Reaktionsfähige Derivate von Verbindungen der Formel III, die zur Acylierung geeignet sind, werden in an sich bekannter Weise, gegebenenfalls in situ, gebildet.

Verbindungen der Formel IV können als Nebenprodukte bei der Herstellung von Verbindungen der Formel I unter basischen Bedingungen erhalten werden, Sie müssen nicht in reiner Form vorliegen, sondern können im Gemisch mit Verbindungen der Formel I eingesetzt werden.

Verbindungen der Formeln (Ic) bis Il) fallen unter die allgemeine Formel I und können gemäss einem der Verfahren a) oder b) erhalten werden. Auch bei diesen Verbindungen können vorhandene Gruppen Am, $R_1$, $R_2$, $R_3$ oder $R_4$ in andere Gruppen Am, $R_1$, $R_2$, $R_3$ oder $R_4$ übergeführt werden, wobei sinngemäss Verfahren angewendet werden können, wie sie für entsprechende Reaktionen zur Herstellung von Verbindungen der Formel I angegeben sind.

Verbindungen der Formel Im können analog den Verfahren b) bis l) und insbesondere Verfahren a) hergestellt werden, wobei in den betreffenden Ausgangsmaterialien Am durch Y ersetzt ist. Der Ersatz von Am durch Y kann beispielsweise bei einer Verbindung der Formel (A) durch Behandlung mit salpetriger Säure (Diazotierung) gefolgt von einem gewünschten Halogenid, z.B. Kupferhalogenid, z.B. Kupferchlorid oder Kupferbromid, oder auch Kaliumjodid, erfolgen. Ein so erhältliches 3-Methyl-5-Y-isothiazol kann analog den Stufen 1 bis 4 in eine entsprechende 2-(5-Y-Isothiazol-3-yl)essigsäureverbindung übergeführt werden.

Neue Ausgangsverbindungen, sowie Zwischenprodukte und Verfahren zu ihrer Herstellung sind ebenfalls Gegenstand der vorliegenden Erfindung.

Die pharmakologisch verwendbaren Verbindungen der vorliegenden Erfindung können z.B. zur Herstellung von pharmazeutischen Präparaten verwendet werden, welche eine wirksame Menge der Aktivsubstanz zusammen oder im Gemisch mit anorganischen oder organischen, festen oder flüssigen, pharmazeutisch verwendbaren Trägerstoffen enthalten, die sich vorzugsweise zur parenteralen Verabreichung eignen.

Vorzugsweise verwendet man die pharmakologisch wirksamen Verbindungen der vorliegenden Erfindung in Form von parenteral, z.B. intramuskulär oder intravenös, verabreichbaren Präparaten oder von Infusionslösungen. Solche Lösungen sind vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfstoffe, z.B. Konservier-, Stabilisier-, Netz und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Lösungs- oder Lyophilisierungsverfahren, hergestellt und enthalten von etwa 0,1% bis 100%, insbesondere von etwa 1% bis etwa 50%, Lyophilisate bis zu 100% des Aktivstoffes.

Verbindungen der Formel I, insbesondere diejenigen, in welchen $R_4$ eine physiologisch spaltbare, veresterte Carboxylgruppe, z.B. Pivaloyloxymethoxycarbonyl, darstellt, können auch oral, z.B. in Form von Kapseln, verabreicht werden. Diese enthalten den Wirkstoff, gegebenenfalls zusammen mit geeigneten Trägerstoffen, in Form eines Granulats und zwar in Dosen von etwa 0,2 g bis etwa 0,5 g pro Dosiseinheit.

Je nach Art der Infektion und Zustand des infizierten Organismus verwendet man tägliche Dosen von etwa 0,5 g bis etwa 5 g s.c. zur Behandlung von Warmblütern von etwa 70 kg Gewicht.

Die folgenden Beispiele dienen zur Illustration der Erfindung; Temperaturen werden in Celsiusgraden angegeben.

In den Beispielen werden folgende Abkürzungen verwendet:

BOC: tert.-Butyloxycarbonyl
F.: Schmelzpunkt.

Dünnschichtchromatographie: Opti-UPC$_{12}$-Platten der Fa. Antec sind mit Silicagel beschichtete Glasplatten, wobei das Silicagel mit Dodecyltrichlorsilan behandelt wurde (zur Chromatographie mit umgekehrter Phase).

## Herstellung der Ausgangsverbindungen

### Beispiel a): 5-tert.Butoxycarbonylamino-3-methyl-isothiazol

Eine Suspension von 20,0 g (0,174 Mol) 5-Amino-3-methyl-isothiazol in 100 ml BOC-Anhydrid wird bei 100° (Badtemperatur) 12 Stunden gerührt. Das Reaktionsgemisch wird abgekühlt, die flüchtigen Anteile werden im Vakuum entfernt und der teilweise kristalline Rückstand wird durch Umkristallisieren aus Isopropylalkohol gereinigt. Man erhält so die Titelverbindung vom Rf-Wert 0,30 (Silicagel, Toluol/Essigester 2:1. UV-Spektrum (Aethanol) : $\lambda$ max ($\varepsilon$) 256 (13'500) nm. IR-Spektrum (Nujol): charakteristische Absorptionsbanden bei 5.81; 6.41; $\mu$; F.: 134-5°.

### Beispiel b): 2-(5-tert.Butoxycarbonylamino-isothiazol-3-yl) essigsäure.

Eine frisch zubereitete Lösung von Lithiumdiisopropylamid in 100 ml abs. Tetrahydrofuran [aus 60 ml (0,421 Mol) Diisopropylamin und 360 ml (0,720 Mol) einer ca. 2 N Lösung von Butyllithium in Hexan] wird bei -78° unter Stickstoff mit 15,7 g (0,073 Mol) 5-tert.Butoxycarbonylamino-3-methyl-isothiazol in 100 ml abs. Tetrahydrofuran versetzt und 30 Min. bei dieser Temperatur gerührt. Nach Erwärmen des Reaktionsgemisches auf -30° wird 100 g Trockeneis zugegeben. Nach 30 Minuten wird das Gemisch mit 100 ml Wasser versetzt und mit 50 ml Aethylacetat verdünnt. Die organische Phase wird abgetrennt, die wässrige Phase mit 2 N Salzsäure auf pH 2,0 gestellt und mit Aethylacetat extrahiert. Die organische Phase wird abgetrennt, mit wässriger Natriumchloridlösung gewaschen, über Magnesiumsulfat getrocknet und eingeengt. Der Rückstand wird aus Diäthyläther gefällt. Man erhält die Titelverbindung vom Rf-Wert 0.41 (Silicagel; Chloroform: Methanol: Eisessig 75:22:3); F.: 155-157°; UV-Spektrum (Methanol): $\lambda$ max ($\varepsilon$) 206 (5800); 259 (9250) nm; IR-Spektrum (Dioxan): charakteristische Absorptionsbanden bei 3.12; 3.31; 5.80; 6.40 $\mu$.

Beispiel c): 2-(5-tert.-Butoxycarbonylamino-isothiazol-3-yl)-2-oxoessigsäure.

Eine Suspension von 10,0 g (38.75 m Mol) 2-(5-tert.-Butoxycarbonylamino-isothiazol-3-yl)essigsäure und 11,0 g Selendioxid in 150 ml abs. Dioxan wird 1/2 Std. bei 60° gerührt. Nach Filtrieren des Reaktionsgemisches wird das Filtrat im Vakuum eingeengt. Der Rückstand wird mit 150 ml Aethylacetat verdünnt und mit wässriger Natriumbicarbonatlösung extrahiert. Die wässrige Natriumbicarbonatphase wird mit 2 N Salzsäure auf pH 1,0 gestellt und mit Aethylacetat extrahiert. Die organische Phase wird abgetrennt, mit wässriger Natrumchloridlösung gewaschen, über Magensiumsulfat getrockent und eingeengt. Man erhält so die einheitliche Titelverbindung in Form eines Schaumes vom Rf-Wert 0.29 (Silicagel, Chlorofrom:Methanol: Eisessig 75:22:3); IR-Spektrum (Dioxan): charakteristische Absorptionsbanden: 2.94; 5.74; 5,85 μ.

Beispiel d): 2-(5-tert.Butoxycarbonylamino-isothiazol-3-yl)-2-Z-methoxyiminoessigsäure.

Eine Suspension von 5,0 g (18,4 m Mol) 2-(5-tert.-Butoxycarbonylamino-isothiazol-3-yl)-2-oxoessigsäure, 2,66 g (31,8 m Mol) Methoxyaminhydrochlorid und 5,0 ml Pyridin in 100 ml 95% Aethanol wird 3 Std. bei 60°C gerührt. Das Reaktionsgemisch wird im Vakuum eingeengt. Der Rückstand wird mit 100 ml Aethylacetat und 100 ml Wasser verdünnt, die wässrige Phase wird mit 2 N HCl auf pH 2 gestellt und anschliessend mit Aethylacetat extrahiert. Die organische Phase wird abgetrennt, mit wässriger Natriumchloridlösung gewaschen, über Magnesiumsulfat getrocknet und eingeengt. Der Rückstand besteht aus einem Z/E-Isomerengemisch. Die Titelverbindung wird als einheitliches Produkt durch fraktionierte Kristallisation aus Methylenchlorid erhalten. Rf-Wert: 0,30 (Silicagel, Chloroform/Methanol/Essigsäure 75:22:3); F.: 150-154° (Zersetzung): IR-Spektrum Dioxan:charakteristische

Absorptionsbanden bei 3.12; 5.73 und 5.82 µ.

Die Mutterlaugen enthalten eine Mischung des im Titel genannten Z-Isomeren und seines E-Isomeren, sowie geringe Mengen 2-(5-tert.Butoxycarbonylamino-isothiazol-3-yl)-2-hydroxyiminoessigsäure.

Herstellung von Verbindungen der Formel I

Beispiel 1: 7β-[2-(5-tert.Butoxycarbonylamino-isothiazol-3-yl)-2-Z-methoxyiminoacetamido]-3-acetoxymethyl-3-cephem-4-carbonsäure-diphenylmethylester.

Eine Lösung von 1,05 g(3,5 m Mol) 2-(5-tert.Butoxycarbonylamino-isothiazol-3-yl)-2-methoxyiminoessigsäure in 100 ml Methylenchlorid und 0,28 ml Pyridin wird bei -15° unter Stickstoff mit 0.51 ml(6,0 m Mol) Diäthylphosphorbromidat versetzt und 30 Min. bei dieser Temperatur gerührt. Nach Zugabe von 1,4 g (3,2 m Mol) 7β-Amino-3-acetoxymethyl-3-cephem-4-carbonsäure-diphenyl-methylester bei -15° wird das Reaktionsgemisch bei Raumtemperatur während 2,5 Std. gerührt, dann mit 250 ml Methylenchlorid verdünnt, nacheinander mit verdünnter Schwefelsäure, Wasser, wässriger Natriumbicarbonatlösung und wässriger Natriumchloridlösung gewaschen, über Magnesiumsulfat getrocknet und im Vakuum eingeengt. Der Rückstand wird durch präparative Schichtchromatographie an Silicagel mit Chloroform gereinigt. Man erhält die einheitliche Titelverbindung vom Rf-Wert 0,38 (Silicagel, Toluol/Aethylacetat 1:1); IR-Spektrum ($CH_2Cl_2$): charakteristische Absorptionsban en bei 2.94; 5.59; 5.81; 5.92 µ; UV-Spektrum (Methanol): $\lambda$ max (ε) 236 (25350) und 261 (21200) nm.

Beispiel 2: 7β-[2-(5-Amino-isothiazol-3-yl)-2-Z-methoxyiminoacetamido]-3-acetoxymethyl-3-cephem-4-carbonsäure (Natriumsalz).

2,91 g (4 m Mol) 7β-[2-(5-tert.Butoxycarbonylamino-isothiazol-3-yl)-2-methoxyiminoacetamido]-3-acetoxymethyl-3-cephem-4-carbonsäure-

diphenylmethylester werden in 20 ml Trifluoressigsäure und 1,0 ml Anisol aufgelöst. Die Lösung wird 20 Min. bei Raumtemperatur gerührt, mit 20 ml Diäthylaether versetzt und auf ca. 5°C abgekühlt. Der Niederschlag wird durch Filtration isoliert und getrocknet. Eine Lösung des erhaltenen Pulvers in 10 ml Methanol wird mit Natrium - 2 - äthylhexanoat (als 50%-ige methanolische Lösung) auf pH 6,8 gestellt, mit Aethylacetat bis zur Bildung eines Niederschlages versetzt und teilweise eingeengt. Durch Abfiltrieren des Niederschlages erhält man die Titelverbindung vom Rf-Wert 0,54 ($UPC_{12}$-Platten, Wasser/Acetonitril 4:1); F.: 160° (Zersetzung); UV-Spektrum (Methanol): $\lambda$ max ($\varepsilon$): 262 (17500) nm; IR-Spektrum (Nujol): charakteristische Absorptionsbanden bei 2.95; 5.64; 5.78; 5.97; 6.20 $\mu$.

Beispiel 3: 7β-[2-(5-tert.Butoxycarbonylamino-isothiazol-3-yl)-2-Z-methoxyiminoacetamido]-3-(1-methyltetrazol-5-yl-thiomethyl)-3-cephem-4-carbonsäure-diphenylmethylester.

Eine Lösung von 1,60 g (5,31 m Mol) 2-(5-tert.Butoxycarbonylamino-isothiazol-3-yl)-2-methoxyiminoessigsäure in 45 ml Methylenchlorid und 0,60 ml (5,46 m Mol) N-Methylmorpholin wird bei -10° unter Stickstoff mit 0,70 ml (5,35 m Mol) Chlorameisensäureisobutylester versetzt und 30 Min. bei dieser Temperatur gerührt.

Nach Zugabe von 7β-Amino-3-(1-methyltetrazol-5-yl-thiomethyl)-3-cephem-4-carbonsäure-diphenylmethylester in 20 ml Methylenchlorid bei -10°, wird das Reaktionsgemisch bei Raumtemperatur noch 4 Std. gerührt, eingeengt in 250 ml Aethylacetat gelöst und nacheinander mit wässeriger Natriumbicarbandlösung, Wasser und wässeriger Natriumchloridlösung gewaschen, über Magnesiumsulfat getrocknet und im Vakuum eingeengt. Der Rückstand wird an 150 g Silicagel gereinigt. Mit Toluol:Aethylacetat 1:1 eluiert man die einheitliche Titelverbindung vom Rf-Wert 0,40 (Silicagel, Toluol/Aethylacetat 1:1); IR-Spektrum ($CH_2Cl_2$): charakteristische Absorptionsbanden bei 3,07; 5,60; 5,85; 5,93; 6,45 $\mu$.

BAD ORIGINAL

Beispiel 4: 7β-[2-(5-Amino-isothiazol-3-yl)-2-Z-methoxyiminoacetamido]-3-(1-methyltetrazol-5-yl-thiomethyl)-3-cephem-4-carbonsäure (Natriumsalz).

Aus 650 mg (0,85 m Mol) 7β-[2-(5-tert.Butoxycarbonylamino-isothiazol-3-yl]-2-Z-methoxyiminoacetamido]-3-(1-methyltetrazol-5-yl-thiomethyl)-3-cephem-4-carbonsäure-diphenylmethylester in 1,4 ml Methylenchlorid, 0,47 ml Anisol und 6,5 ml Trifluoressigsäure erhält man analog Beispiel 2 die Titelverbindung vom Rf-Wert 0,40 ($UPC_{12}$-Platten, Wasser/Acetonitril 4:1); UV-Spektrum (Wasser): $\lambda$ max (ε) 235 (18800); 264 (21400) nm; IR-Spektrum (Nujol): charakteristische Absorptionsbanden bei 3,00; 5,70; 5,95; 6,48 µ.

Beispiel 5: 7β-[5-tert.Butoxycarbonylamino-isothiazol-3-yl)acetamido]-3-acetoxymethyl-3-cephem-4-carbonsäure-diphenylmethylester.

Aus 1.28 g (4,96 m Mol) 2-(5-tert-Butoxycarbonylamino-isothiazol-3-yl)essigsäure, 0,54 ml (4.96 m Mol) N-Methylmorpholin, 0,65 ml (4.96 m Mol) Chlorameisensäureisobutylester in 40 ml Methylenchlorid und 2,1 g (4,8 m Mol) 7β-Amino-cephalosporansäure-diphenylmethylester in 40 ml Methylenchlorid erhält man analog Beispiel 3 die Titelverbindung vom Rf-Wert 0,30 (Toluol/Aethylacetat 1:1).
UV-Spektrum (Alkohol): $\lambda$ max (ε) 258 (21000); IR-Spektrum (Nujol): charakteristische Absorptionsbanden bei 3,10; 5,60; 5,75; 5,80; 6,00; 6.40 µ.

Beispiel 6: 7β-[2-(5-Amino-isothiazol-3-yl)acetamido]-3-acetoxymethyl-3-cephem-4-carbonsäure Natriumsalz.

Aus 568 mg (0.83 m Mol) 7β-[2-(5-tert.Butoxycarbonylamino-isothiazol-3-yl)acetamido]-3-acetoxymethyl-3-cephem-4-carbonsäure-diphenylmethylester in 5 ml Trifluoressigsäure und 0,2 ml Anisol erhält man analog Beispiel 4 die Titelverbindung vom Rf 0,30. UV-Spektrum ($H_2O/NaHCO_3$): $\lambda$ max (ε) 235 (8600); 262 (11700) nm.

Beispiel 7: 7β-[2-(5-tert.-Butoxycarbonylamino-isothiazol-3-yl)-2-E-methoxyiminoacetamido]-3-acetoxymethyl-3-cephem-4-carbonsäure-diphenylmethylester

Eine Lösung von 0,76 ml (9,96 m Mol) Dimethylformamid in 5 ml Aethylacetat wird bei -5° mit 0,87 ml (9,96 m Mol) Oxalylchlorid versetzt und 10 Min. gerührt. Dann 2,5 g (8,3 m Mol) 2- (5-tert.Butoxycarbonylamino-isothiazol-3-yl)-2-methoxyiminoessigsäure (Z/E-Isomerengemisch aus Beispiel d) in 40 ml Essigester bei -5 bis 0° zugetropft, 45 Min. bei 0° gerührt und 3,63 g (8,3 m Mol) 7β-Amino-cephalosporansäure-diphenylmethylester zugegeben. Nach 2 1/2 Std. bei 0° wird mit Essigester verdünnt, nacheinander mit Wasser, verdünnter Natriumbicarbonatlösung und wässriger Natriumchloridlösung gewaschen, über $MgSO_4$ getrocknet und im Vakuum eingeengt. Der Rückstand wird an Silicagel mit Toluol:Aethylacetat 1:1 chromatographisch getrennt. Man eluiert vorerst das Z-Isomere (Vgl. Beispiel 1) und anschliessend die Titelverbindung vom Rf=0,21 (Silicagel, Toluol/Aethylacetat 1:1).

Beispiel 8: 7β-[2-(5-Amino-isothiazol-3-yl)-2-E-methoxyiminoacetamido] 3-acetoxymethyl-3-cephem-4-carbonsäure Natriumsalz.

190 mg (0,26 m Mol) 7β-[2-(5-tert.Butoxycarbonylamino-isothiazol-3-yl)-2-E-methoxyiminoacetamido]-3-acetoxymethyl-3-cephem-4-carbonsäure-diphenylmethylester werden in 0,1 ml Anisol und 3 ml Trifluoressigsäure gelöst. Nach 5 Min. tritt Kristallisation ein. Die Reaktionsmischung wird eingeengt und mit Diäthyläther versetzt. Die Kristalle werden abfiltriert und in 3 ml Methanol gelöst. Die Lösung wird mit Natrium-2-äthylhexanolat in Methanol neutral gestellt, eingeengt und mit Diäthyläther versetzt, worauf die ausfallende Titelverbindung abgenutscht wird. Rf-Wert: 0,16 ($UPC_{12}$-Platten, Wasser /Acetonitril 9:1). IR-Spektrum (Nujol): charakteristische Absorptionsbanden bei 2.95; 5.65; 6.22, 6.55 μ.

Beispiel 9: 7β-[2-(5-tert.Butoxycarbonylamino-isothiazol-3-yl)-2-E-methoxyiminoacetamido]-3-(1-methyltetrazol-5-yl-thiomethyl)-3-cephem-4-carbonsäure-diphenylmethylester.

Der im Beispiel d) erhaltene Rückstand bestehend aus Z/E-Isomerengemisch sowie geringen Mengen 2-(5-tert.Butoxycarbonylamino-isothiazol-3-yl)2-hydroxyiminoessigsäure wird in der im Beispiel 3 beschriebenen Weise umgesetzt. Auftrennung des erhaltenen Gemisches an Kieselgel-Platten mit Toluol/Essigester 1:1 liefert die Titelverbindung, Rf 0.20 (Toluol/Essigester 1:1), IR-Spektrum (Nujol): 3.07; 5.58; 5.83; 6.14 μ, sowie das mit dem Produkt aus Beispiel 3 identische Z-Isomere.

Beispiel 10: 7β-[2-(5-Aminoisothiazol-3-yl-)-2-E-methoxyiminoacetamido]-3-(1-methyltetrazol-5-yl-thiomethyl)-3-cephem-4-carbonsäure (Natriumsalz).

Aus 7β-[2-(5-tert.Butyloxycarbonylamino-isothiazol-3-yl)-2-E-methoxyiminoacetamido]-3-(1-methyltetrazol-5-yl-thiomethyl)-3-cephem-4-carbonsäure-diphenylmethylester erhält man gemäss Beispiel 4 die Titelverbindung. Rf-Wert: 0.51 (Silicagel n-Butanol/Essigsäure/Wasser 45:45:10); IR-Spektrum (Nujol): charakteristische Absorptionsbanden bei 2.98; 5.66; 6.19 μ; UV-Spektrum ($H_2O$):
$\lambda_{max}$ ($\varepsilon$) 228 (18.400), 262 (20300) nm.

Beispiel 11: 7β-[2-(5-tert.Butyloxycarbonylamino-isothiazol-3-yl)-2-hydroxyiminoacetamido]-3-(1-methyltetrazol-5-yl-thiomethyl)-3-cephem-4-carbonsäure-diphenylmethylester.

Die im Beispiel 9 beschriebene chromatographische Auftrennung ergab als weitere Komponente die Titelverbindung von Rf = 0,31 (Toluol/Essigester 1:1).

Beispiel 12: 7β-[2-(5-Aminoisothiazol-3-yl-)-2-hydroxyiminoacetamido]-3-(1-methyltetrazol-5-yl-thiomethyl)-3-cephem-4-carbonsäure (Natriumsalz).

Aus 7β-[2-(5-tert.Butyloxycarbonylamino-isothiazol-3-yl)-2-hydroxyiminoacetamido]-3-(1-methyltetrazol-5-yl-thiomethyl)-3-cephem-4-carbonsäure-diphenylmethylester erhält man gemäss Beispiel 4 die Titelverbindung. Rf- Wert 0,42 (Silicagel; Butanol/Eisessig/Wasser 45:45:10); Infrarotspektrum (Nujol): charakteristische Absorptionsbanden bei 2.98; 5.66; 5.95; 6,19 μ, UV-Spektrum (Wasser): $\lambda_{max}$ ($\varepsilon$) 228 (18400), 262 (20300) nm.

Beispiel 13: 7β-[2-(5-tert.Butoxyamino-isothiazol-3-yl)-2-Z-methoxy-iminoacetamido]-3-(1-carboxymethyl-tetrazol-5-yl-thiomethyl)-3-cephem-4-carbonsäure-diphenylmethylester.

Eine Lösung von 0,76 ml (9.93 m Mol) Dimethylformamid in 15 ml Aethylacetat wird bei -5° mit 0,84 ml (9.93 m Mol) Oxalylchlorid versetzt, mit 10 ml Aethylacetat verdünnt und 15 Min. bei -5 bis 0° gerührt. Eine Lösung von 2.49 g (8.28 m Mol) 2-(5-tert.Butoxycarbonylamino-isothiazol-3-yl)-2-Z-methoxyiminoessigsäure in 50 ml Aethylacetat wird dazugetropft und weitere 45 Min. bei 0° gerührt (Lösung 1).

Eine Suspension von 3.08g(8.28 mM) 7β-Amino-3-(1-carboxymethyl-tetrazol-5-ylthiomethyl)-3-cephem-4-carbonsäure in 25 ml Methylenchlorid wird mit 4 ml (17 m Mol) N,O-Bis-(trimethylsilyl) acetamid versetzt und die erhaltene Lösung bei 0° zu Lösung 1 getropft. Nach 1 Std. bei 0° und 1 1/2 Std. bei 10° wird das Reaktionsgemisch eingeengt, in Aethylacetat gelöst, nacheinander mit 2 N Salzsäure und Wasser gewaschen und mit wässriger Natriumbicarbonatlösung extrahiert. Die basischen Extrakte werden mit 2 N Salzsäure sauer gestellt und mit Aethylacetat extrahiert. Die organische Phase wird mit kon-

BAD ORIGINAL

zentrierter wässriger Natriumchloridlösung gewaschen, über Magnesiumsulfat getrocknet und im Vakuum eingedampft. Man erhält die Titelverbindung vom Rf-Wert 0,38 (Kieselgel, Butanol/Eisessig/Wasser 45:45:10).

Beispiel 14: 7β-[2-(5-Amino-isothiazol-3-yl)-2-Z-methoxyimino-acetamido]-3-(1-carboxymethyltetrazol-5-yl-thiomethyl)-3-cephem-4-carbonsäure-Dinatriumsalz

Eine Lösung von 2.1g(3.2 m Mol) 7β-[2-(5-tert.Butoxycarbonylamino-isothiazol-3-yl)-2-Z-methoxyimino-acetamido]-3-(1-carboxymethyltetrazol-5-ylthiomethyl)-3-cephem-4-carbonsäure-diphenylmethylester in 25 ml Trifluoressigsäure wird 30 Min. bei Raumtemperatur stehen gelassen, im Vakuum eingeengt und mit Diäthyläther versetzt. Der Niederschlag wird abfiltriert, mit Diäthyläther gewaschen, in 5 ml Methanol gelöst und bis zur Neutralität mit einer methanolischen Lösung von Natrium-2-äthylhexanolat versetzt. Durch Zugabe von Aethylacetat wird die Titelverbindung ausgefällt und dann abfiltriert. Rf-Wert 0.25 (Kieselgel-Platten, Butanol/Eisessig/Wasser 45:45:10); IR Spektrum (Nujol): charakteristische Absorptionsbanden bei 2.95; 5.65; 6.18; 6.55 μ.

Beispiel 15: 7β-[2-(5-tert.Butoxycarbonylamino-isothiazol-3-yl)-2-Z-methoxyiminoacetamido]-3-cephem-4-carbonsäurediphenylmethylester

Eine Suspension von 67 ml Methylenchlorid, 1,32 ml Dimethylformamid und 1,50 ml (17,49 mMol) Oxalsäuredichlorid wird bei -10° 30 Min. unter Stickstoff gerührt. Nach Zugabe von 6,0 g (19,93 mMol) 2-(5-tert.-Butoxycarbonylamino-isothiazol-3-yl)-2-Z-methoxyiminoessigsäure und 1,67 ml (15,21 mMol) N-Methylmorpholin zur Suspension wird die entstandene klare Lösung 30 Min. bei 0°-5° unter Stickstoff gerührt (Lösung I).

Eine frisch hergestellte Lösung von 6 g (16,36 mMol) 7β-Amino-3-cephem-4-carbonsäure-diphenylmethylester in 125,5 ml Methylenchlorid

wird bei -20° innert 10 Min. zur Lösung I zugetropft und 3 Std. bei einer Temperatur von 0°-5° unter Stickstoff gerührt.

Die Reaktionslösung wird nacheinander mit 1N Salzsäure, Phosphatpuffer pH 8,0 und mit wässriger Natriumchloridlösung gewaschen, über Magnesiumsulfat getrocknet und am Vakuum eingedampft. Der Rückstand wird an Kieselgel chromatographiert. Man erhält die Titelverbindung vom Rf-Wert 0,48 (Silicagel, Toluol-Essigester 1:1), IR-Spektrum (Nujol) charakteristische Absorptionsbanden bei 5.57; 5.82; 5.90; 6,42 μ.

Beispiel 16: 7β-[2-(5-tert.Butoxycarbonylamino-isothiazol-3-yl)-2-Z-methoxyiminoacetamido]-3-cephem-4-carbonsäure Natriumsalz

Eine Lösung von 5,10 g (7,84 mMol) 7β-[2-(5-tert.Butoxycarbonylamino-isothiazol-3-yl)-2-Z-methoxyiminoacetamido]-3-cephem-4-carbonsäurediphenylmethylester in 5.0 ml Methylenchlorid und 24 ml (0,31 Mol) Trifluoressigsäure wird bei einer Temperatur von 0°-5° 30 Min. unter Stickstoff gerührt. Nach Zugabe einer Lösung von Toluol-Aether 1:1 wird das Gemisch bei 30° Badtemperatur an Wasserstrahlvakuum zur Trockne eingedampft. Der feste Rückstand wird mit Aether verrührt, abfiltriert und am Hochvakuum getrocknet.

Das trockene Produkt wird in 25 ml Methanol gelöst und unter Eiskühlung langsam mit 3 ml Natrium-2-äthylhexanoat versetzt. Die Titelverbindung fällt als Niederschlag aus, welcher anschliessend abfiltriert und am Hochvakuum getrocknet wird. Rf-Wert 0,6 (Silicagel, Eisessig-Wasser-N-Butanol 45:10:45).

Beispiel 17: 7β-[2-(5-tert.Butoxycarbonylamino-isothiazol-3-yl)-2-Z-methoxyiminoacetamido]-3-cephem-4-carbonsäure-pivaloyloxymethylester

Zu einer auf 0°-5° vorgekühlten Lösung von 3,2 g (6,33 mMol) 7β-[2-(5-tert.Butoxycarbonylamino-isothiazol-3-yl)-2-Z-methoxyiminoacetamido]-3-cephem-4-carbonsäure Natriumsalz in 32,2 ml Dimethylformamid gibt man eine Lösung von 1,75 ml Jodmethylpivalat in 0,80 ml

Dimethylformamid und rührt 30 Min. bei 0°-5° unter Stickstoff.

Die Lösung wird mit Essigsäure-äthylester verdünnt, nacheinander mit Wasser, verdünnter wässriger Natriumbicarbonatlösung und wässriger Natriumchloridlösung gewaschen, über Magnesiumsulfat getrocknet und am Vakuum zur Trockne eingedampft. Man erhält die Titelverbindung mit dem Rf-Wert von 0,42 (Silicagel, Toluol-Essigsäureäthylester 1:1).

Beispiel 18: 7β-[2-(5-Amino-isothiazol-3-yl)-2-Z-methoxyiminoacetamido]-3-cephem-4-carbonsäure-pivaloyloxymethylester

Eine Lösung von 3,2 g (5,35 mMol) 7β-[2-(5-tert.Butoxycarbonylamino-isothiazol-3-yl)-2-Z-methoxyiminoacetamido]-3-cephem-4-carbonsäure-pivaloyloxymethylester in 16,0 ml (0,20 Mol) Trifluoressigsäure und 16 ml Methylenchlorid wird unter Stickstoff 1 Std. bei Raumtemperatur gerührt, dann mit Toluol/Chloroform 1:1 (30 ml) versetzt und bei 30° Badtemperatur zur Trockne eingedampft.

Der Rückstand wird mit Aether/Hexan 2:1 verrieben, abfiltriert, getrocknet und in 50 ml Essigester gelöst. Die Lösung wird nacheinander mit verdünnter, wässriger Natriumbicarbonatlösung, Wasser und wässriger Natriumchloridlösung gewaschen, über Magnesiumsulfat getrocknet und am Vakuum zur Trockne eingedampft. Der Rückstand wird an Kieselgel chromatographiert. Man erhält die Titelverbindung vom Rf-Wert 0,26 (Silicagel, Toluol-Essigester 1:1), IR-Spektrum (Methylenchlorid) charakteristische Absorptionsbanden bei 5.55; 5.68; 5.92 μ.

Beispiel 19: 7β-[2-(5-Amino-isothiazol-3-yl)-2-Z-methoxyiminoacetamido]-3-(4-carbamoylpyridiniomethyl)-3-cephem-4-carboxylat

Zu einer auf 70° vorgewärmten Lösung von 2,78 g (18,55 mMol) Natriumjodid und 0,28 g (2,29 mMol) Isonicotinsäureamid in 1,40 ml Wasser gibt man 0,1 ml (1,73 mMol) Essigsäure zu.

Anschliessend werden 0,81 g (1,69 mMol) 7β-[2-(5-Amino-isothiazol-3-yl)-2-Z-methoxyiminoacetamido]-3-acetoxymethyl-3-cephem-4-carbonsäure-natriumsalz zur Lösung gegeben und 2 Std. bei 70° gerührt.

Die Reaktionslösung wird unter Kühlung auf -10° in 60 ml Aceton eingetragen. Der gebildete Niederschlag wird abfiltriert, mit Aceton gewaschen und getrocknet. Das Produkt wird präparativ auf $UPC_{12}$-Platten im System Wasser-Acetonitril 4:1 gereinigt. Man erhält die Titelverbindung mit dem Rf-Wert 0,34 ($UPC_{12}$-Platten, Wasser-Acetonitril 4:1).

Beispiel 20: In analoger Weise zu den vorstehenden Beispielen werden, ausgehend von entsprechenden Ausgangsmaterialien, die folgenden Verbindungen erhalten:

7β-[2-(5-Amino-isothiazol-3-yl)-2-Z-methoxyiminoacetamido]-3-(2-methyl-1,3,4-thiadiazol-5-yl-thiomethyl)-3-cephem-4-carbonsäure-natriumsalz, IR-Spektrum (Nujol): Banden bei 2.98; 5.70; 5.96; 6.49 µ.

7β-[2-(5-Amino-isothiazol-3-yl)-2-Z-methoxyiminoacetamido]-3-[1-(2-sulfomethyl)-1H-tetrazol-5-yl-thiomethyl]-3-cephem-4-carbonsäure-natriumsalz,IR-Spektrum (Nujol): Banden bei 2.95; 5.66; 6.20; 6.51 µ.

7β-[2-(5-Amino-isothiazol-3-yl)-2-Z-methoxyiminoacetamido]-3-(3-hydroxypyridazin-6-yl-thiomethyl)-3-cephem-4-carbonsäure-natriumsalz. IR-Spektrum (Nujol): Banden bei 2.98; 5.67; 6.21 µ.

7β-[2-(5-Amino-isothiazol-3-yl)-2-Z-(2-carboxyprop-2-yloxyimino)acetamido]-3-(1-methyltetrazol-5-yl-thiomethyl)-3-cephem-4-carbonsäure-natriumsalz, IR-Spektrum (Nujol): Banden bei 2.97; 5.67:, 5.99; 6.23 µ.

7β-[2-(5-Amino-isothiazol-3-yl)-2-Z-(2-carboxyprop-2-yloxyimino)acetamido]-3-acetoxymethyl-3-cephem-4-carbonsäure-natriumsalz, IR-Spektrum (Nujol): Banden bei 3.04; 5.66; 6.25; 6.58 µ.

7β-[2-(5-Amino-isothiazol-3-yl)-2-Z-(2-carboxymethoxyimino)acetamido]-3-acetoxymethyl-3-cephem-4-carbonsäure-natriumsalz, IR-Spektrum (Nujol): Banden bei 3.03; 5.69; 6.24; 6.55 µ.

7β-[2-(5-Amino-isothiazol-3-yl)-2-Z-carbamoyloxyiminoacetamido]-3-acetoxymethyl-3-cephem-4-carbonsäure-natriumsalz, IR-Spektrum (Nujol): Banden bei 3.03; 5.7 (breit); 6.26 µ.

7β-[2-(5-Amino-isothiazol-3-yl)-2-Z-(1-carboxycyclopropyloxyimino)acetamido]-3-(1-methyltetrazol-5-ylthiomethyl-3-cephem-4-carbonsäure-natriumsalz, IR-Spektrum (Nujol): Banden bei3.01; 5.66; 6.23; 6.55 µ.

7β-[2-(5-Amino-isothiazol-3-yl)-2-Z-methoxyiminoacetamido]-3-carbamoyloxymethyl-3-cephem-4-carbonsäure-natriumsalz, IR-Spektrum (Nujol): Banden bei 3.01; 5.67; 5.74; 6.51 µ.

7β-[2-(5-Amino-isothiazol-3-yl)-2-Z-(2-carboxyprop-2-yloxyimino)acetamido]-3-(4-carbamoylpyridiniomethyl)-3-cephem-4-carboxylat, IR-Spektrum (Nujol): Banden bei 3.05; 5.66; 6.21 µ.

7β-[2-(5-Amino-isothiazol-3-yl)-2-Z-methoxyiminoacetamido]-3-(1-methyl-1H-tetrazol-5-yl-thiomethyl)-3-cephem-4-carbonsäurepivaloyloxymethylester, IR-Spektrum (Nujol): Banden bei 5.68; 5.93 µ.

7β-[2-(5-Amino-isothiazol-3-yl)-2-Z-methoxyiminoacetamido]-3-(1,2,5,6-tetrahydro-2-methyl-5,6-dioxo-1,2,4-triazin-3-ylthiomethyl-3-cephem-4-carbonsäure-dinatriumsalz, IR-Spektrum (Nujol): Banden bei 5.67; 6.00; 6.29 µ.

Beispiel 21: Trockenampullen oder Vials, enthaltend 0,5 g Wirksubstanz, z.B. 7β-[2-(5-Amino-isothiazol-3-yl)-2-Z-methoxyiminoacetamido]-3-(1-methyltetrazol-5-yl-thiomethyl)-3-cephem-4-carbonsäure (Natriumsalz), werden wie folgt hergestellt:

Zusammensetzung (für 1 Ampulle oder Vial)

| | |
|---|---|
| Wirksubstanz | 0,5 g |
| Mannit | 0,05g |

Eine sterile, wässrige Lösung der Wirksubstanz und des Mannits wird unter aseptischen Bedingungen in 5 ml.- Ampullen oder 5 ml.-Vials verschlossen und geprüft.

Beispiel 22: Trockenampullen oder Vials, enthaltend 0,25 g Wirksubstanz, z.B. 7β-[2-(5-Amino-isothiazol-3-yl)-2-Z-methoxyiminoacetamido]-3-(1-methyltetrazol-5-yl-thiomethyl)-3-cephem-4-carbonsäure (Natriumsalz), werden wie folgt hergestellt:

Zusammensetzung (für 1-Ampulle oder Vial)

| | |
|---|---|
| Wirksubstanz | 0,25 g |
| Mannit | 0,03 g |

Eine sterile, wässrige Lösung der Wirksubstanz und des Mannits wird unter aseptischen Bedingungen in 5 ml.-Ampullen oder 5 ml.-Vials verschlossen und geprüft.

Patentansprüche: (für alle benannten Länder ausser Oesterreich)

1. 7β-Aminoisothiazolylacetamido-3-cephem-4-carbonsäureverbindungen der Formel

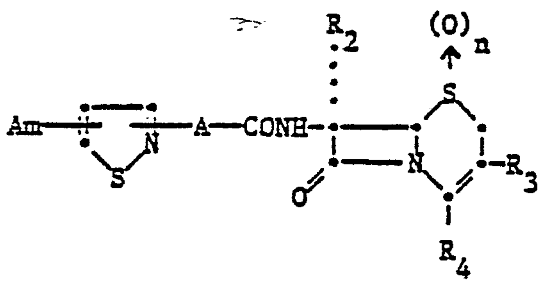

(I),

worin n für 0 oder 1 steht, Am eine gegebenenfalls geschützte Aminogruppe darstellt, A eine Gruppe $-CH_2-$, $-C(=O)-$, oder $-C(=N-O-R_1)-$ bedeutet, worin $R_1$ für Wasserstoff, gegebenenfalls substituiertes Niederalkyl oder Cycloalkyl, oder gegebenenfalls N-substituiertes Carbamoyl steht, $R_2$ Wasserstoff oder Niederalkoxy bedeutet, $R_3$ für Wasserstoff, veräthertes oder verestertes Hydroxy, veräthertes Mercapto, Formyl oder den Rest der Formel $-CH_2-R_3^o$ steht, worin $R_3^o$ für Wasserstoff, Hydroxy, Acyloxy, Heterocyclylthio oder Ammonio steht, und $R_4$ eine gegebenenfalls geschützte Carboxylgruppe darstellt, und Salze von solchen Verbindungen, die eine salzbildende Gruppierung aufweisen.

2. Verbindungen der Formel I nach Patentanspruch 1, worin n für 0 oder 1 steht, Am Amino oder Methylamino bedeutet, A eine Gruppe $-CH_2-$, $-C(=O)-$ oder $-C(=N-OR_1)-$ bedeutet, worin $R_1$ für Wasserstoff, Niederalkyl, durch Carboxy substituiertes Niederalkyl oder Cycloalkyl, oder für gegebenenfalls N-niederalkyliertes Carbamoyl steht, $R_2$ Wasserstoff oder Methoxy darstellt, $R_3$ für Wasserstoff, Niederalkoxy, Halogen, Niederalkylthio, Heterocyclylthio, worin Heterocyclyl für einen fünf- oder sechsgliedrigen monocyclischen, gegebenenfalls partiell gesättigten, aromatischen Heterocyclylrest steht, der mindestens 1 Ringstickstoffatom und gegebenenfalls 1-3 zusätzliche Ringstickstoffatome und/oder ein Ringsauerstoff- oder Ringschwefelatom enthält, und der durch gegebenenfalls Substituenten,

wie Hydroxy, Amino, Diniederalkylamino, Carboxy, Carbamoyl oder Sulfo enthaltendes Niederalkyl, durch gegebenenfalls Substituenten, z.B. Niederalkyl, Niederalkoxy und/oder Halogen enthaltendes Phenyl, oder durch Thienyl substituiert sein kann, oder für den Rest der Formel $-CH_2-R_3^o$ steht, worin $R_3^o$ Niederalkanoyloxy, Carbamoyloxy, N-Niederalkylcarbamoyloxy, gegebenenfalls substituiertes, mit einem Ringkohlenstoffatom an die Thiogruppe gebundenes Heterocyclylthio, wie Triazolylthio, Tetrazolylthio, Thiazolylthio, Thiatriazolylthio, Thiadiazolylthio, Oxazolylthio, Oxadiazolylthio, Pyridazinylthio, Pyrimidinylthio oder Triazinylthio, worin die heterocyclischen Ringe partiell hydriert und gegebenenfalls durch Niederalkyl, N,N-Diniederalkylaminoniederalkyl, Carboxyniederalkyl, Sulfoniederalkyl, Amino, Carboxyniederalkanoylamino, Carbamoyl, Oxo oder Hydroxy substituiert sein können, oder gegebenenfalls durch Carbamoyl substituiertes Pyridinio bedeutet, und $R_4$ Carboxyl oder in physiologisch spaltbarer Form verestertes Carboxyl darstellt, und pharmazeutisch verwendbare Salze von solchen Verbindungen.

3. Verbindungen der Formel I nach Patentanspruch 1, worin n für 0 steht, Am Amino oder Methylamino bedeutet, A eine Gruppe der Formel $-C(=N-O-R_1)$ bedeutet, worin $R_1$ für Wasserstoff, Methyl, 2-Carboxy-2-propyl oder 1-Carboxy-1-cyclopropyl steht, $R_2$ Wasserstoff oder Methoxy darstellt, $R_3$ für Wasserstoff, Niederalkoxy, Halogen, Heterocyclylthio, worin Heterocyclyl gegebenenfalls durch Niederalkyl substituiertes Oxadiazolyl oder Thiadiazolyl, gegebenenfalls durch Niederalkyl substituiertes Triazolyl, oder gegebenenfalls durch Niederalkyl, z.B. Methyl oder Aethyl, das Diniederalkylamino, z.B. Dimethylamino, Carboxy, Carbamoyl oder Sulfo als Substituenten enthalten kann, substituiertes 1H-Tetrazol-5-yl bedeutet, oder einen Rest der Formel $-CH_2-R_3^o$ steht, worin $R_3^o$ Acetoxy, Carbamoyloxy, gegebenenfalls durch Niederalkyl oder Carboxyniederalkyl substituiertes Triazolylthio gegebenenfalls durch Niederalkyl, Carboxyniederalkyl, Sulfoniederalkyl

oder Diniederalkylaminoniederalkyl substituiertes Tetrazolylthio, gegebenenfalls durch Niederalkyl substituiertes Thiadiazolylthio, gegebenenfalls durch Hydroxy substituiertes Pyridazinylthio, gegebenenfalls durch Hydroxy substituiertes Pyrimidinylthio, oder durch Niederalkyl und zwei Hydroxy substituiertes Triazinylthio darstellt und $R_4$ Carboxyl oder in physiologisch spaltbarer Form verestertes Carboxyl bedeutet, und pharmazeutisch verwendbare Salze von solchen Verbindungen.

4. Verbindungen der Formel I nach Patentanspruch 1, worin n für 0 steht, Am in 5-Stellung des Isothiazolringes steht und Amino bedeutet, A mit der 3-Stellung des Isothiazolringes verknüpft ist und eine Gruppe $-C(=N-O-R_1)-$ in syn(Z)-Konfiguration bedeutet, worin $R_1$ für Methyl steht, $R_2$ Wasserstoff darstellt, $R_3$ für Wasserstoff oder eine Gruppe $-CH_2R_3^o$ steht, worin $R_3^o$ Niederalkanoyloxy, wie Acetoxy, oder gegebenenfalls durch Methyl oder Carboxyniederalkyl substituiertes Tetrazolylthio darstellt und $R_4$ Carboxyl bedeutet und pharmazeutisch verwendbare Salze von solchen Verbindungen.

5. 7β-[2-(5-Amino-isothiazol-3-yl)-2-Z-methoxyiminoacetamido]-3-acetoxymethyl-3-cephem-4-carbonsäure und pharmazeutisch verwendbare Salze davon nach Patentanspruch 1.

6. 7β-[2-(5-Amino-isothiazol-3-yl)-2-Z-methoxyiminoacetamido]-3-(1-methyltetrazol-5-yl-thiomethyl)-3-cephem-4-carbonsäure und pharmazeutisch verwendbare Salze davon nach Patentanspruch 1.

7. 7β-[2-(5-Amino-isothiazol-3-yl)acetamido]-3-acetoxymethyl-3-cephem-4-carbonsäure und pharmazeutisch verwendbare Salze davon nach Patentanspruch 1.

8. 7β-[2-(5-Amino-isothiazol-3-yl)-2-E-methoxyiminoacetamido] 3-acetoxymethyl-3-cephem-4-carbonsäure und pharmazeutisch verwendbare Salze davon nach Patentanspruch 1.

9. 7β-[2-(5-Aminoisothiazol-3-yl-)-2-E-methoxyiminoacetamido]-3-(1-methyltetrazol-5-yl-thiomethyl)-3-cephem-4-carbonsäure und

pharmazeutisch verwendbare Salze davon nach Patentanspruch 1.

10. 7β-[2-(5-Aminoisothiazol-3-yl-)-2-hydroxyiminoacetamido]-3-(1-methyltetrazol-5-yl-thiomethyl)-3-cephem-4-carbonsäure und pharmazeutisch verwendbare Salze davon nach Patentanspruch 1.

11. 7β-[2-(5-Amino-isothiazol-3-yl)-2-Z-methoxyimino-acetamido]-3-(1-carboxymethyltetrazol-5-yl-thiomethyl)-3-cephem-4-carbonsäure und pharmazeutisch verwendbare Salze davon nach Patentanspruch 1.

12. 7β-[2-(5-Amino-isothiazol-3-yl)-2-Z-methoxyiminoacetamido]-3-cephem-4-carbonsäure und pharmazeutisch verwendbare Salze davon nach Patentanspruch 1.

13. 7β-[2-(5-Amino-isothiazol-3-yl)-2-Z-methoxyiminoacetamido]-3-cephem-4-carbonsäure-pivaloyloxymethylester nach Patentanspruch 1.

14. 7β-[2-(5-Amino-isothiazol-3-yl)-2-Z-methoxyiminoacetamido]-3-(4-carbamoylpyridiniomethyl)-3-cephem-4-carboxylat nach Patentanspruch 1.

15. Das Natriumsalz einer Verbindung der Formel I gemäss einem der Patentansprüche 1-11.

16. Das Natriumsalz einer Verbindung der Formel I gemäss einem der Ansprüche 12 - 14.

17. Pharmazeutisch verwendbare Präparate enthaltend Verbindungen der Formel I gemäss Patentanspruch 1 oder pharmazeutisch verwendbare Salze von solchen Verbindungen mit salzbildenden Gruppen.

18. Verbindungen der Formel I gemäss Patentanspruch 1 und pharmazeutisch verwendbarer Salze von solchen Verbindungen mit salzbildenden Gruppen zur Verwendung als antibiotisch wirksame Verbindungen bei der Bekämpfung von bakteriellen Infektionen.

19. Verbindungen der Formel I gemäss Patentanspruch 1 und pharmazeutisch verwendbare Salze von solchen Verbindungen mit salzbildenden Gruppen als antibiotisch wirksame Mittel.

20. Verwendung von Verbindungen der Formel I gemäss Patentanspruch 1 und pharmazeutisch verwendbare Salze von solchen Verbindungen mit salzbildenden Gruppen zur Herstellung von pharmazeutischen Präparaten.

21. Verfahren zur Herstellung von Verbindungen der Formel I gemäss Patentanspruch 1 und Salzen von solchen Verbindungen mit salzbildenden Gruppen, dadurch gekennzeichnet, dass man

a) in einer Verbindung der Formel

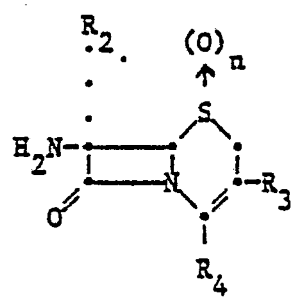

(II),

worin die 7β-Aminogruppe gegebenenfalls durch eine die Acylierung erlaubende Gruppe substituiert ist, und worin weitere gegebenenfalls vorhandene reaktionsfähige funktionelle Gruppen geschützt sein können, die 7β-Aminogruppe durch Behandeln mit einem den Acylrest einer Carbonsäure der Formel

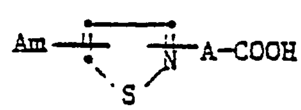

(III)

einführenden Acylierungsmittel, worin Am und A die oben angegebenen Bedeutungen haben, worin weitere gegebenenfalls vorhandene reaktionsfähige funktionelle Gruppen geschützt sein können, acyliert oder

b) eine 2-Cephemverbindung der Formel

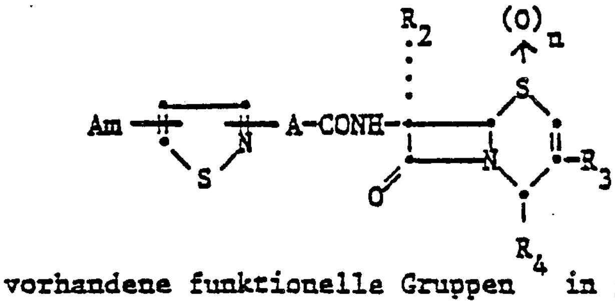

(IV),

worin gegebenenfalls vorhandene funktionelle Gruppen in geschützter Form vorliegen können, zur entsprechenden 3-Cephemverbindung isomerisiert, oder

c) zur Herstellung einer Verbindung der Formel (I), worin A eine Gruppe der Formel -C(=O)- ist, eine Verbindung der Formel

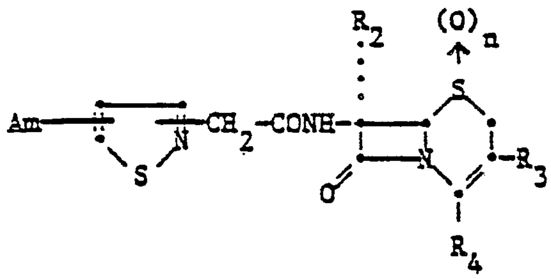

(Ic),

worin gegebenenfalls vorhandene funktionelle Gruppen in geschützter Form vorliegen können, mit einem geeigneten Oxidationsmittel behandelt, oder

d) zur Herstellung einer Verbindung der Formel (I), worin A eine Gruppe der Formel $-C(=N-O-R_1)-$ ist, eine Verbindung der Formel

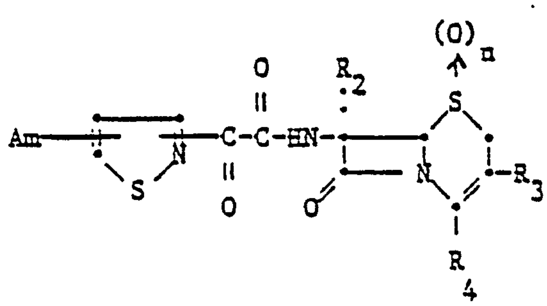

(Id),

worin gegebenenfalls vorhandene funktionelle Gruppen in geschützter Form vorliegen können, mit einem gegebenenfalls O-substituierten Hydroxylamin der Formel $H_2N-O-R_1$ (V) umsetzt, oder

e) zur Herstellung einer Verbindung der Formel (I), worin A eine Gruppe der Formel $-C(=N-O-R_1)-$ ist, worin $R_1$ Wasserstoff bedeutet, eine Verbindung der Formel

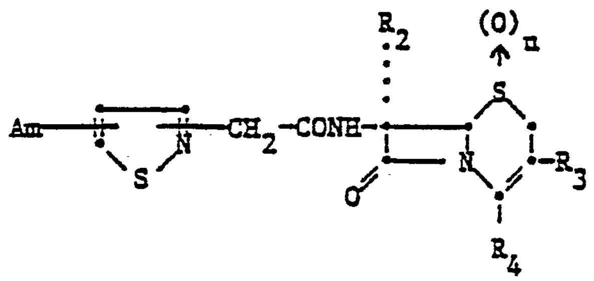

(Ie),

worin gegebenenfalls vorhandene funktionelle Gruppen in geschützter Form vorliegen können mit einem Nitrosierungsmittel behandelt, oder

f) zur Herstellung einer Verbindung der Formel (I), worin A eine Gruppe der Formel $-C(=N-O-R_1)-$ ist, worin $R_1$ gegebenenfalls substituiertes Niederalkyl bedeutet, in einer Verbindung der Formel

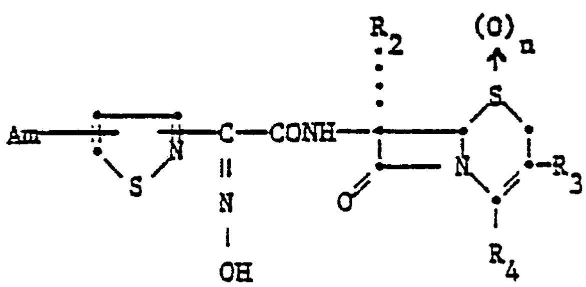

(If),

worin weitere gegebenenfalls vorhandene reaktionsfähige, funktionelle Gruppen geschützt sein können, die Hydroxyiminogruppe mit einem den gegebenenfalls substituierten Niederalkylrest $R_1$ einführenden Alkylierungsmittel behandelt, oder

g) zur Herstellung einer Verbindung der Formel (I), worin $R_2$ Niederalkoxy bedeutet, in eine Verbindung der Formel

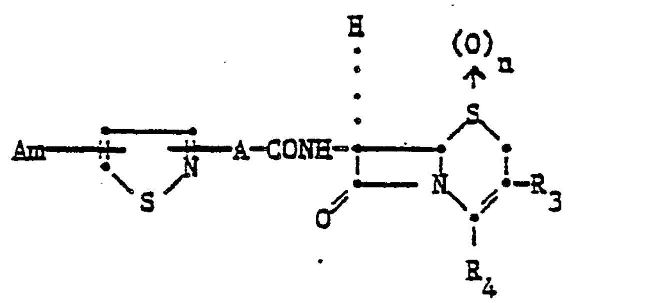

(Ig),

worin funktionelle Gruppen in geschützter Form vorliegen können, in 7α-Stellung eine Niederalkoxygruppe einführt, oder

h) zur Herstellung einer Verbindung der Formel (I), worin $R_3$ eine Gruppe der Formel $-CH_2-R_3^o$ bedeutet, worin $R_3^o$ für Heterocyclthio steht, eine Verbindung der Formel

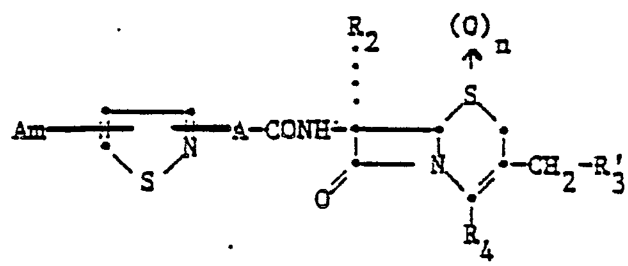

(Ih),

worin weitere gegebenenfalls vorhandene reaktionsfähige funktionelle Gruppen geschützt sein können, und $R_3'$ einen durch einen Heterocyclylthiorest $R_3^o$ ersetzbaren Rest bedeutet, mit einem Mercaptan $H-R_3^o$ , behandelt, oder

i) zur Herstellung einer Verbindung der Formel (I), worin $R_3^o$ für Acyloxy steht, eine Verbindung der Formel

BAD ORIGINAL

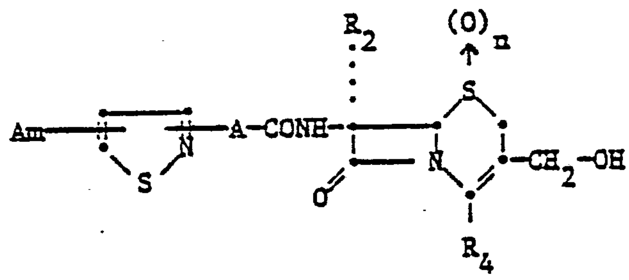

(Ii),

worin weitere gegebenenfalls vorhandene reaktionsfähige funktionelle Gruppen geschützt sein können, mit einem Acylierungsmittel behandelt, oder

j) zur Herstellung einer Verbindung der Formel (I), worin $R_3$ den Rest der Formel $-CH_2-R_3^o$ darstellt, worin $R_3^o$ eine Ammoniogruppe bedeutet, eine Verbindung der Formel

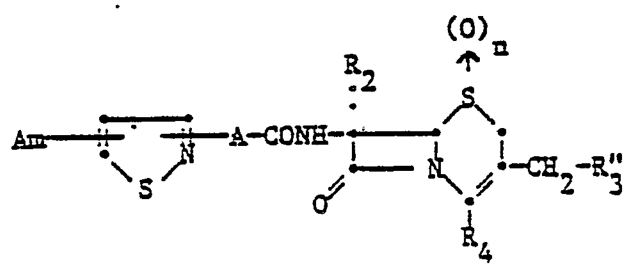

(Ij),

worin $R_3''$ einen durch nucleophile Substitution ersetzbaren Rest darstellt, und worin gegebenenfalls vorhandene funktionelle Gruppen in geschützter Form vorliegen können, mit einer organischen tertiären Aminbase umsetzt, oder

k) zur Herstellung einer Verbindung der Formel (I), worin $R_3$ für Wasserstoff steht,

ka) in einer Verbindung der Formel

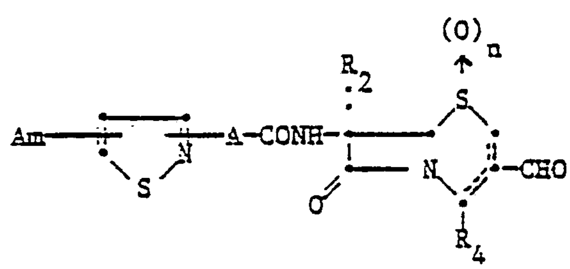

(Ika)

BAD ORIGINAL

worin die Doppelbindung in 2,3- oder in 3,4-Stellung sein kann, und worin gegebenenfalls vorhandene funktionelle Gruppen in geschützter Form vorliegen können, die Formylgruppe mittels Decarbonylierung durch Wasserstoff ersetzt, oder

kb) in einer Verbindung der Formel

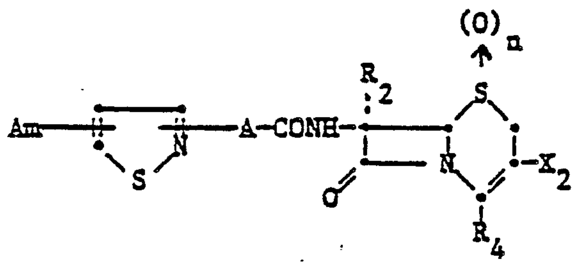

(Ikb)

worin $X_2$ Hydroxy oder gegebenenfalls substituiertes Amino darstellt, und worin weitere gegebenenfalls vorhandene funktionelle Gruppen in geschützter Form vorliegen können, oder eine entsprechende tautomere Verbindung davon, $X_2$ durch Wasserstoff ersetzt, oder

l) zur Herstellung einer Verbindung der Formel I, worin $R_3$ für veräthertes oder verestertes Hydroxy steht, eine Verbindung der Formel

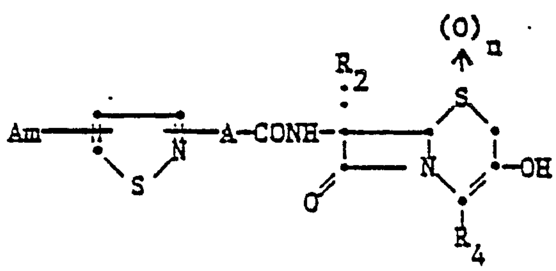

(Il),

oder die entsprechende tautomere 3-Oxo-cepham-Verbindung, worin $R_4$ funktionell abgewandeltes Carboxy darstellt, und weitere gegebenenfalls vorhandene funktionelle Gruppen in geschützter Form vorliegen können, mit einem veräthernden oder veresternden Mittel behandelt, oder

BAD ORIGINAL

m) zur Herstellung von Verbindungen der Formel (I), worin Am eine primäre, sekundäre oder tertiäre Aminogruppe ist, eine Verbindung der Formel

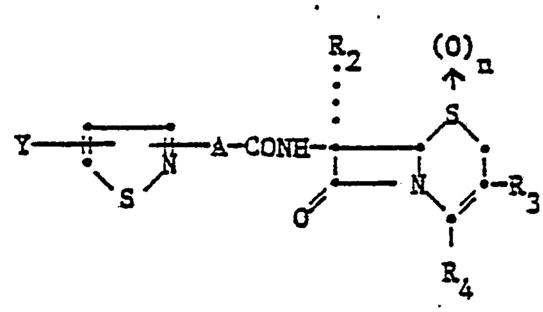

(Im),

worin y Halogen ist, worin weitere gegebenenfalls vorhandene reaktionsfähige funktionelle Gruppen geschützt sein können, mit Ammoniak oder einem primären oder sekundären Amin Am-H, oder einem Metallamid davon umsetzt, und wenn erwünscht, eine erfindungsgemäss erhältliche Verbindung der Formel I in eine andere Verbindung der Formel I umwandelt, und/oder, wenn erwünscht oder notwendig, in einer erfindungsgemäss erhältlichen Verbindung der Formel I eine in geschützte Form vorliegende funktionelle Gruppe in die freie funktionelle Gruppe überführt, und/oder, wenn erwünscht, ein erfindungsgemäss erhältliches Salz in die freie Verbindung oder in ein anderes Salz überführt, und/oder, wenn erwünscht, eine erfindungsgemäss erhältliche freie Verbindung mit einer salzbildenden Gruppe in ein Salz überführt, und/oder, wenn erwünscht, ein erfindungsgemäss erhältliches Gemisch von isomeren Verbindungen der Formel I in die einzelnen Isomeren auftrennt.

22. Die nach dem Verfahren gemäss Patentanspruch 21 erhältlichen Verbindungen.

23. Verbindungen der Formel

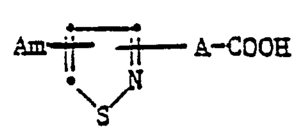

(III),

BAD ORIGINAL

worin Am eine gegebenenfalls geschützte Aminogruppe darstellt und A eine Gruppe $-CH_2-$,$-C(=O)-$, oder $-C(=N-O-R_1)-$ bedeutet, worin $R_1$ für Wasserstoff, gegebenenfalls substituiertes Niederalkyl oder Cycloalkyl, oder gegebenenfalls N-substituiertes Carbamoyl steht, und ihre zur Acylierung geeigneten Derivate.

24. Verfahren zur Herstellung von Verbindungen der Formel III gemäss Patentanspruch 23, dadurch gekennzeichnet, dass man

a) zur Herstellung von Verbindungen der Formel III, worin A eine Gruppe $-CH_2-$ ist, eine Verbindung der Formel

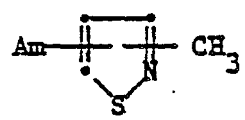 (B)

worin Am eine geschützte Aminogruppe $Am_o$ ist, carboxyliert, oder

b) zur Herstellung von Verbindungen der Formel III, worin A eine Gruppe $-C(=O)-$ ist, eine Verbindung der Formel III, worin A eine Gruppe $-CH_2-$ und Am eine geschützte Aminogruppe $Am_o$ ist, mit einem geeigneten Oxidationsmittel behandelt, oder

c) zur Herstellung von Verbindungen der Formel III, worin A eine Gruppe $-C(=N-O-R_1)$ ist, eine Verbindung der Formel III, worin A eine Gruppe $-C(=O)-$ und Am eine geschützte Aminogruppe $Am_o$ ist, mit einem Hydroxylamin $H_2N-O-R_1$ (V) behandelt oder eine Verbindung der Formel III, worin A eine Gruppe $-CH_2-$ und Am eine geschützte Aminogruppe $Am_o$ ist, nitrosiert und gewünschtenfalls eine erhaltene Verbindung der Formel III, worin $R_1$ Wasserstoff bedeutet, in eine Verbindung der Formel III überführt, worin $R_1$ von Wasserstoff verschieden ist, und gewünschtenfalls eine geschützte Aminogruppe $Am_o$ in eine freie oder eine andere geschützte Aminogruppe $Am_o$ überführt.

Patentansprüche: (für Oesterreich)

1. Verfahren zur Herstellung von 7β-Aminoisothiazolylacetamido-3-cephem-4-carbonsäureverbindungen der Formel

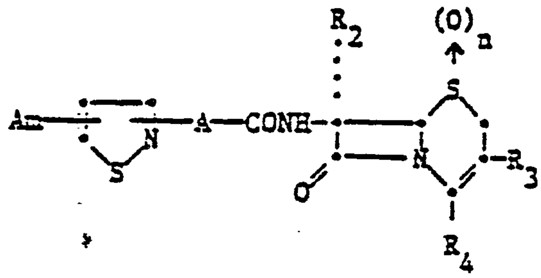

(I),

worin n für 0 oder 1 steht, Am eine gegebenenfalls geschützte Aminogruppe darstellt, A eine Gruppe $-CH_2-$, $-C(=O)-$, oder $-C(=N-O-R_1)-$ bedeutet, worin $R_1$ für Wasserstoff, gegebenenfalls substituiertes Niederalkyl oder Cycloalkyl, oder gegebenenfalls N-substituiertes Carbamoyl steht, $R_2$ Wasserstoff oder Niederalkoxy bedeutet, $R_3$ für Wasserstoff, veräthertes oder verestertes Hydroxy, veräthertes Mercapto, Formyl oder den Rest der Formel $-CH_2-R_3^o$ steht, worin $R_3^o$ für Wasserstoff, Hydroxy, Acyloxy, Heterocyclylthio oder Ammonio steht, und $R_4$ eine gegebenenfalls geschützte Carboxylgruppe darstellt, und Salzen von solchen Verbindungen, die eine salzbildende Gruppierung aufweisen, dadurch gekennzeichnet, dass man

a) in einer Verbindung der Formel

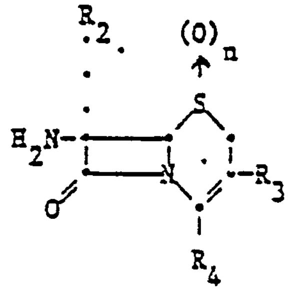

(II),

worin die 7β-Aminogruppe gegebenenfalls durch eine die Acylierung erlaubende Gruppe substituiert ist, und worin weitere gegebenenfalls vorhandene reaktionsfähige funktionelle Gruppen geschützt sein können, die 7β-Aminogruppe durch Behandeln mit einem den Acylrest einer Carbonsäure der Formel

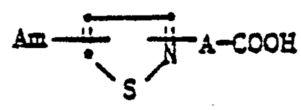

(III)

einführenden Acylierungsmittel, worin Am und A die oben angegebenen Bedeutungen haben, worin weitere gegebenenfalls vorhandene reaktionsfähige funktionelle Gruppen geschützt sein können, acyliert oder

b) eine 2-Cephemverbindung der Formel

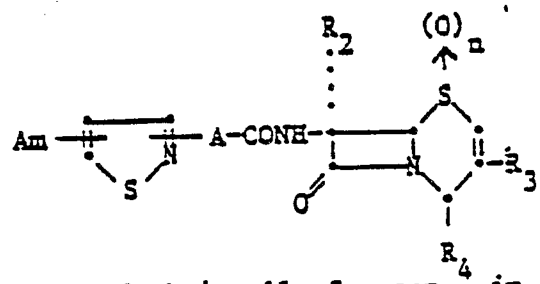

(IV),

worin gegebenenfalls vorhandene funktionelle Gruppen in geschützter Form vorliegen können, zur entsprechenden 3-Cephemverbindung isomerisiert, oder

c) zur Herstellung einer Verbindung der Formel (I), worin A eine Gruppe der Formel -C(=O)- ist, eine Verbindung der Formel

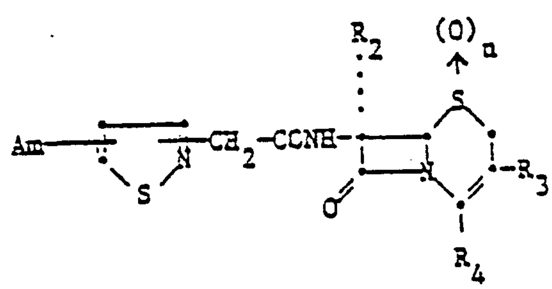

(Ic),

worin gegebenenfalls vorhandene funktionelle Gruppen in geschützter Form vorliegen können, mit einem geeigneten Oxidationsmittel behandelt, oder

d) zur Herstellung einer Verbindung der Formel (I), worin A eine Gruppe der Formel $-C(=N-O-R_1)-$ ist, eine Verbindung der Formel

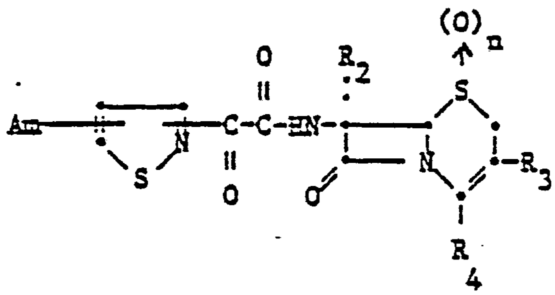

(Id),

worin gegebenenfalls vorhandene funktionelle Gruppen in geschützter Form vorliegen können, mit einem gegebenenfalls O-substituierten Hydroxylamin der Formel $H_2N-O-R_1$ (V) umsetzt, oder

e) zur Herstellung einer Verbindung der Formel (I), worin A eine Gruppe der Formel $-C(=N-O-R_1)-$ ist, worin $R_1$ Wasserstoff bedeutet, eine Verbindung der Formel

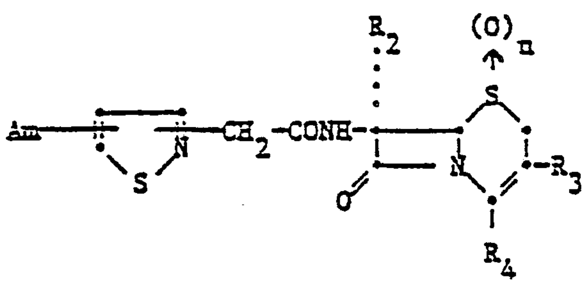

(Ie),

worin gegebenenfalls vorhandene funktionelle Gruppen in geschützter Form vorliegen können mit einem Nitrosierungsmittel behandelt, oder

f) zur Herstellung einer Verbindung der Formel (I), worin A eine Gruppe der Formel $-C(=N-O-R_1)-$ ist, worin $R_1$ gegebenenfalls substituiertes Niederalkyl bedeutet, in einer Verbindung der Formel

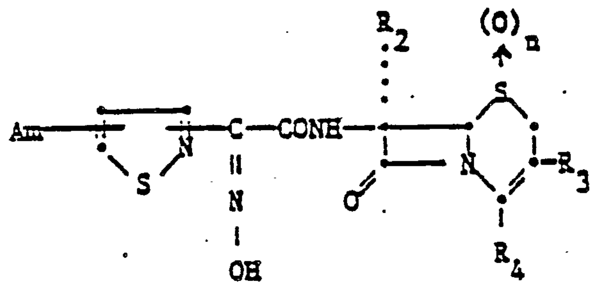

(If),

worin weitere gegebenenfalls vorhandene reaktionsfähige, funktionelle Gruppen geschützt sein können, die Hydroxyiminogruppe mit einem den gegebenenfalls substituierten Niederalkylrest $R_1$ einführenden Alkylierungsmittel behandelt, oder

g) zur Herstellung einer Verbindung der Formel (I), worin $R_2$ Niederalkoxy bedeutet, in eine Verbindung der Formel

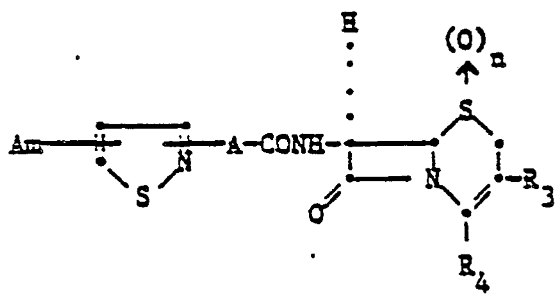

(Ig),

worin funktionelle Gruppen in geschützter Form vorliegen können, in 7α-Stellung eine Niederalkoxygruppe einführt, oder

h) zur Herstellung einer Verbindung der Formel (I), worin $R_3$ eine Gruppe der Formel $-CH_2-R_3^o$ bedeutet, worin $R_3^o$ für Heterocyclthio steht, eine Verbindung der Formel

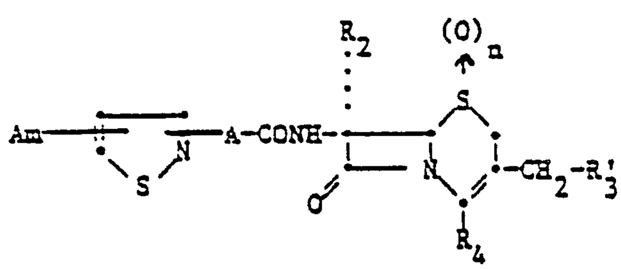

(Ih),

worin weitere gegebenenfalls vorhandene reaktionsfähige funktionelle Gruppen geschützt sein können, und $R_3'$ einen durch einen Heterocyclylthiorest $R_3^o$ ersetzbaren Rest bedeutet, mit einem Mercaptan $H-R_3^o$, behandelt, oder

i) zur Herstellung einer Verbindung der Formel (I), worin $R_3^o$ für Acyloxy steht, eine Verbindung der Formel

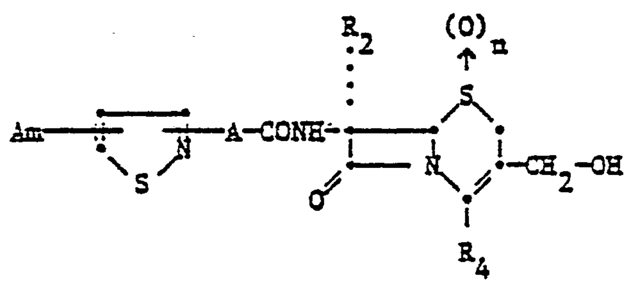

(Ii),

worin weitere gegebenenfalls vorhandene reaktionsfähige funktionelle Gruppen geschützt sein können, mit einem Acylierungsmittel behandelt, oder

j) zur Herstellung einer Verbindung der Formel (I), worin $R_3$ den Rest der Formel $-CH_2-R_3^o$ darstellt, worin $R_3^o$ eine Ammoniogruppe bedeutet, eine Verbindung der Formel

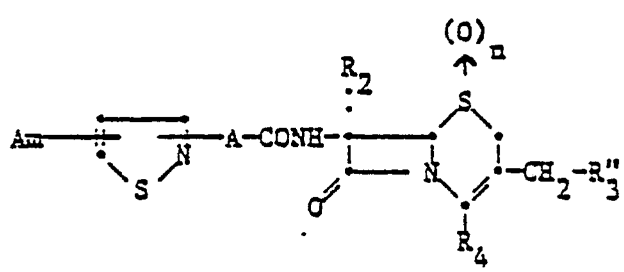

(Ij),

worin $R_3''$ einen durch nucleophile Substitution ersetzbaren Rest darstellt, und worin gegebenenfalls vorhandene funktionelle Gruppen in geschützter Form vorliegen können, mit einer organischen tertiären Aminbase umsetzt, oder

k) zur Herstellung einer Verbindung der Formel (I), worin $R_3$ für Wasserstoff steht,

ka) in einer Verbindung der Formel

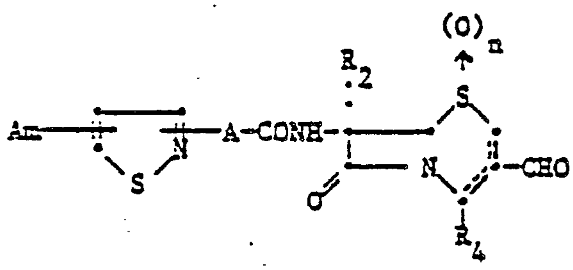

(Ika)

worin die Doppelbindung in 2,3- oder in 3,4-Stellung sein kann, und worin gegebenenfalls vorhandene funktionelle Gruppen in geschützter Form vorliegen können, die Formylgruppe mittels Decarbonylierung durch Wasserstoff ersetzt, oder

kb) in einer Verbindung der Formel

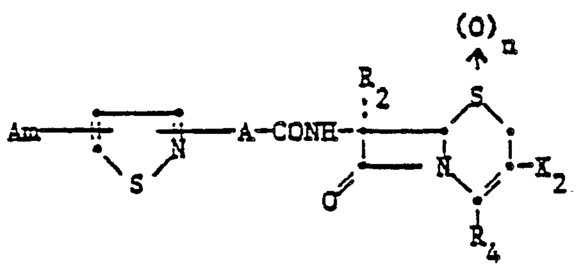

(Ikb)

worin $X_2$ Hydroxy oder gegebenenfalls substituiertes Amino darstellt, und worin weitere gegebenenfalls vorhandene funktionelle Gruppen in geschützter Form vorliegen können, oder eine entsprechende tautomere Verbindung davon, $X_2$ durch Wasserstoff ersetzt, oder

l) zur Herstellung einer Verbindung der Formel I, worin $R_3$ für veräthertes oder verestertes Hydroxy steht, eine Verbindung der Formel

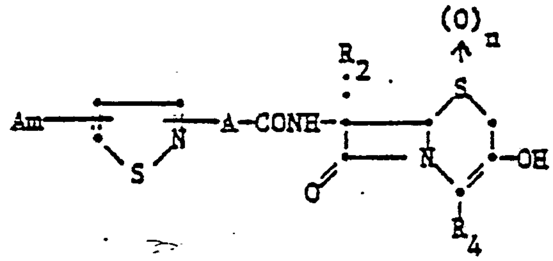

(I1),

oder die entsprechende tautomere 3-Oxo-cepham-Verbindung, worin $R_4$ funktionell abgewandeltes Carboxy darstellt, und weitere gegebenenfalls vorhandene funktionelle Gruppen in geschützter Form vorliegen können, mit einem veräthernden oder veresternden Mittel behandelt, oder

m) zur Herstellung von Verbindungen der Formel (I), worin Am eine primäre, sekundäre oder tertiäre Aminogruppe ist, eine Verbindung der Formel

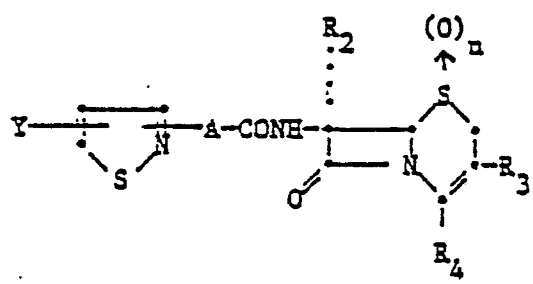

(Im),

worin y Halogen ist, worin weitere gegebenenfalls vorhandene reaktionsfähige funktionelle Gruppen geschützt sein können, mit Ammoniak oder einem primären oder sekundären Amin Am-H, oder einem Metallamid davon umsetzt, und wenn erwünscht, eine erfindungsgemäss erhältliche Verbindung der Formel I in eine andere Verbindung der Formel I umwandelt, und/oder, wenn erwünscht oder notwendig, in einer erfindungsgemäss erhältlichen Verbindung der Formel I eine in geschützter Form vorliegende funktionelle Gruppe in die freie funktionelle Gruppe überführt, und/oder, wenn erwünscht, ein erfindungsgemäss erhältliches Salz in die freie Verbindung oder in ein anderes Salz überführt, und/oder, wenn erwünscht, eine erfindungsgemäss erhältliche freie Verbindung

mit einer salzbildenden Gruppe in ein Salz überführt, und/oder, wenn erwünscht, ein erfindungsgemäss erhältliches Gemisch von isomeren Verbindungen der Formel I in die einzelnen Isomeren auftrennt.

2. Verfahren zur Herstellung von Verbindungen der Formel I nach Patentanspruch 1, worin n für 0 oder 1 steht, Am Amino oder Methylamino bedeutet, A eine Gruppe $-CH_2-$, $-C(=O)-$ oder $-C(=N-OR_1)-$ bedeutet, worin $R_1$ für Wasserstoff, Niederalkyl, durch Carboxy substituiertes Niederalkyl oder Cycloalkyl, oder für gegebenenfalls N-niederalkyliertes Carbamoyl steht, $R_2$ Wasserstoff oder Methoxy darstellt, $R_3$ für Wasserstoff, Niederalkoxy, Halogen, Niederalkylthio, Heterocyclylthio, worin Heterocyclyl für einen fünf- oder sechsgliedrigen monocyclischen, gegebenenfalls partiell gesättigten, aromatischen Heterocyclylrest steht, der mindestens 1 Ringstickstoffatom und gegebenenfalls 1-3 zusätzliche Ringstickstoffatome und/oder ein Ringsauerstoff- oder Ringschwefelatom enthält, und der durch gegebenenfalls Substituenten, wie Hydroxy, Amino, Diniederalkylamino, Carboxy, Carbamoyl oder Sulfo enthaltendes Niederalkyl, durch gegebenenfalls Substituenten, z.B. Niederalkyl, Niederalkoxy und/oder Halogen enthaltendes Phenyl, oder durch Thienyl substituiert sein kann, oder für den Rest der Formel $-CH_2-R_3^o$ steht, worin $R_3^o$ Niederalkanoyloxy, Carbamoyloxy, N-Niederalkylcarbamoyloxy, gegebenenfalls substituiertes, mit einem Ringkohlenstoffatom an die Thiogruppe gebundenes Heterocyclylthio, wie Triazolylthio, Tetrazolylthio, Thiazolylthio, Thiatriazolylthio, Thiadiazolylthio, Oxazolylthio, Oxadiazolylthio, Pyridazinylthio, Pyrimidinylthio oder Triazinylthio, worin die heterocyclischen Ringe partiell hydriert und gegebenenfalls durch Niederalkyl, N,N-Diniederalkylaminoniederalkyl, Carboxyniederalkyl, Sulfoniederalkyl, Amino, Carboxyniederalkanoylamino, Carbamoyl, Oxo oder Hydroxy substituiert sein können, oder gegebenenfalls durch Carbamoyl substituiertes Pyridinio bedeutet, und $R_4$ Carboxyl oder in physiologisch spaltbarer Form verestertes Carboxyl darstellt, und pharmazeutisch verwendbaren Salzen von solchen Verbindungen.

3. Verfahren zur Herstellung von Verbindungen der Formel I nach Patentanspruch 1, worin n für 0 steht, Am Amino oder Methylamino bedeutet, A eine Gruppe der Formel $-C(=N-O-R_1)$ bedeutet, worin $R_1$ für Wasserstoff, Methyl, 2-Carboxy-2-propyl oder 1-Carboxy-1-cyclopropyl steht, $R_2$ Wasserstoff oder Methoxy darstellt, $R_3$ für Wasserstoff, Niederalkoxy, Halogen, Heterocyclylthio, worin Heterocyclyl gegebenenfalls durch Niederalkyl substituiertes Oxadiazolyl oder Thiadiazolyl, gegebenenfalls durch Niederalkyl substituiertes Triazolyl, oder gegebenenfalls durch Niederalkyl, z.B. Methyl oder Aethyl, das Diniederalkylamino, z.B. Dimethylamino, Carboxy, Carbamoyl oder Sulfo als Substituenten enthalten kann, substituiertes 1H-Tetrazol-5-yl bedeutet, oder einen Rest der Formel $-CH_2-R_3^\circ$ steht, worin $R_3^\circ$ Acetoxy, Carbamoyloxy, gegebenenfalls durch Niederalkyl oder Carboxyniederalkyl substituiertes Triazolylthio gegebenenfalls durch Niederalkyl, Carboxyniederalkyl, Sulfoniederalkyl oder Diniederalkylaminoniederalkyl substituiertes Tetrazolylthio, gegebenenfalls durch Niederalkyl substituiertes Thiadiazolylthio, gegebenenfalls durch Hydroxy substituiertes Pyridazinylthio, gegebenenfalls durch Hydroxy substituiertes Pyrimidinylthio, oder durch Niederalkyl und zwei Hydroxy substituiertes Triazinylthio darstellt und $R_4$ Carboxyl oder in physiologisch spaltbarer Form verestertes Carboxyl bedeutet, und pharmazeutisch verwendbaren Salzen von solchen Verbindungen.

4. Verfahren zur Herstellung von Verbindungen der Formel I nach Patentanspruch 1, worin n für 0 steht, Am in 5-Stellung des Isothiazolringes steht und Amino bedeutet, A mit der 3-Stellung des Isothiazolringes verknüpft ist und eine Gruppe $-C(=N-O-R_1)-$ in syn(Z)-Konfiguration bedeutet, worin $R_1$ für Methyl steht $R_2$ Wasserstoff darstellt, $R_3$ für Wasserstoff oder eine Gruppe $-CH_2R_3^\circ$ steht, worin $R_3^\circ$ Niederalkanoyloxy, wie Acetoxy, oder gegebenenfalls durc Methyl oder Carboxyniederalkyl substituiertes Tetrazolylthio darstellt und $R_4$ Carboxyl bedeutet und pharmazeutisch verwendbaren Salzen von solchen Verbindungen.

5. Verfahren zur Herstellung von 7β-[2-(5-Amino-isothiazol-3-yl)-2-Z-methoxyiminoacetamido]-3-acetoxymethyl-3-cephem-4-carbonsäure und pharmazeutisch verwendbaren Salzen davon nach Patentanspruch 1.

6. Verfahren zur Herstellung von 7β-[2-(5-Amino-isothiazol-3-yl)-2-Z-methoxyiminoacetamido]-3-(1-methyltetrazol-5-yl-thiomethyl)-3-cephem-4-carbonsäure und pharmazeutisch verwendbaren Salzen davon nach Patentanspruch 1.

7. Verfahren zur Herstellung von 7β-[2-(5-Amino-isothiazol-3-yl)acetamido]-3-acetoxymethyl-3-cephem-4-carbonsäure und pharmazeutisch verwendbaren Salzen davon nach Patentanspruch 1.

8. Verfahren zur Herstellung von 7β-[2-(5-Amino-isothiazol-3-yl)-2-E-methoxyiminoacetamido]-3-acetoxymethyl-3-cephem-4-carbonsäure und pharmazeutisch verwendbaren Salzen davon nach Patentanspruch 1.

9. Verfahren zur Herstellung von 7β-[2-(5-Aminoisothiazol-3-yl-)-2-E-methoxyiminoacetamido]-3-(1-methyltetrazol-5-yl-thiomethyl)-3-cephem-4-carbonsäure und pharmazeutisch verwendbaren Salzen davon nach Patentanspruch 1.

10. Verfahren zur Herstellung von 7β-[2-(5-Aminoisothiazol-3-yl-)-2-hydroxyiminoacetamido]-3-(1-methyltetrazol-5-yl-thiomethyl)-3-cephem-4-carbonsäure und pharmazeutisch verwendbaren Salzen davon nach Patentanspruch 1.

11. Verfahren zur Herstellung von 7β-[2-(5-Amino-isothiazol-3-yl)-2-Z-methoxyimino-acetamido]-3-(1-carboxymethyltetrazol-5-yl-thiomethyl)-3-cephem-4-carbonsäure und pharmazeutisch verwendbaren Salzen davon nach Patentanspruch 1.

12. Verfahren zur Herstellung von 7β-[2-(5-Amino-isothiazol-3-yl)-2-Z-methoxyiminoacetamido]-3-cephem-4-carbonsäure und pharmazeutisch verwendbaren Salzen davon nach Patentanspruch 1.

13. Verfahren zur Herstellung von 7β-[2-(5-Amino-isothiazol-3-yl)-2-Z-methoxyiminoacetamido]-3-cephem-4-carbonsäure-pivaloyloxymethylester nach Patentanspruch 1.

14. Verfahren zur Herstellung von 7β-[2-(5-Amino-isothiazol-3-yl)-2-Z-methoxyiminoacetamido]-3-(4-carbamoylpyridiniomethyl)-3-cephem-4-carboxylat nach Patentanspruch 1.

15. Verfahren zur Herstellung des Natriumsalzes einer Verbindung der Formel I gemäss einem der Patentansprüche 1-11.

16. Verfahren zur Herstellung des Natriumsalzes einer Verbindung der Formel I gemäss Anspruch 12.

17. Verfahren zur Herstellung von Verbindungen der Formel

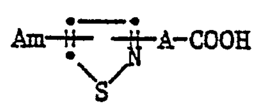

(III),

dadurch gekennzeichnet, dass man

a) zur Herstellung von Verbindungen der Formel III, worin A eine Gruppe $-CH_2-$ ist, eine Verbindung der Formel

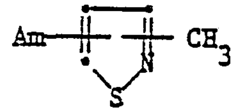

(B)

worin Am eine geschützte Aminogruppe $Am_o$ ist, carboxyliert, oder

b) zur Herstellung von Verbindungen der Formel III, worin A eine Gruppe $-C(=O)-$ ist, eine Verbindung der Formel III, worin A eine Gruppe $-CH_2-$ und Am eine geschützte Aminogruppe $Am_o$ ist, mit einem geeigneten Oxidationsmittel behandelt, oder

c) zur Herstellung von Verbindungen der Formel III, worin A eine Gruppe $-C(=N-O-R_1)$ ist, eine Verbindung der Formel III, worin A eine Gruppe $-C(=O)-$ und Am eine geschützte Aminogruppe $Am_o$ ist, mit einem Hydroxylamin $H_2N-O-R_1$ (V) behandelt oder eine Verbindung der Formel III, worin A eine Gruppe $-CH_2-$ und Am eine geschützte Aminogruppe $Am_o$ ist, nitrosiert und gewünschtenfalls eine erhaltene Verbindung der Formel III, worin $R_1$ Wasserstoff bedeutet, in eine Verbindung der Formel III überführt, worin $R_1$ von Wasserstoff verschieden ist, und gewünschtenfalls eine geschützte Aminogruppe $Am_o$ in eine freie oder eine andere geschützte Aminogruppe $Am_o$ überführt.